(19) Europäisches Patentamt
European Patent Office
Office européen des brevets

(11) EP 4 623 702 A1

(12) EUROPEAN PATENT APPLICATION

(43) Date of publication:
**01.10.2025 Bulletin 2025/40**

(21) Application number: **24166746.8**

(22) Date of filing: **27.03.2024**

(51) International Patent Classification (IPC):
***A23L 33/105** (2016.01)* ***A61K 31/00** (2006.01)*

(52) Cooperative Patent Classification (CPC):
**A23L 33/105; A61K 31/00**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Nicoventures Trading Limited**
**London WC2R 3LA (GB)**

(72) Inventor: **DAVIES, Ashley**
**London, WC2R 3LA (GB)**

(74) Representative: **D Young & Co LLP**
**3 Noble Street**
**London EC2V 7BQ (GB)**

(54) **ORAL PRODUCT**

(57) The present disclosure relates to an oral product comprising a combination of active ingredients, processes for preparation of said oral product, and uses of said oral product.

FIGURE 1

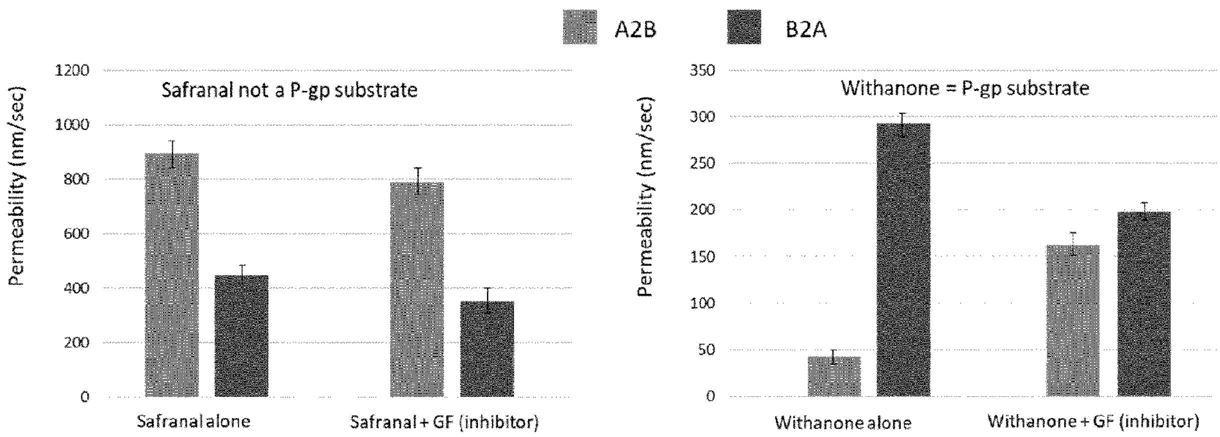




Processed by Luminess, 75001 PARIS (FR)

## Description

### FIELD OF THE INVENTION

The present disclosure relates to an oral product, a process for producing the oral product, as well as to uses of said oral product.

### BACKGROUND

The present disclosure relates to products and compositions intended for human use. The products are configured for oral ingested use and deliver substances such as flavours and/or active ingredients during use.

Products that are easy to administer orally and which can provide a beneficial effect on particular mood states of a human or animal have become popular in recent years. For example, confectionary-type products (e.g. gummies or pastilles) containing vitamins or other mood-enhancing actives provide a convenient and pleasant mode of administration for such active ingredients. Other convenient modes of administration are food and beverages, for example energy drinks. Such products may include active ingredients that are delivered to the user in order to cause a biological response in the user that may enhance physical or mental performance of the user.

It would be desirable to provide an oral product configured for oral use which may deliver active ingredients to the consumer in an enjoyable and effective form, such as in the form of a liquid shot or in the form of a chewable solid product. A liquid shot may be described as a drink or beverage that can be consumed quickly, e.g. in one action by the consumer: one "gulp". The oral product in this form may, for example, have a volume of from about 10 ml to about 100 ml, such as from about 25 ml to about 75 ml. A chewable solid product may be a dissolvable chew and similarly be consumed quickly, e.g. in one action, by the user. When formulating these types of products, there can be challenges associated with handling certain active ingredients. It may, for instance, be difficult to achieve the desired effect on particular mood states of a human or animal without using high amounts of a particular active, but such amounts may be difficult to prepare in the desired dosage form, be subject to compliance or safety restraints, and/or provide an unpleasant sensorial experience. Hence, there is a desire to include active ingredients in oral products which enhance, boost or improve the effect(s) of one or more other actives in the product.

### BRIEF SUMMARY

The present disclosure generally provides products configured for oral use, the products comprising a combination of active ingredients. The oral products may be in any form suitable for oral use, such as in a chewable, tablet or lozenge form, as a loose powder, or liquid form (e.g. as a beverage, such as a shot). In preferred embodiments, the oral product may be in a chewable form or a liquid form.

In accordance with some embodiments described herein, there is provided an oral product comprising a combination of active ingredients, wherein the combination of active ingredients comprises (i) piperine or a geometric isomer thereof, and (ii) at least one p-glycoprotein substrate compound having a log D value at pH 7.4 from about -2.5 to about 4.5.

In accordance with some embodiments described herein, there is provided a process for preparing an oral product. The process comprising: (a) contacting at least one binding agent and at least one bulking agent selected from the group consisting of a sugar alcohol, or a sugar, or a combination of at least one sugar alcohol and at least one sugar, and optionally adding water, (b) heating the mixture of binding agent, sugar alcohol and/or sugar, and optionally water, (c) adding a combination of active ingredients, wherein the combination of active ingredients is as defined herein for the oral product, and (d) allowing the resulting mixture to set to provide an oral product in the form of a chew. Alternatively the process comprises: (a) combining active ingredients, wherein the combination of active ingredients is as defined herein for the oral product, (b) contacting the active ingredients with water, and (c) mixing the active ingredients with the water to prepare an oral product in the form of a liquid.

In accordance with some embodiments described herein, there is provided piperine or a geometric isomer thereof for use in increasing the blood-brain barrier permeation of at least one p-glycoprotein substrate compound, wherein the at least one p-glycoprotein substrate compound has a log D value at pH 7.4 from about -2.5 to about 4.5. In preferred embodiments, the at least one p-glycoprotein substrate compound is selected from crocetin, crocin, withanolide A, withanone, or a combination thereof.

These and other features, aspects, and advantages of the disclosure will be apparent from a reading of the following detailed description. The invention includes any combination of two, three, four, or more of the above-noted embodiments as well as combinations of any two, three, four, or more features or elements set forth in this disclosure, regardless of whether such features or elements are expressly combined in a specific embodiment description herein. This disclosure is intended to be read holistically such that any separable features or elements of the disclosed invention, in any

of its various aspects and embodiments, should be viewed as intended to be combinable unless the context clearly dictates otherwise.

For ease of reference, these and further aspects of the present invention are now discussed under appropriate section headings. However, the teachings under each section are not necessarily limited to each particular section.

## BRIEF DESCRIPTION OF THE DRAWINGS

Embodiments of the invention will now be described, by way of example only, with reference to accompanying drawings, in which:

Figure 1 is a bar chart from Example 1 showing the MDCK-MDR1 assay results for blood-brain barrier (BBB) permeability (nm/sec) in both directions of safranal and withanone, respectively with and without the P-gp inhibitor GF 120918. These results show that safranal is not a P-gp substrate and withanone is a P-gp substrate.

Figures 2 to 11 are bar charts from Example 2 showing the MDCK-MDR1 assay results for BBB permeability (nm/sec) in the A2B direction for each named compound alone, in combination with piperine at 30 μM, and in combination with quercetin at 30 μM. Figures 2 to 11 respectively relate to safranal, crocetin, crocin, withanone, withanolide A, withanolide B, oleanoic acid, ursolic acid, apigenin, and luteolin-7-glucorinide.

## DETAILED DESCRIPTION

It is to be understood that this invention is not limited to the particular configurations, process steps, and materials disclosed herein as such configurations, process steps, and materials may vary somewhat. It is also to be understood that the terminology employed herein is used for the purpose of describing particular embodiments only and is not intended to be limiting since the scope of the present invention will be limited only by the appended claims and equivalents thereof.

As used in this specification and the claims, the singular forms "a," "an," and "the" include plural referents unless the context clearly dictates otherwise. Reference to "dry % by weight" or "dry weight basis" refers to weight on the basis of dry ingredients (i.e., all ingredients except water). Reference to "wet weight" refers to the weight of the product or composition including water. Unless otherwise indicated, reference to "% by weight" (or "% by weight") of a product or composition reflects the total weight of the product or composition.

In this specification, unless otherwise stated, the term "about" modifying the quantity of an ingredient in the oral product of the invention or employed in the methods of the invention refers to variation in the numerical quantity that can occur, for example, through typical measuring and liquid handling procedures used for making concentrates or use solutions in the real world; through inadvertent error in these procedures; through differences in the manufacture, source, or purity of the ingredients employed to make the oral product, or to carry out the methods; and the like. The term "about" also encompasses amounts that differ due to different equilibrium conditions for a product or composition resulting from a particular initial mixture. Whether or not modified by the term "about", the claims include equivalents to the quantities.

### Oral Product

As described herein, there is provided an oral product comprising a combination of active ingredients, wherein the combination comprises (i) piperine or a geometric isomer thereof, and (ii) at least one p-glycoprotein substrate compound having a log D value at pH 7.4 from about -2.5 to about 4.5. The oral product is configured for oral use, and thus for insertion into the user's mouth (i.e. oral cavity). In this specification, unless otherwise stated, the term "oral" in connection to a product which, in normal use, is suited to be ingested or placed somewhere in the oral cavity of the user. For example, the product may be in the form of a liquid that may be consumed orally by the user, e.g. the product may be in the form of a beverage with a volume of 250 mL or greater, or a liquid shot with a volume of from about 1 mL to about 200 mL, preferably about 20 mL to about 100 mL. The product may also be placed in the mouth, e.g. in the form of a lozenge or a chew.

The person skilled in the art will understand that the ranges provided herein may be taken alone or combined with one or more other component ranges to provide a preferred aspect of the invention.

<u>Combination of active ingredients</u>

The product as disclosed herein includes active ingredients. The active ingredients may be naturally occurring or synthetically obtained. The combination of active ingredients comprises compounds that cause a biological or physiological response in a human or animal. In particular, the combination of active ingredients comprises (i) piperine or a geometric isomer thereof, and (ii) at least one p-glycoprotein substrate compound having a log D value at pH 7.4 of from

about -2.5 to about 4.5. Each of the active ingredients may be present in an amount suitable to provide the desired response in a human or animal.

In some embodiments, the at least one p-glycoprotein substrate compound is present in an amount to provide an effect on a mood state of the human or animal. As used herein, the term "mood" or "mood state" is defined as a short-term feeling that exists for minutes or days. Moods influence well-being and can impact both behavior patterns and perceived health. They may be assessed by techniques known in the art including the Profile of Mood States (POMS); a 65 item self-report psychological instrument intended for use with adults aged 18 or above. The mood state being effected by the at least one p-glycoprotein substrate compound is not limited in the present disclosure. The mood state may relate to physical or mental wellbeing and be measured by the Profile of Mood States. In some embodiments, the mood state may be selected from tension-anxiety, depression-dejection, anger-hostility, vigor-activity, fatigue-inertia, and confusion-bewilderment, as measured by the Profile of Mood States.

As will be understood by the person skilled in the art, in order for active ingredients to provide the desired response in a human or animal, they must have a high bioavailability; bioavailability meaning the extent to which a substance becomes available to its intended biological destination(s). When an active ingredient is administered intravenously, it generally has 100% bioavailability. However, when an active ingredient is administrated orally, as in the present disclosure, its bioavailability is typically lower than 100% due to intestinal epithelium absorption and first-pass metabolism once ingested.

In the context of oral products having an effect on "mood state" or "mood", it is specifically desirable for the active ingredient(s) to be bioavailable such that they are effectively delivered to the human or animal brain and hence they are required to permeate the blood-brain barrier. The focus of the present disclosure is therefore on the blood-brain barrier.

The blood-brain barrier (BBB) is a selective, semi-permeable membrane that controls the transfer of active ingredients from the blood into the brain. It comprises brain microvascular endothelial cells surrounded by pericytes, astrocytic endfeet, microglia and neurons. These components form a complex and functional 'neurovascular unit'. A feature of the BBB is its low and selective permeability to molecules which can be attributed to its unique biological characteristics, including specialized tight junctions between adjacent endothelial cells. Small water-soluble molecules can diffuse paracellularly through the tight junctions, but not to any great extent, whereas small lipid soluble substances such as alcohol and steroid hormones can penetrate transcellularly by dissolving in the lipid plasma membrane. A 2012 review by Chen and Liu (Adv Drug Deliv Rev. 2012 May 15; 64(7): 640-65) on brain permeability and efflux of nutraceuticals and neuroprotective phytochemicals showed that whilst many chemicals are sufficiently lipophilic to cross the BBB passively, they are subject to active efflux and thus have low BBB permeability.

The p-glycoprotein (P-gp) is known in the art as an ATP-dependent efflux pump which is present in the capillary endothelial cells composing the blood-brain barrier. In particular, P-gp is a glycoprotein that in humans is encoded by the ABCB1 gene and is a well-characterized ABC-transporter of the MDR/TAP subfamily which transports a wide variety of substrates across extra- and intra-cellular membranes. Hence, p-glycoproteins play a role in the active efflux of the BBB; they may be described as efflux transporters. Specifically, it is known that a number of lipophilic active ingredients are substrates for the p-glycoprotein and thus targeted by its efflux pump mechanism when crossing the blood-brain barrier resulting in low BBB permeability of the ingredients. When such ingredients are included in an oral product which is intended to have an effect on "mood state" or "mood", this low BBB permeability can cause problems in product efficacy and/or preparation.

As noted above, it is desirable to provide an oral product configured for oral use which may deliver active ingredients having an effect to the consumer in an enjoyable and effective form, such as in the form of a liquid shot or in the form of a chewable solid product. A liquid shot may be described as a drink or beverage that can be consumed quickly, e.g. in one action by the consumer: one "gulp". The oral product in this form may, for example, have a volume of from about 10 ml to about 100 ml, such as from about 25 ml to about 75 ml. A chewable solid product may be a dissolvable chew and similarly be consumed quickly and in one "action" by the user thereby necessitating a small size. The size/volume restraints on the oral product coupled with requirement for an effect, e.g. an effect on "mood" or "mood state", means that certain active ingredients may have to be included at high amounts to achieve the desired effects, but such amounts may be difficult to prepare in the desired dosage form, be subject to compliance or safety restraints, and/or provide an unpleasant sensorial experience.

The present disclosure solves these problems by combining piperine or a geometric isomer thereof with the defined p-glycoprotein substrate compound(s) in the oral product. As supported by the Examples herein, piperine was surprisingly found to improve the blood-brain barrier permeation of the compound(s) thereby enabling such compound(s) to have an effect when consumed by a user without having to modify the inclusion levels of said compound(s). This is beneficial for the reasons presented above. In particular, the combination of piperine and the defined p-glycoprotein substrate compound(s) have a BBB permeability greater than the permeability of the compound alone.

It has also been found that the combination of active ingredients described herein may provide a safe product, with desirable pharmacokinetics (Tmax, Cmax, half life), bioavailability and metabolism.

It has been found that the oral product of the present invention is effective for use over the short, medium or long

term. Such use can include daily use of the oral product. For example, a single dosage form of the oral product may be consumed once per day by the user, and the oral product may be used for at least 7 days, at least 14 days, at least 21 days, or at least 28 days.

Piperine is an alkaloid found in fruits of the pepper species in the family *Piperaceae,* such as black pepper (*Piper nigrum*)*,* long pepper (*Piper longum*)*,* red long pepper (*Piper retrofactum*) and voatsiperifery pepper (*Piper borbonense*). Piperine is a known p-glycoprotein inhibitor. Its IUPAC name is (2E,4E)-5-(2H-1,3-benzodioxol-5-yl)-1-(piperidin-1-yl)-penta-2,4-dien-1-one and it has the following chemical structure:

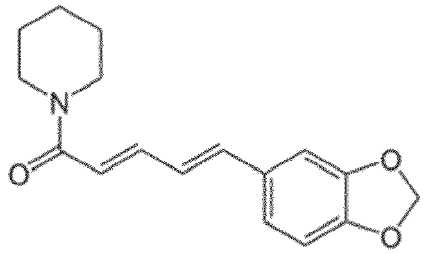

All embodiments include, where appropriate, all enantiomers and tautomers of the compounds described herein. The person skilled in the art will recognize compounds that possess optical properties (one or more chiral carbon atoms) or tautomeric characteristics. The corresponding enantiomers and/or tautomers may be isolated or prepared by methods known in the art. The compounds described herein may exist as stereoisomers and/or geometric isomers; e.g. they may possess one or more asymmetric and/or geometric centres and so may exist in two or more stereoisomeric and/or geometric forms. All embodiments include, where appropriate, the use of stereoisomers and geometric isomers of those compounds, and mixtures thereof. Piperine is, for instance, known to have three geometric isomers: cis-trans isomer (isopiperine), cis-cis isomer (chavicine) and trans-cis isomer (isochavicine). Hence, the present disclosure refers to piperine or a geometric isomer thereof. Reference herein to a geometric isomer of piperine is to isopiperine, chavicine and isochavicine. In preferred embodiments, the present disclosure is focused on piperine with the chemical structure above, i.e. (2E,4E)-5-(2H-1,3-benzodioxol-5-yl)-1-(piperidin-1-yl)-penta-2,4-dien-1-one.

The origin of piperine or a geometric isomer thereof is not limited in the present disclosure. In some embodiments it may be obtained from a black pepper extract. Piperine and its geometric isomers are typically extracted from black pepper by using organic solvents such as dichloromethane. Piperine can also be prepared by treating the solvent-free residue from a concentrated alcoholic extract of black pepper with a solution of potassium hydroxide to remove resin. The solution is decanted from the insoluble residue and left to stand overnight in alcohol. During this period, the alkaloid crystallizes from the solution. Synthetic preparation methods are also known, e.g. the action of piperonyl chloride on piperidine. Commercial sources are also available. The geometric isomers are generally formed from piperine in light, especially ultra-violet light, with isomerisation increasing with an increase in light intensity and time exposure. The chemical structures of the geometric isomers are shown below.

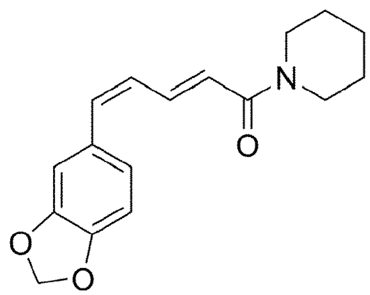

Isochavicine

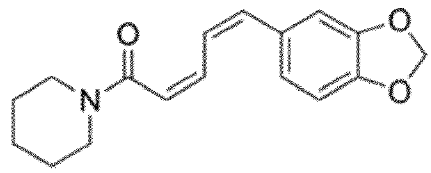

Chavicine

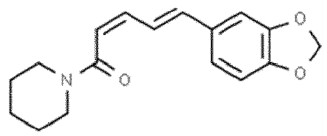

Isopiperine

In some embodiments, the oral product may comprise about 0.0001 wt% to about 0.5 wt% of piperine or a geometric isomer thereof, based on the total weight of the oral product. In preferred embodiments the oral product may comprise about 0.0005 wt% to about 0.1 wt% of piperine or a geometric isomer thereof, based on the total weight of the oral product. In particularly preferred embodiments the oral product may comprise about 0.001 wt% to about 0.05 wt% of piperine or a geometric isomer thereof, based on the total weight of the oral product.

In some embodiments, the oral product may comprise about 5 mg to about 20 mg of piperine or a geometric isomer thereof. In preferred embodiments, the oral product may comprise about 8 mg to about 18 mg of piperine or a geometric isomer thereof. In particularly preferred embodiments, the oral product may comprise about 10 mg to about 15 mg of piperine or a geometric isomer thereof.

In addition to piperine or a geometric isomer thereof, the oral product includes at least one p-glycoprotein substrate compound having a log D value at pH 7.4 from about - 2.5 to about 4.5. The term "p-glycoprotein substrate" or "p-glycoprotein substrate compound" as used herein, is a substance that binds to the p-glycoprotein efflux transporter within the blood-brain barrier which limits permeation of the substance across the blood-brain barrier. Such a p-glycoprotein substrate compound may be identified by using the *in vitro* MDCK-MDR1 assay as demonstrated by the Examples herein.

In some embodiments, the at least one p-glycoprotein substrate compound is a physiologically active compound, which is a compound intended to achieve or enhance a physiological response. The compound may, for example, be selected from nutraceuticals, phytochemicals, botanicals, nootropics, or psychoactives for non-therapeutic use.

In some embodiments, the at least one p-glycoprotein substrate compound is derived from a botanical. The term "derived from" means that the p-glycoprotein substrate compound is obtained from the botanical, i.e. the botanical is used as a source ingredient for the respective compound. Withanone and withanolide A are, for example, known compounds obtained from or sourced from Ashwaganda. The manner in which the compounds are sourced is not limited, the term "derived from" includes extracts or isolates from the botanical. Extraction methods are known in the art; they include, without being limited to, solvent extraction with ethanol and/or other organic solvents, and solvent-free methods such as pressurized water-based liquid extraction, microwave extraction, supercritical fluid extraction, cold press, and the like.

As used herein, the term "botanical" or "botanical ingredient" refers to any plant material or fungal-derived material, including plant material in its natural form and plant material derived from natural plant materials, such as extracts or isolates from plant materials or treated plant materials (e.g. plant materials subjected to heat treatment, fermentation, bleaching, or other treatment processes capable of altering the physical and/or chemical nature of the material). For the purposes of the present disclosure, a "botanical" includes, but is not limited to, "herbal materials," which refer to seed-producing plants that do not develop persistent woody tissue and are often valued for their medicinal or sensory characteristics (e.g., teas or tisanes). The botanical materials useful in the present disclosure may comprise, without limitation, any of the compounds and sources set forth herein, including mixtures thereof. Certain botanical materials of this type are sometimes referred to as dietary supplements, nutraceuticals, "phytochemicals" or "functional foods." Certain botanicals, as the plant material or an extract thereof, have found use in traditional herbal medicine, and are described further herein.

Non-limiting examples of botanicals or botanical-derived materials include ashwagandha (Indian ginseng), *Bacopa monniera,* baobab, basil, *Camellia sinensis, Centella asiatica,* Chai-hu, chamomile, cherry blossom, chlorophyll, cinnamon, citrus, cloves, cocoa, cordyceps, damiana, *Dorstenia arifolia, Dorstenia odorata,* essential oils, eucalyptus, fennel, *Galphimia glauca,* ginger, *Ginkgo biloba,* ginseng (e.g., *Panax ginseng), Grif.fonia simplicifolia,* guarana, cannabis, hemp, hops, jasmine, *Kaempferia parviflora* (Thai ginseng), kava, lavender, lemon balm, lemongrass, licorice, lutein, maca, matcha, Nardostachys chinensis, oil-based extract of *Viola odorata,* peppermint, resveratrol, *Rhizoma gastrodiae, Rhodiola, rooibos,* rose essential oil, rosemary, saffron, *Sceletium tortuosum,* Schisandra, Skullcap, spearmint extract, Spikenard, terpenes, tisanes, *Turnera aphrodisiaca,* valerian, white mulberry, echinacea and *Yerba mate.*

In some embodiments the at least one glycoprotein substrate compound is derived from a botanical and is a physiologically active compound. The compound may, for example, be selected from nutraceuticals, phytochemicals, nootropics, and psychoactives for non-therapeutic use.

In some embodiments the at least one glycoprotein substrate compound is derived from a botanical, wherein the botanical is selected from ginseng, maca root, lemon balm, chamomile, saffron, cocoa, Echinaceae, and *Camellia sinensis.* In some embodiments the at least one glycoprotein substrate compound is also a physiologically active compound for non-therapeutic use. In preferred embodiments the botanical is selected from ginseng and saffron, particularly preferred is Ashwagandha or saffron.

The p-glycoprotein substrate compounds included in the oral product of the present disclosure are further defined by their log D value at pH 7.4. It is known in the art that "log D" is a distribution coefficient which is used as a measure of lipophilicity. It is pH dependent and a pH of 7.4 is used herein because it is the physiological pH of blood serum. One of the most common methods for determining this parameter is by measuring the partition of a compound between an organic solvent, typically octanol, and aqueous buffer at the specified pH (7.4) in a "shake-flask" method. For example, octanol (presaturated with buffer) may be added to the test compound. Buffer at pH 7.4 (presaturated with octanol) may then be added to the octanol. A ratio of 2:1 v/v buffer to octanol may be prepared. The system is mixed to allow distribution of the compound between the two phases. After separation, the compound is quantified in the aqueous phase and octanol phase by LC-MS/MS and the following equation is used to calculate the Log $D_{7.4}$.

$$\text{Log}\, D_{7.4} = \text{Log}\, [\frac{C_{oct}}{C_{aq}}]$$

Where:$C_{oct}$ = concentration in octanol sample (corrected for dilution), and $C_{aq}$ = concentration in aqueous sample (corrected for dilution). Log $D_{7.4}$ values are also available from computer software tools. In the Examples of the present disclosure, the modelling software GastroPlus® (ADMET Predictor Module, available from SimulationsPlus) was used.

An optimal range for lipophilicity may be achieved if the compound has a log $D_{7.4}$ value between 0 and 3. These compounds typically have a good balance between solubility and permeability. The optimal log $D_{7.4}$ for blood-brain barrier permeation is approximately 2. Hydrophilic compounds have a log $D_{7.4}$ less than 0 and are typically highly soluble but exhibit low permeability across the blood-brain barrier. Highly lipophilic compounds have a log $D_{7.4}$ greater than 5 and exhibit problems with metabolic instability and low solubility which can lead to variable and poor oral absorption.

In the present disclosure, it was found that piperine increased the blood-brain barrier permeation of p-glycoprotein substrate compounds having a log D value at pH 7.4 (log $D_{7.4}$) from about -2.5 to about 4.5. In preferred embodiments, the log D value at pH 7.4 of the at least one p-glycoprotein substrate compound is from about -2.5 to about 4.0. In particularly preferred embodiments, the log D value at pH 7.4 is either from about -2.0 to about 0.5, or from about 3.0 to about 3.5. Hence, the compounds have partial lipophilic and/or partial hydrophilic character. The Examples demonstrate that ursolic acid (log $D_{7.4}$ = 4.89), a lipophilic compound, was not enhanced by piperine.

In some embodiments the at least one p-glycoprotein substrate compound may be further defined by a log P value. Log P is a partition coefficient which is also used as a measure of lipophilicity for neutral compounds, or where the compound exists in a single form. For compounds with no ionizable groups, log P will be equal to log D. For compounds with ionizable groups, e.g. crocetin, log P is not equal to log D. Like log D, log P may be measured in a shake-flask method, except by measuring the partition of the compound between octanol and water. For example, octanol may be added to the test compound. Water may then be added to the octanol. A ratio of 2:1 v/v water to octanol may be prepared. The system may be mixed to allow distribution of the compound between the two phases. After separation, the compound is quantified in the water and octanol by LC-MS/MS and the following equation is used to calculate the Log P.

$$\text{Log P} = \text{Log}\, [\frac{C_{oct}}{C_{water}}]$$

Where:$C_{oct}$ = concentration in octanol sample, and $C_{water}$ = concentration in water. Log P values are also available from computer software tools. In the Examples of the present disclosure, the modelling software GastroPlus® (ADMET Predictor Module, available from SimulationsPlus) was used.

In the present disclosure, it was found that piperine increased the blood-brain barrier permeation of p-glycoprotein substrate compounds having a log P value from about -2.5 to about 5.0. In preferred embodiments, the log P value of the at least one p-glycoprotein substrate compound is from about -1.0 to about 4.0. In particularly preferred embodiments, the log P value is from about -1.0 to about 3.5. The Examples demonstrate that ursolic acid (log P = 4.89), a lipophilic compound, was not enhanced by piperine.

In some embodiments, the at least one p-glycoprotein substrate compound has a log D value at pH 7.4 from about -2.5 to about 4.5 and a log P value from about -2.5 to about 5.0. In some embodiments, the at least one p-glycoprotein substrate compound has a log D value at pH 7.4 from about -2.5 to about 4.0, and a log P value from about -1.0 to about 4.0.

In some embodiments, the at least one p-glycoprotein substrate compound has a log D value at pH 7.4 either from about -2.0 to about 0.5, or from about 3.0 to about 3.5, and a log P value from about -1.0 to about 3.5. In any of these embodiments the at least one p-glycoprotein substrate compound may be derived from a botanical as defined above. Piperine or a geometric isomer thereof may also be used in an amount from about 0.0001 wt% to about 0.5 wt%, based on the total weight of the oral product.

In some embodiments the log P to log $D_{7.4}$ ratio of the at least one p-glycoprotein substrate compound is 1 or more, such as 1 to 25. In preferred embodiments, the log P to log $D_{7.4}$ ratio is 1 meaning that the at least one p-glycoprotein substrate compound is non-ionizable.

In some embodiments the at least one p-glycoprotein substrate compound may be defined by its molar mass in g/mol. The molar mass may be calculated from the standard atomic weights of the elements present in the compound. It is also commonly reported in the literature. In some embodiments the at least one p-glycoprotein substrate compound has a molar mass of greater than about 300 g/mol. In preferred embodiments the at least one p-glycoprotein substrate compound has a molar mass of greater than about 375 g/mol. In particularly preferred embodiments the at least one p-glycoprotein substrate compound has a molar mass of greater than about 400 g/mol.

The upper limit is not particularly limited in the present disclosure. It may be controlled by the other parameters defined herein, such as the log $D_{7.4}$ and/or log P value. In some embodiments, however, the at least one p-glycoprotein substrate compound has a molar mass of no more than about 1500 g/mol. In preferred embodiments, the at least one p-glycoprotein substrate compound has a molar mass of no more than about 1200 g/mol. In particularly preferred embodiments, the at least one p-glycoprotein substrate compound has a molar mass of no more than about 1000 g/mol.

In some embodiments, the at least one p-glycoprotein substrate compound has a molar mass of greater than about 300 g/mol and no more than about 1500 g/mol. In some embodiments the at least one p-glycoprotein substrate compound has a molar mass of greater than about 300 g/mol and no more than about 1200 g/mol. In some embodiments the at least one p-glycoprotein substrate compound has a molar mass of greater than about 300 g/mol and no more than about 1000 g/mol.

In preferred embodiments, the at least one p-glycoprotein substrate compound has a molar mass of greater than 375 g/mol and no more than about 1500 g/mol. In other preferred embodiments, the at least one p-glycoprotein substrate compound has a molar mass of greater than 375 g/mol and no more than about 1200 g/mol. In other preferred embodiments, the at least one p-glycoprotein substrate compound has a molar mass of greater than 375 g/mol and no more than about 1000 g/mol.

In particularly preferred embodiments, the at least one p-glycoprotein substrate compound has a molar mass of greater than 400 g/mol and no more than about 1500 g/mol. In other particularly preferred embodiments, the at least one p-glycoprotein substrate compound has a molar mass of greater than 400 g/mol and no more than about 1200 g/mol. In other particularly preferred embodiments, the at least one p-glycoprotein substrate compound has a molar mass of greater than 400 g/mol and no more than about 1000 g/mol.

As the skilled person will understand, these molar mass ranges may be combined with the above definitions of log D values at pH 7.4 and/or log P values. These combinations are supported by the Examples herein. In any of these embodiments the at least one p-glycoprotein substrate compound may be derived from a botanical as defined above. Piperine or a geometric isomer thereof may also be used in an amount from about 0.0001 wt% to about 0.5 wt%, based on the total weight of the oral product.

The amount of the at least one p-glycoprotein substrate compound is not limited. In some embodiments, however, it may be defined as from about 0.0001 wt% to about 0.1 wt%, based on the total weight of the oral product. In preferred embodiments the at least one p-glycoprotein substrate compound may be present in an amount from about 0.0005 wt% to about 0.05 wt%, based on the total weight of the oral product. These amounts may be lower than an oral product without piperine or a geometric isomer thereof because of the enhancement of blood-brain barrier permeation underlying the present disclosure.

In some embodiments, the oral product comprises from about 0.0001 wt% to about 0.5 wt% piperine or a geometric isomer thereof, and from about 0.001 wt% to about 0.1 wt% of the at least one p-glycoprotein substrate compound as defined herein, the wt% being based on the total weight of the oral product. In preferred embodiments, the oral product comprises from about 0.0005 wt% to about 0.1 wt% piperine or a geometric isomer thereof, and from about 0.0005 wt% to about 0.05 wt% of the at least one p-glycoprotein substrate compound. In particularly preferred embodiments, the oral product comprises from about 0.001 wt% to about 0.05 wt% piperine or a geometric isomer thereof, and from about 0.0005 wt% to about 0.05 wt% of the at least one p-glycoprotein substrate compound. As the skilled person will understand, these ranges may be combined with the above definitions of log D values at pH 7.4 and/or log P values and/or molar mass. These combinations are supported by the Examples herein. In any of these embodiments the at least one p-glycoprotein substrate compound may be derived from a botanical as defined above.

In some embodiments at least one p-glycoprotein substrate compound may be derived from ginseng. Ginseng is the root of plants in the genus Panax, which are characterised by the presence of unique steroid saponin phytochemicals (ginsenosides) and gintonin. The putative major active components of ginseng comprise more than 100 species specific

triterpene saponins or 'ginsenosides'. Ginseng and ginseng extracts also contain a range of other potentially bioactive components, including alkaloids, phytosterols, sesquiterpenes, and polyphenols. Ginseng finds use as a dietary supplement in energy drinks or herbal teas, and in traditional medicine.

The ginseng may include any suitable form of ginseng, such as Panax ginseng (Korean ginseng), Panax notoginseng (China ginseng) and Panax quinquefolius (American ginseng). The ginseng may also comprise Ashwagandha (Withania somnifera), commonly known as Indian Ginseng. The ginseng may be present in the form of ginseng extract, chopped ginseng, shredded ginseng or powdered ginseng. Preferably, the ginseng is included in the form of ginseng extract or powdered ginseng extract. The ginseng may white ginseng, fresh ginseng or red ginseng. In some embodiments, the ginseng is red ginseng, such as red ginseng extract or powdered red ginseng extract. The inclusion of ginseng has been found to be beneficial in improving cognitive effects of a product, and increasing the boost in immune response or immunity in the user. Ginseng is, for example, known as an immune modulator and has been used for maintaining immune homeostasis and enhancing resistance to illness or microbial attacks through effects on the immune system. Ginseng also has anti-inflammatory and antioxidant properties.

The ginseng may be present in the liquid oral product in any suitable amount, such as in an amount of at least about 0.01 % by weight, at least about 0.05% by weight or at least about 0.1% by weight of the oral product. In some embodiments, the ginseng may be present in an amount of no greater than about 5% by weight, no greater than about 2% by weight, or no greater than about 1% by weight of the oral product. In some embodiments, the ginseng is present in an amount of from about 0.01% to about 5% by weight of the oral product, such as in an amount of from about 0.05% to about 2% by weight of the oral product. In preferred embodiments, the ginseng is present in an amount of from about 0.1% to about 1% by weight of the oral product.

In any of these embodiments, the oral product may comprise from about 0.0001 wt% to about 0.5 wt% piperine or a geometric isomer thereof, and from about 0.001 wt% to about 0.1 wt% of the at least one p-glycoprotein substrate compound as defined herein, the wt% being based on the total weight of the oral product.

In some embodiments, the ginseng is present in an amount of from about 10 mg to about 600 mg, such as from about 20 mg to about 550 mg. In preferred embodiments, the ginseng is present in an amount of from about 50 mg to about 500 mg. In any of these embodiments, the oral product may further comprise from about 5 mg to about 20 mg piperine or a geometric isomer thereof.

In preferred embodiments the ginseng is Indian ginseng or Ashwagandha and the at least one p-glycoprotein substrate compound is selected from withanolide A and withanone. Withanolide A and withanone have the following chemical structures:

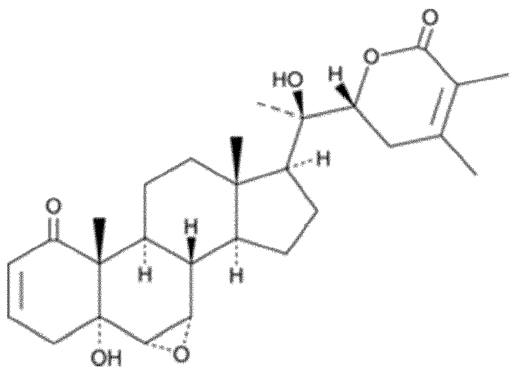

Withanolide A

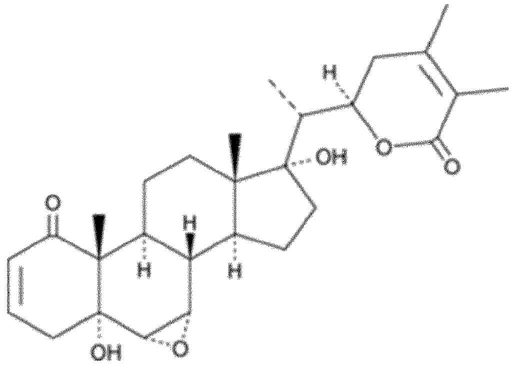

Withanone

In some embodiments at least one p-glycoprotein substrate compound may be derived from saffron. Saffron is a spice derived from the flower of *Crocus sativus;* its taste and fragrance result from the phytochemicals picrocrocin and safranal, and its most biologically active compounds are crocetin and its glycoside crocin. Chemically, crocin is the diester formed from the disaccharide gentioboise and the dicarboxylic acid crocetin. Like ginseng, saffron finds use as a dietary supplement in energy drinks or herbal teas, and in traditional medicine, it has been known for many years to provide therapeutic benefits. The saffron may be present in any form and as any type. For example, in the form of saffron extract, chopped saffron, shredded saffron or powdered saffron. Preferably, the saffron is included in the form of an extract or a powder.

The saffron may be present in the liquid oral product in any suitable amount, such as in an amount of at least about 0.001 % by weight, at least about 0.005% by weight or at least about 0.01% by weight of the oral product. In some embodiments, the saffron may be present in an amount of no greater than about 5% by weight, no greater than about 2% by weight, or no greater than about 1% by weight of the oral product. In some embodiments, the saffron is present in an amount of from about 0.001% to about 5% by weight of the oral product, such as in an amount of from about 0.005% to about 2% by weight of the oral product. In preferred embodiments, the saffron is present in an amount of from about 0.01% to about 1% by weight of the oral product.

In any of these embodiments, the oral product may comprise from about 0.0001 wt% to about 0.5 wt% piperine or a geometric isomer thereof, and from about 0.001 wt% to about 0.1 wt% of the at least one p-glycoprotein substrate compound as defined herein, the wt% being based on the total weight of the oral product.

In some embodiments, the saffron is present in an amount of from about 1 mg to about 100 mg, such as from about 2 mg to about 80 mg. In preferred embodiments, the saffron is present in an amount of from about 5 mg to about 50 mg. In any of these embodiments, the piperine or a geometric isomer thereof may be present in an amount of from about 5 mg to about 20 mg.

In preferred embodiments the at least one p-glycoprotein substrate compound is derived from saffron and selected from crocetin and crocin, preferably the compound is crocin. Crocetin and crocin have the following chemical structures:

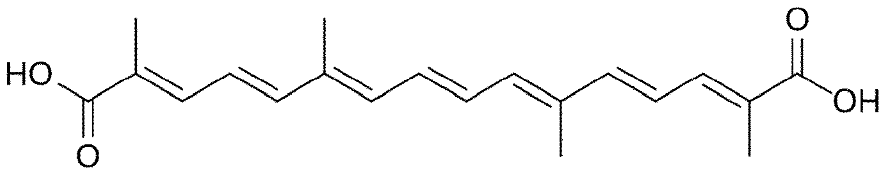

Crocetin

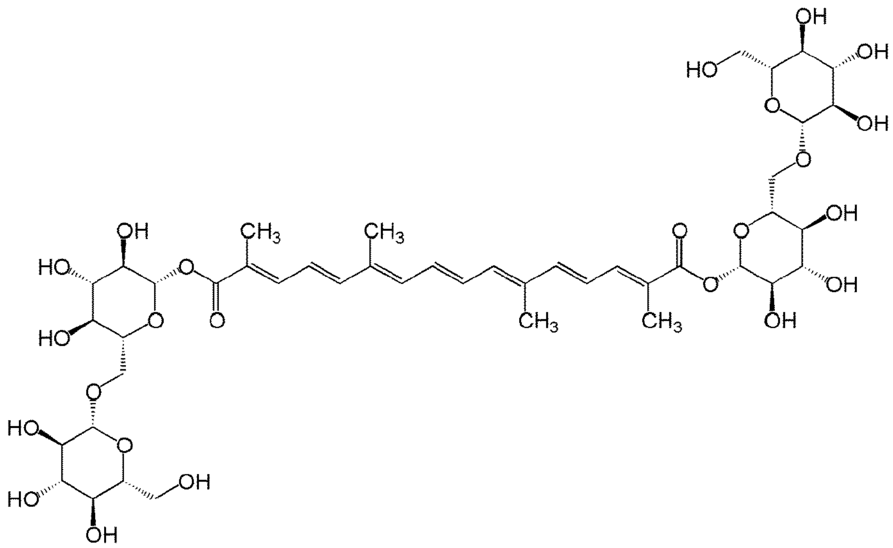

Crocin

In some embodiments, the at least one glycoprotein substrate compound is selected from crocetin, crocin, withanolide A, withanone, or a combination thereof. In preferred embodiments, the at least one glycoprotein substrate compound is selected from crocin, withanolide A, withanone, or a combination thereof. In particularly preferred embodiments, the at least one glycoprotein substrate compound is selected from withanolide A, withanone, or a combination thereof.

In some embodiments, the at least one glycoprotein substrate compound is selected from withanolide A, withanone, or a combination thereof, and the oral product comprises ginseng in the form of Ashwagandha in an amount of from about 10 mg to about 600 mg, and piperine or a geometric isomer thereof in an amount of from about 5 mg to about 20 mg piperine or a geometric isomer thereof.

In some embodiments, the at least one glycoprotein substrate compound is selected from crocetin, crocin, or a combination thereof, and the oral product comprises saffron in an amount of from about 1 mg to about 100 mg, and piperine or a geometric isomer thereof in an amount of from about 5 mg to about 20 mg piperine or a geometric isomer thereof.

<u>Other active ingredients</u>

In some embodiments the oral product may comprise additional active ingredients to piperine or a geometric isomer thereof and the at least one p-glycoprotein substrate compound. The additional active ingredients may be any suitable active ingredient that has a biological response in a human or animal. The additional active ingredient may be naturally occurring or synthetically obtained.

Non-limiting examples of additional active ingredients include those falling in the categories of botanical ingredients, amino acids, nicotine components, and/or nutraceutical, and medicinal ingredients (e.g., vitamins, such as A, B1, B2, B3, B5, B6, B7, B9, B12, and/or cannabinoids, such as tetrahydrocannabinol (THC) and cannabidiol (CBD)). The additional active ingredient may comprise for example taurine, theanine, vitamins such as B vitamins (e.g. B1, B2, B3, B5, B6, B7, B9 and/or B12), vitamin A, vitamin E, or vitamin K, melatonin, gamma-aminobutyric acid (GABA), cannabinoids, or constituents, derivatives, or combinations thereof.

In some embodiments, the one or more additional active ingredients is selected from a botanical ingredient (e.g., lavender, peppermint, chamomile, basil, rosemary, ginger, maca, lemon balm, and tisanes), an amino acid (e.g., taurine, phenylalanine, tyrosine, GABA, and tryptophan), a cannabinoid, and/or a nutraceutical, or medicinal ingredient (e.g., a vitamin, such as a B vitamin).

In some embodiments, the combination of active ingredients further comprises lemon balm. Lemon balm is also referred to as *Melissa officinalis* and is a perennial herbaceous plant in the mint family. Lemon balm contains a complex mixture of terpenes, terpenoids, flavonoids, polyphenols, and other molecules. The lemon balm may be in the form of leaves, stems or roots from the lemon balm plant. In some embodiments, the lemon balm is included in the form of lemon balm leaves, such as chopped, shredded or powdered lemon balm leaves. In some embodiments, the lemon balm is included in the form of a lemon balm extract, such as an aqueous extract of lemon balm. In some embodiments, the lemon balm is included in the form of lemon balm essential oil. Lemon balm has previously been found to exert antioxidant, anti-inflammatory and neuroprotective properties.

The lemon balm may be present in any suitable amount, such as in an amount of at least about 0.01% by weight, at least 0.05% by weight or at least 0.1% by weight of the oral product. In some embodiments, the lemon balm is present in an amount of no greater than about 3% by weight or no greater than about 2% by weight of the oral product. In some embodiments, the lemon balm is present in an amount of from about 0.01% to about 3% by weight of the oral product, such as in an amount of from about 0.05% by weight to about 3% by weight. In preferred embodiments, the lemon balm is present in an amount of from about 0.1% to about 2% by weight of the oral product.

In some embodiments, the combination of active ingredients further comprises chamomile. Chamomile (Matricaria recuita) is a flowering plant in the daisy (Asteraceae) family. Native to Europe and Western Asia, it is now found around the world. There are two different chamomile plants, German chamomile and Roman chamomile. German chamomile is generally considered the more potent variety and the type most widely used for medicinal purposes. Different classes of bioactive constituents are present in chamomile, such as flavanols (apigenin, luteolin, quercetin) coumarins and terpenoids. Research has shown chamomile to have benefits when it comes to boosting immunity or the immune system to help fight infections. Chamomile is also known to have anti-inflammatory and anti-oxidant effects.

The chamomile may be in the form of chamomile extract. The chamomile (e.g. chamomile extract) may be present in an amount of at least about 0.001% by weight, at least about 0.005% by weight or at least about 0.01% by weight of the oral product. In some embodiments, the chamomile (e.g. chamomile extract) is present in an amount of no greater than about 1% by weight, no greater than about 0.5% by weight, or no greater than about 0.1% by weight of the oral product.

The combination of active ingredients may further comprise a combination of at least two B vitamins selected from vitamin B1, vitamin B2, vitamin B3, vitamin B5, vitamin B6, vitamin B7, vitamin B9 and vitamin B12. Preferably the combination of B vitamins comprises vitamin B2 and vitamin B6, more preferably the combination of B vitamins further comprises one or more of vitamin B1 or vitamin B7.

The term "B vitamins" includes a group of eight water-soluble vitamins that have effect in cellular functioning; e.g. cell metabolism and synthesis of red blood cells. B vitamins act as coenzymes in a substantial proportion of the enzymatic processes for cellular physiological functioning. The B vitamins may generally be divided into two categories: those that act as co-enzymes for catabolic enzymatic reactions (leading to generation of energy) and those that act as co-enzymes for anabolic enzymatic reactions (leading to enhanced brain function).

B vitamins are considered to have roles in aspects of brain function and energy production. The eight water soluble B vitamins are vitamin B1 (thiamin), vitamin B2 (riboflavin), vitamin B3 (niacin), vitamin B5 (panthothenic acid or salt thereof, e.g. calcium pantothenate), vitamin B6 (pyridoxine hydrochloride), vitamin B7 (biotin), vitamin B9 (folate or folic acid), vitamin B12 (cyanocobalamin). Vitamins B2, B3, B6, B9 and B12 have been found to support anabolic metabolism and to boost brain function. Vitamins B1, B2, B3, B5 and B7 have been found to support catabolic metabolism and to boost energy levels.

In preferred embodiments, the oral product includes at least two of the B vitamins that support anabolic metabolism. In other words, according to a first aspect, the combination of at least two B vitamins is selected from vitamins B2, B3, B6, B9 and B12. It has been found that the inclusion of at least these B vitamins may reduce perceived physical and mental fatigue as well as enhance psychological and cognitive functions. In particularly preferred embodiments, the oral product includes all five B vitamins that support anabolic metabolism. In other words, according to a first aspect, the combination of active ingredients includes a combination of vitamins B2, B3, B6, B9 and B12. Each of these B vitamins may contribute to the reduction of tiredness and fatigue, and the inclusion of all five B vitamins may lead to a synergistic effect in this regard.

In some embodiments, the combination of B vitamins consists essentially of all anabolic vitamins; i.e. a combination of vitamins B2, B3, B6, B9 and B12. In some embodiments, the combination of B vitamins consists of all anabolic vitamins; i.e. a combination of vitamins B2, B3, B6, B9 and B12. The term "anabolic B vitamin" refers to those B vitamins that have an effect on anabolic pathways, while the term "catabolic B vitamin" refers to those B vitamins that have an effect on catabolic pathways.

In some embodiments, the combination of B vitamins further comprises at least one catabolic B vitamin selected from the group consisting of vitamin B1, vitamin B5, vitamin B7 and combinations thereof.

In any such embodiments, the ratio of the total amount of anabolic B vitamins (i.e. vitamins B2, B3, B6, B9 and B12) to the total amount of catabolic B vitamins (i.e. vitamins B1, B5 and B7) may be from about 5:1 to about 1:5, preferably from about 2:1 to about 1:2 and more preferably from about 2:1 to about 1:1.

In other embodiments, the combination of B vitamins may comprise at least vitamin B1 and vitamin B2, or at least vitamin B6 and vitamin B7, or at least vitamin B1 and B6, or at least vitamin B2 and vitamin B7, preferably the combination of B vitamins may comprise at least vitamin B1, vitamin B2 and vitamin B6. In such embodiments, the combination of B vitamins may comprise at least vitamin B1, vitamin B2, vitamin B6, and vitamin B7, preferably the combination of B vitamins may consist essentially of vitamin B1, vitamin B2, and vitamin B6, and vitamin B7.

The amounts of the B vitamins included in the composition may be any amount suitable to provide the desired effect while also providing a product that is safe to consume and has reduced side effects. The total amount of B vitamins included in the combination may be any suitable amount to provide the desired effect of boosting brain function. In some

embodiments, the total amount of anabolic B vitamins (i.e. the total amount of the combination of vitamins B2, B3, B6, B9 and B12) may be at least about 0.0001% by weight, preferably at least about 0.001% by weight, and more preferably at least about 0.01% by weight of the oral product. For example, the total amount of anabolic B vitamins may be from about 0.0001% to about 5% by weight of the oral product, and preferably from about 0.001% to about 1%. For example, this amount may be from about 0.01% to about 0.5% by weight of the oral product, or from about 0.05% to about 0.5% by weight of the oral product.

Where present, the total amount of catabolic B vitamins (i.e. the total amount of the combination of vitamins B1, B5 and B7) may be at least about 0.0001% by weight, preferably at least about 0.001% by weight, and more preferably at least about 0.01% by weight of the oral product. For example, the total amount of catabolic B vitamins (e.g. where the catabolic B vitamins are vitamin B1, vitamin B2, vitamin B3, vitamin B5 and/or vitamin B7) may be from about 0.0001% to about 5% by weight of the oral product, and preferably from about 0.001% to about 1%. For example, this amount may be from about 0.01% to about 0.5% by weight of the oral product.

The total amount of B vitamins included in the combination may be any suitable amount to provide the desired effect of boosting brain function. In some embodiments, the total amount of B vitamins is from about 0.001% to about 10% by weight, and preferably from about 0.001% to about 5% by weight of the oral product. In some embodiments, the total amount of B vitamins may be from about 0.01% to about 4% by weight, such as from about 0.025% to about 3% by weight, preferably from about 0.05% to about 2% by weight of the oral product.

In some embodiments, vitamin B2 may be present in an amount of from about 1% to about 20% by weight of the total amount of B vitamin in the oral product. For example, vitamin B2 may be present in an amount of from about 5% to about 15% by weight of the total amount of B vitamin in the oral product. In other embodiments, vitamin B2 may be present in an amount of from about 1% to about 40% by weight of the total amount of B vitamin in the oral product. For example, vitamin B2 may be present in an amount of from about 10% to about 40% by weight of the total amount of B vitamin in the oral product.

In some embodiments, vitamin B3 may be present in an amount of from about 20% to about 80% by weight of the total amount of B vitamin in the oral product. For example, vitamin B3 may be present in an amount of from about 40% to about 70% by weight, and preferably from about 45% to about 60% by weight of the total amount of B vitamin in the oral product. In other embodiments, the combination of B vitamins do not include vitamin B3.

In some embodiments, vitamin B6 may be present in an amount of from about 1% to about 15% by weight of the total amount of B vitamin in the oral product. For example, vitamin B6 may be present in an amount of from about 5% to about 10% by weight of the total amount of B vitamin in the oral product. In other embodiments, vitamin B6 may be present in an amount of from about 1% to about 50% by weight of the total amount of B vitamin in the oral product. For example, vitamin B6 may be present in an amount of from about 5% to about 50% by weight of the total amount of B vitamin in the oral product.

In some embodiments, vitamin B9 may be present in an amount of from about 0.1% to about 10% by weight of the total amount of B vitamin in the oral product. For example, vitamin B9 may be present in an amount of from about 1% to about 5% by weight of the total amount of B vitamin in the oral product.

In some embodiments, vitamin B12 may be present in an amount of from about 0.01% to about 30% by weight, such as from about 0.1% to about 25% by weight, such as from about 1% to about 20% by weight of the total amount of B vitamin in the oral product. For example, vitamin B12 may be present in an amount of from about 5% to about 15% by weight of the total amount of B vitamin in the oral product.

Where present, vitamin B1 may be present in an amount of from about 1% to about 10% by weight of the total amount of B vitamin in the oral product. For example, where present, vitamin B1 may be present in an amount of from about 2.5% to about 7.5% by weight of the total amount of B vitamin in the oral product. In some embodiments, the oral product may be substantially free of (i.e. include no more than 0.0001% by weight of) vitamin B1, or may be free of (i.e. include 0%) vitamin B1. In other embodiments, vitamin B1 may be present in an amount of from about 1% to about 40% by weight of the total amount of B vitamin in the oral product. For example, vitamin B1 may be present in an amount of from about 5% to about 35% by weight of the total amount of B vitamin in the oral product.

Where present, vitamin B5 may be present in an amount of from about 1% to about 20% by weight of the total amount of B vitamin in the oral product. For example, where present, vitamin B5 may be present in an amount of from about 5% to about 15% by weight of the total amount of B vitamin in the oral product. In some embodiments, the oral product may be substantially free of vitamin B5, or may be free of vitamin B5.

Where present, vitamin B7 may be present in an amount of from about 0.01% to about 5% by weight of the total amount of B vitamin in the oral product. For example, where present, vitamin B7 may be present in an amount of from about 0.01% to about 1% by weight or about 0.01% to about 2% by weight of the total amount of B vitamin in the oral product. In some embodiments, the oral product may be substantially free of vitamin B7, or may be free of vitamin B7.

The oral product may be caffeine-free, or may include caffeine in the combination of active ingredients. Caffeine is a central nervous system stimulant. Caffeine (1,3,7-trimethylxanthine) exists as a bitter substance, an alkaloid which is naturally found in a variety of plant species, including tea leaves, kola nuts, cocoa, guarana, and coffee beans. Once

ingested, caffeine is metabolised through the liver *via* the cytochrome p450 enzyme system and is capable of entering all body tissues, as well as crossing the blood brain barrier. Caffeine may be used to enhance alertness, attention, cognitive performance as well as physical performance.

The caffeine may be included as a natural stimulant, meaning that it is naturally derived. By "naturally derived" is meant that the caffeine is in a purified form, outside its natural (e.g. botanical) matrix. For example, caffeine may be obtained by extraction and purification from botanical sources (e.g. tea). Alternatively or in addition, the caffeine may be provided in synthetic form; i.e. obtained by chemical synthesis. The caffeine may be provided in the form of an extract or in the form of a powder (e.g. a powder extract). For example, the caffeine may be included in the form of a green tea powder extract, black tea powder extract. The caffeine may also be provided in the form of guarana seed powder extract. In some embodiments, the caffeine is present in an encapsulated form. An example of an encapsulated caffeine is Vitashure®, available from Balchem Corp., 52 Sunrise Park Road, New Hampton, NY, 10958.

The caffeine may be present in any suitable amount, such as in an amount of at least about 0.01% by weight, at least about 0.1% by weight, at least about 1% by weight, or at least 2% by weight of the oral product. In some embodiments, the caffeine may be present in an amount of no greater than about 35% by weight, no greater than about 20% by weight, no greater than about 10% by weight, or no greater than about 5% by weight of the oral product.

The caffeine may be present in an amount of from about 0.01% to about 35% by weight of the oral product. In some embodiments, the caffeine is present in an amount of from about 0.1% to about 20% by weight of the oral product. The caffeine may preferably be present in an amount of from about 0.1% to about 10% by weight of the oral product. Particularly preferred is an amount of caffeine from about 0.1% to about 5% by weight of the oral product. The caffeine may alternatively be present in an amount of from about 1% to about 35% by weight.

In some embodiments the combination of active ingredients may include beta-alanine. Beta-alanine or 3-aminopropanoic acid is a naturally occurring beta amino acid. There is evidence that beta-alanine supplements can increase energy and cognitive performance as well as prevent muscle soreness and fatigue. Beta-alanine is converted within muscle cells to carnosine which acts as a buffer for the lactic acid produced during high intensity exercise, and can thereby help delay the onset of neuromuscular fatigue.

The beta-alanine may be present in any suitable amount, such as in an amount of at least about 0.01% by weight, or at least about 0.1% by weight of the oral product, or at least about 1% by weight of the oral product. In some embodiments, the beta-alanine may be present in an amount of no greater than about 60% by weight, no greater than about 50% by weight, or no greater than about 10% by weight of the oral product.

The beta-alanine may be present in an amount of from about 0.01% to about 60% by weight of the oral product. In some embodiments, the beta-alanine is present in an amount of from about 0.1% to about 50% by weight of the oral product. In other embodiments (for example when the oral product is in liquid dosage form), the beta-alanine is present in an amount of from about 1% to about 10% by weight of the oral product.

In some embodiments the combination of active ingredients may comprise L-theanine. L-theanine is an amino acid analogue that is also referred to as L-γ-glutamylethylamide and $N^5$-ethyl-L-glutamine. It is a water-soluble amino acid isolated from green tea (*Camellia sinensis*) that has anti-inflammatory activity, antioxidative properties and hepatoprotective effect. The inclusion of L-theanine may improve the effects of the oral product on the immune system. For example, the inclusion of L-theanine may help to regulate immune response, prevent disease, and reduce anti-oxidation stress. Studies have shown that L-theanine can enhance innate immune function by regulating the secretion of immune cytokines.

The L-theanine may be present in the oral product in any suitable amount, such as in an amount of at least about 0.01% by weight, at least about 0.05% by weight, or at least about 0.1% by weight of the oral product. In some embodiments, the L-theanine may be present in an amount of no greater than about 15% by weight, no greater than about 10% by weight, or no greater than about 8% by weight of the oral product.

In some embodiments, the L-theanine may be present in an amount of from about 0.01% to about 15% by weight of the oral product, such as in an amount of from about 0.05% to about 10% by weight of the oral product. In preferred embodiments, the L-theanine is present in an amount of from about 0.1% to about 8% by weight of the oral product.

The combination of active ingredients further comprises vitamin C (ascorbic acid). Vitamin C acts as an antioxidant, helping to protect cells from the damage caused by free radicals. It is also used by the body to make collagen, a protein that may help wounds heal. In addition, vitamin C improves the absorption of iron from plant-based foods and helps the immune system work properly to protect the body from disease. The inclusion of vitamin C may reduce tiredness and fatigue.

The vitamin C may be present in the oral product in any suitable amount, such as in an amount of at least about 0.01% by weight, at least about 0.05% by weight, or at least about 0.1% by weight of the oral product. In some embodiments, the vitamin C may be present in an amount of no greater than about 10% by weight, no greater than about 8% by weight, or no greater than about 5% by weight of the oral product.

In some embodiments, the vitamin C may be present in an amount of from about 0.01% to about 10% by weight, such as from about 0.05% to about 8% by weight, preferably in an amount of from about 0.1% to about 5% by weight of the

oral product.

The combination of active ingredients may further comprise vitamin D. Vitamin D is a group of fat-soluble secosteroids responsible for increasing intestinal absorption of calcium, magnesium, and phosphate. In humans the most important compounds in this group are vitamin $D_3$ (cholecalciferol) and $D_2$ (ergocalciferol). Vitamin D as used herein thus refers to $D_2$, $D_3$ or both. The structural difference between vitamin $D_2$ and $D_3$ is in the side chain, which contains a double bond, between carbons 22 and 23, and a methyl group on carbon 24 in vitamin $D_2$. As well as calcium, magnesium, and phosphate absorption, vitamin D affects the immune system and vitamin D receptors (VDRs) are expressed in several white blood cells, including monocytes and activated T and B cells. In general, vitamin D functions to activate the innate and dampen the adaptive immune systems with antibacterial, antiviral and anti-inflammatory effects.

When present in the oral product, the vitamin D may be used in any suitable amount, such as in an amount of at least about 0.0000001% by weight, at least about 0.0000025% by weight or at least about 0.00001% by weight of the oral product. In some embodiments, the vitamin D may be present in an amount of no greater than about 0.1% by weight, no greater than about 0.01% by weight, no greater than about 0.005% by weight, or no greater than about 0.001% by weight of the oral product.

In some embodiments, the vitamin D is present in an amount of from about 0.000001% to about 0.1% by weight of the oral product, such as in an amount of from about 0.0000025% to about 0.005% by weight of the oral product. In preferred embodiments, the vitamin D is present in an amount of from about 0.00001% to about 0.001% by weight of the oral product.

The combination of active ingredients may further comprise selenium. Selenium is a trace element that is nutritionally essential for humans as a component of multiple selenoproteins that play critical roles in reproduction, thyroid hormone metabolism, DNA synthesis, and protection from oxidative damage and infection. When present in the oral product, the selenium may be used in any suitable amount such as in an amount of at least 0.0000001% by weight, at least about 0.0000005% by weight, or at least about 0.000001% by weight of the oral product. In some embodiments, the selenium may be present in an amount of no greater than about 0.001% by weight, no greater than about 0.0005% by weight, or no greater than about 0.0001% by weight. In some embodiments, the selenium may be provided in the form of one or more inorganic forms (e.g. selenate and selenite salts), one or more organic forms (e.g. selenomethionine and selenocysteine) and/or mixtures thereof.

In some embodiments, selenium is present in an amount of from about 0.0000001% by weight to about 0.001% by weight of the oral product, such as in an amount of from about 0.0000005% by weight to about 0.0005% by weight of the oral product. In preferred embodiments, the selenium is present in an amount of from about 0.000001% by weight to about 0.0001% by weight of the oral product.

The combination of active ingredients may further comprise Echinacea. The Echinacea is either *Echinacea purpurea, Echinacea angustifolia* or *Echinacea pallida* and the upper parts (e.g. flowers, fruit, leaves) and/or roots of the plant may be utilized. Echinacea plants contain a variety of active compounds including caffeic acid, alkamides, phenolic acids, rosmarinic acid, polyacetylenes, flavonoids, and cichoric acid. Studies have linked Echinacea and its compounds to many health benefits including reduced inflammation, improved immunity and lower blood sugar levels. The Echinacea may be present in the form of an extract, chopped or shredded or powdered. Preferably, the Echinacea is included in the form of an extract.

The Echinacea may be present in the oral product in any suitable amount, such as in an amount of at least about 0.001% by weight, at least about 0.01% by weight or at least about 0.05% by weight of the oral product. In some embodiments, the Echinacea may be present in an amount of no greater than about 20% by weight, no greater than about 15% by weight, or no greater than about 10% by weight of the oral product.

In some embodiments, the Echinacea is present in an amount of from about 0.001% to about 20% by weight of the oral product, such as in an amount of from about 0.01% to about 15% by weight of the oral product. In preferred embodiments, the Echinacea is present in an amount of from about 0.05% to about 10% by weight of the oral product.

The combination of active ingredients may further comprise a vitamin selected from vitamin A, vitamin E and vitamin K, or mixtures thereof.

The combination of active ingredients may further comprise an amino acid, such as an amino acid selected from the group consisting of alanine, arginine, asparagine, aspartic acid, cysteine, glutamine, glutamic acid, glycine, histidine, isoleucine, leucine, lysine, methionine, phenylalanine, proline, serine, threonine, tryptophan, tyrosine, valine, gamma-aminobutyric acid (GABA), taurine (2-aminoethanesulfonic acid), hydroxyproline, and beta-alanine. In some embodiments, the combination of active ingredients comprises GABA.

In some embodiments, the combination of active ingredients comprises a cannabinoid. The cannabinoid may be a derivative or extract of cannabis. Cannabinoids are a class of natural or synthetic chemical compounds which act on cannabinoid receptors (i.e., CB1 and CB2) in cells that repress neurotransmitter release in the brain. Cannabinoids are cyclic molecules exhibiting particular properties such as the ability to easily cross the blood-brain barrier. Cannabinoids may be naturally occurring (Phytocannabinoids) from plants such as cannabis, (endocannabinoids) from animals, or artificially manufactured (synthetic cannabinoids). Cannabis species express at least 85 different phytocannabinoids, and

these may be divided into subclasses, including cannabigerols, cannabichromenes, cannabidiols, tetrahydrocannabinols, cannabinols and cannabinodiols, and other cannabinoids. In some embodiments,, the cannabinoid is selected from the group consisting of cannabigerol (CBG), cannabichromene (CBC), cannabidiol (CBD), tetrahydrocannabinol (THC), cannabinol (CBN) and cannabinodiol (CBDL), cannabicyclol (CBL), cannabivarin (CBV), tetrahydrocannabivarin (THCV), cannabidivarin (CBDV), cannabichromevarin (CBCV), cannabigerovarin (CBGV), cannabigerol monomethyl ether (CBGM), cannabinerolic acid, cannabidiolic acid (CBDA), Cannabinol propyl variant (CBNV), cannabitriol (CBO), tetrahydrocannabimolic acid (THCA), tetrahydrocannabivarinic acid (THCV A), and mixtures thereof. In some embodiments, the cannabinoid is or comprises at least tetrahydrocannabinol (THC). In some embodiments, the cannabinoid is or comprises at least cannabidiol (CBD).

In some embodiments, the combination of active ingredients comprises a cannabinoid (such as cannabidiol) in an amount of at least about 0.001% by weight of the oral product, such as in a range from about 0.001% to about 10% by weight, such as from about 0.01% to about 5% by weight, such as from about 0.1% to about 2.5% by weight, such as from 0.5% to about 1% by weight of the oral product.

In some embodiments, the combination of active ingredients comprises magnesium glycinate. Where present, magnesium glycinate may be included in an amount of from about 0.1% to about 10% by weight, such as from about 0.5% to about 5% by weight, or from about 0.5% to about 1% by weight of the oral product.

In any of the embodiments defined herein, the term "comprises" may be replaced with the term "consists essentially of' or "consists of'.

In some embodiments the combination of active ingredients may include the following further actives: L-theanine, vitamin C, Echinacea, and optionally vitamin D or selenium. This combination may be beneficial when combined with ginseng, e.g. Ashwagandha, as the botanical from which the at least one p-glycoprotein substrate compound is derived. The resulting combination has been found to be beneficial in improving physical wellbeing of a human or animal including boosting immune response or supporting immunity of a human or animal including supporting resilience against infection compared to a placebo product. The resulting combination has also been found to reduce sleepiness of a human or animal, and improve alertness and feelings of being in control, compared to a placebo product.

In some embodiments the combination of active ingredients may include the following further actives: caffeine, optionally a combination of B vitamins comprising at least vitamin B1, B2, B6, and B7, taurine, and optionally vitamin C. This combination may be beneficial when combined with ginseng, e.g. Ashwagandha, as the botanical from which the at least one p-glycoprotein substrate compound is derived. The resulting combinations have been found to be beneficial in increasing alertness and/or energy levels, and increasing cognitive performance, compared to a placebo product.

In some embodiments the combination of active ingredients may include the following further actives: caffeine, optionally a combination of B vitamins comprising at least vitamin B1, B2, B6, and B7, L-theanine, and optionally vitamin C. This combination may be beneficial when combined with ginseng, e.g. Ashwagandha, as the botanical from which the at least one p-glycoprotein substrate compound is derived. The resulting combinations have been found to be beneficial in increasing alertness and/or energy levels, and increasing cognitive performance, compared to a placebo product.

In some embodiments the combination of active ingredients may include the following further actives: L-theanine, lemon balm, chamomile, vitamin C, and optionally L-tryptophan. This combination may be beneficial when combined with ginseng, e.g. Ashwagandha, as the botanical from which the at least one p-glycoprotein substrate compound is derived. The resulting combinations have been found to be beneficial in providing a relaxing effect for or to provide a calming effect of a human or animal, compared to a placebo product. For example, the combination of active ingredients allows the user to restore their inner balance after moments of tension or stress, and when consumed, provide short-term mood enhancement for the user. The combination of actives may also in some instances aid sleep.

In some embodiments the combination of active ingredients may include the following further actives: maca root or an extract thereof, a combination of B vitamins comprising at least two selected from vitamin B2, B3, B6, B9 and B12, coenzyme Q10, and optionally vitamin C. This combination may be beneficial when combined with ginseng, e.g. Ashwagandha, as the botanical from which the at least one p-glycoprotein substrate compound is derived. The oral product is preferably caffeine-free. The resulting combinations have been found to be beneficial in increasing alertness and/or energy levels, and increasing cognitive performance, compared to a placebo product.

In some embodiments the combination of active ingredients may include the following further actives: caffeine, optionally a combination of B vitamins comprising at least two selected from vitamin B1, B2, B3, B5, B6, B7, B9 and B12, beta-alanine, and optionally vitamin C. This combination may be beneficial when combined with ginseng, e.g. Ashwagandha, as the botanical from which the at least one p-glycoprotein substrate compound is derived. The resulting combinations have been found to be beneficial in increasing alertness and/or energy levels, and increasing cognitive performance, compared to a placebo product.

In some embodiments the combination of active ingredients may include the following further actives: maca root or an extract thereof, a combination of B vitamins comprising at least vitamin B2, and B6, ginseng which is other than Ashwagandha, passionflower, and optionally vitamin C. This combination may be beneficial when combined with Ashwagandha as the botanical from which the at least one p-glycoprotein substrate compound is derived. The resulting

combinations have been found to be beneficial in increasing sexual drive and/or desire for sexual activity of a human or animal, compared to a placebo product.

Any of these embodiments may also include saffron, for example as a flavouring agent, and thereby include further p-glycoprotein substrate compounds in the form of crocin and crocetin.

It will be understood that all disclosure herein concerning the oral product applies to these combinations of active ingredients. The disclosure is not repeated for conciseness.

Additives

Depending on the type of oral product, the product may include one or more additional components in addition to the combination of active ingredients. For example, the oral product may further comprise an additive selected from the group consisting of a flavouring agent, sweetener, buffering agent, acidifying agent, thickener, filler, binder, humectant, preservative, salt, colouring agent, oral care additive, distintegration aid, antioxidant, water or mixtures thereof. In some embodiments, the oral product may further comprise water and one or more additives selected from the group consisting of a flavouring agent, sweetener, acidifying agent, preservative, and mixtures thereof. In other embodiments, the oral product may further comprise one or more additives selected from the group consisting of a flavouring agent, sweetener, acidifying agent, thickener, filler, binder, humectant, preservative, and mixtures thereof.

The moisture content (e.g., water content) of the oral product, prior to use by a consumer of the product, may vary according to the desired properties.

For the liquid oral product, the water content may be at least about 50%, such as at least about 60%, such as at least about 75% by weight of the oral product. Preferably, the water content of the liquid oral product may be at least about 90% by weight of the oral product. Preferably, the water content of a liquid oral product may be from about 60% to about 99.5% by weight, such as from about 75% to about 99% by weight, such as from about 80% to about 98% by weight of the oral product.

Typically, for a solid oral product, prior to insertion into the mouth of the user, the water content is less than about 60% by weight, and generally is from about 1 to about 60% by weight, for example, from about 5 to about 55% by weight, about 10 to about 50% by weight, about 20 to about 45% by weight, or about 25 to about 40% by weight of the oral product; including water amounts of at least about 5% by weight, at least about 10% by weight, at least about 15% by weight, and at least about 20% by weight of the oral product.

*Flavouring Agent*

As used herein, the term "flavouring agent" (or "flavour" or "flavourant") refers to materials which, where local regulations permit, may be used to create a desired taste, aroma or other somatosensorial sensation in a product for adult consumers. Examples of sensory characteristics that can be modified by the flavoring agent include taste, mouthfeel, moistness, coolness/heat, and/or fragrance/aroma. Flavouring agents may be natural or synthetic, and the character of the flavours imparted thereby may be described, without limitation, as fresh, sweet, herbal, confectionary, floral, fruity, or spicy.

The flavouring agent may be selected from the group consisting of naturally occurring flavour materials, botanicals, extracts of botanicals, synthetically obtained materials, or combinations thereof (e.g., tobacco, cannabis, licorice (liquorice), hydrangea, eugenol, Japanese white bark magnolia leaf, chamomile, fenugreek, clove, maple, matcha, menthol, Japanese mint, aniseed (anise), cinnamon, turmeric, Indian spices, Asian spices, herb, wintergreen, cherry, berry, red berry, cranberry, raspberry, strawberry, peach, apple, orange, mango, pineapple, clementine, lemon, lime, tropical fruit, papaya, rhubarb, grape, durian, dragon fruit, cucumber, blueberry, mulberry, citrus fruits, Drambuie, bourbon, scotch, whiskey, gin, tequila, rum, spearmint, peppermint, lavender, aloe vera, cardamom, coconut, celery, cascarilla, nutmeg, sandalwood, bergamot, geranium, khat, naswar, betel, shisha, pine, honey essence, rose oil, vanilla, lemon oil, orange oil, orange blossom, cherry blossom, cassia, caraway, cognac, jasmine, ylang-ylang, sage, fennel, wasabi, piment, ginger, coriander, coffee, hemp, a mint oil from any species of the genus Mentha, eucalyptus, star anise, cocoa, lemongrass, rooibos, flax, ginkgo biloba, hazel, hibiscus, laurel, mate, orange skin, rose, tea such as green tea or black tea, thyme, juniper, elderflower, basil, bay leaves, cumin, oregano, paprika, rosemary, saffron, lemon peel, mint, beefsteak plant, curcuma, cilantro, myrtle, cassis, valerian, pimento, mace, damien, marjoram, olive, lemon basil, chive, carvi, verbena, tarragon, limonene, thymol, camphene), flavour enhancers, bitterness receptor site blockers, sensorial receptor site activators or stimulators, sugars and/or sugar substitutes (e.g., honey, sucralose, acesulfame potassium, aspartame, saccharine, cyclamates, lactose, sucrose, glucose, fructose, sorbitol, or mannitol), and other additives such as charcoal, chlorophyll, minerals, botanicals, or breath freshening agents. They may be imitation, synthetic or natural ingredients or blends thereof. They may be in any suitable form, for example, liquid such as an oil, solid such as a powder, or gas.

In some embodiments, the flavouring agent comprises a natural flavoring, such as berry (e.g. raspberry,

blueberry or strawberry), honey, citrus (such as lemon, bergamot, orange or lime), or other botanical material.

In some embodiments, the flavouring agent comprises menthol, spearmint and/or peppermint. In some embodiments, the flavouring agent comprises flavour components of cucumber, blueberry, citrus fruits and/or redberry. In some embodiments, the flavouring agent comprises eugenol. In some embodiments, the flavouring agent comprises flavour components extracted from tobacco. In some embodiments, the flavouring agent comprises flavour components extracted from cannabis.

In some embodiments, the flavouring agent may comprise a sensate, which is intended to achieve a somatosensorial sensation which are usually chemically induced and perceived by the stimulation of the fifth cranial nerve (trigeminal nerve), in addition to or in place of aroma or taste nerves, and these may include agents providing heating, cooling, tingling, numbing effect. A suitable heat effect agent may be, but is not limited to, vanillyl ethyl ether and a suitable cooling agent may be, but not limited to eucalyptol, WS-3.

In some embodiments, the flavouring agent may comprise a terpene. In some embodiments, the flavouring agent may comprise a monoterpene and/or a diterpene and/or a sesquiterpene. In some embodiments, the flavouring agent may comprise a monoterpene. In some embodiments, the terpene is selected from pinene (alpha and beta), geraniol, linalool, limonene, carvone, eucalyptol, menthone, iso-menthone, piperitone, myrcene, beta-bourbonene, germacrene, thymol, citral, eugenol, and mixtures thereof. In some embodiments" the flavouring agent is selected from the group consisting of geraniol, citronellol, nerol, maltol, ethylmaltol, fenchol, homofuraneol, furaneol, norfuraneol, 1-octen-3-ol, borneol, linalool, farnesol, hydroxycitronellol, 3,7-dimethyloctanol, myrcenol, lavandulol, nerolidol, terpineol, alpha-terpineol, menthol, thymol, carvacrol, myrtenol, carveol, santalol, piperitol, perillyl alcohol, patchouli alcohol, hexanol, 1-hexanol, 3-cis-hexanol, cis-3-hexen-1-ol, phenylethanol, eugenol, sesamol, sotolone, maple furanone, methyl anthranilate, guaiacol, raspberry ketone, 2-methoxy-4-vinylphenol, 4-ethylguaiacol, benzylalcohol, homofuraneol, vanillin, ethylvanillin, and combinations thereof. In some embodiments, the flavouring agent is selected from the group consisting of geraniol, citronellol, nerol, maltol, ethylmaltol, fenchol, homofuraneol, furaneol, norfuraneol, 1-octen-3-ol, borneol, linalool, farnesol, hydroxycitronellol, 3,7-dimethyloctanol, myrcenol, lavandulol, nerolidol, terpineol, alpha-terpineol, menthol, thymol, carvacrol, myrtenol, carveol, santalol, piperitol, perillyl alcohol, patchouli alcohol, hexanol, 1-hexanol, 3-cis-hexanol, cis-3-hexen-1-ol, phenylethanol, eugenol, sesamol, sotolone, maple furanone, methyl anthranilate, guaiacol, raspberry ketone, 2-methoxy-4-vinylphenol, 4-ethylguaiacol, benzylalcohol, homofuraneol, and combinations thereof.

Where present, a flavouring agent may be included in the oral product in an amount up to about 10% by weight, such as up to about 5% by weight, such as up to about 1% by weight of the oral product. In some embodiments, a flavouring agent is present in an amount of from about 0.01% to about 5% by weight, such as in an amount of from about 0.1% to about 2.5% by weight of the oral product, preferably in an amount of from about 0.25% to about 1% by weight of the oral product.

*Humectants*

In some embodiments, the oral product comprises at least one humectant. Examples of suitable humectants that may be included in the product include, but are not limited to, glycerin, 1,2-propanediol (propylene glycol), 1,3-propanediol, dipropylene glycol, sorbitol, xylitol, mannitol, and the like. In some embodiments, the humectant is or comprises glycerin. In some embodiments, the oral product comprises glycerin. In some embodiments, the humectant is or comprises propylene glycol. In some embodiments, the oral product comprises propylene glycol. The amount of humectant utilised in the oral product can vary, but may be up to about 5% by weight, and certain embodiments can be characterised by a humectant content of at least about 1% by weight, such as about 2 to about 5% by weight of the oral product. In some embodiments, the humectant (such as glycerin and/or propylene glycol) may be present in an amount of from about 0.01% to about 25% by weight of the oral product, such as from about 0.1% to about 20% by weight of the oral product, such as from about 0.5% to about 15% by weight of the oral product, such as from about 1% to about 10% by weight of the oral product, such as from about 5% to about 10% by weight of the oral product.

*Sweeteners*

In order to improve the sensory properties of the oral product, one or more sweeteners may be added. The sweeteners can be any sweetener or combination of sweeteners, in natural or artificial form, or as a combination of natural and artificial sweeteners. Examples of natural sweeteners include fructose, sucrose, glucose, maltose, mannose, galactose, lactose, stevia, honey, and the like. Examples of artificial sweeteners include sucralose, isomaltulose, maltodextrin, saccharin, aspartame, acesulfame K, neotame and the like.

In some embodiments, the sweetener comprises one or more sugar alcohols. The sugar alcohol may include erythritol, arabitol, ribitol, isomalt, maltitol, dulcitol, iditol, mannitol, xylitol, lactitol, sorbitol, or combinations thereof. In some embodiments, the sweetener is selected from the group consisting of fructose, sucrose, glucose, maltose, mannose, galactose, lactose, stevia, honey, sucralose, isomaltulose, maltodextrin, saccharin, aspartame, acesulfame K, neotame,

erythritol, arabitol, ribitol, isomalt, maltitol, dulcitol, iditol, mannitol, xylitol, lactitol, sorbitol, and mixtures thereof.

In some embodiments, the sweetener is selected from the group consisting of sucralose, acesulfame K, aspartame, maltodextrin, mannitol, sucrose, and mixtures thereof. Preferably, the sweetener may be sucralose and/or acesulfame K. When present in the oral product, the sweetener (such as sucralose and/or acesulfame K) may be present in an amount of from about 0.001% to about 5% by weight, such as from about 0.01% to about 3% by weight, preferably from about 0.01% to about 1% by weight of the oral product.

*Buffering agents*

Non-limiting examples of suitable buffering agents that may be included in the oral product include alkali metals acetates, glycinates, phosphates, glycerophosphates, citrates, carbonates, hydrogen carbonates, borates, or mixtures thereof. In some embodiments, in which a buffering agent is present, the buffering agent is selected from the group consisting of sodium carbonate, sodium bicarbonate, sodium phosphate, ammonium phosphate, dicalcium phosphate, tricalcium phosphate, and mixtures thereof. In some embodiments, the buffering agent is sodium bicarbonate and/or sodium carbonate. Where present, the buffering agent (e.g. sodium bicarbonate and/or sodium carbonate) may be included in an amount less than about 5% based on the weight of the oral product; for example, from about 0.5% to about 5%, such as, e.g., from about 0.75% to about 4%, from about 0.75% to about 3%, or from about 1% to about 2% by weight, based on the total weight of the oral product.

*Organic acid*

In some embodiments, the product comprises an organic acid. As used herein, the term "organic acid" refers to an organic (i.e., carbon-based) compound that is characterised by acidic properties. Typically, organic acids are relatively weak acids (i.e., they do not dissociate completely in the presence of water), such as carboxylic acids ($-CO_2H$) or sulfonic acids ($-SO_2OH$). As used herein, reference to organic acid means an organic acid that is intentionally added. In this regard, an organic acid may be intentionally added as a specific mixture ingredient as opposed to merely being inherently present as a component of another mixture ingredient (e.g., the small amount of organic acid which may inherently be present in a mixture ingredient such as a tobacco material). In some embodiments, the one or more organic acids are added neat (i.e., in their free acid, native solid or liquid form) or as a solution in, e.g. water. In some embodiments, the one or more organic acids are added in the form of a salt.

In some embodiments, the organic acid is a carboxylic acid or a sulfonic acid. The carboxylic acid or sulfonic acid functional group may be attached to any alkyl, cycloalkyl, heterocycloalkyl, aryl, or heteroaryl group having, for example, from one to twenty carbon atoms ($C_1$-$C_{20}$). In some embodiments, the organic acid is an alkyl, cycloalkyl, heterocycloalkyl, aryl, or heteroaryl carboxylic or sulfonic acid.

In some embodiments, the organic acid is an alkyl carboxylic acid. Non-limiting examples of alkyl carboxylic acids include formic acid, acetic acid, propionic acid, octanoic acid, nonanoic acid, decanoic acid, undecanoic acid, dodecanoic acid, stearic acid, oleic acid, linoleic acid, linolenic acid, and the like. In some embodiments, the organic acid is an alkyl sulfonic acid. Non-limiting examples of alkyl sulfonic acids include propanesulfonic acid and octanesulfonic acid.

In some embodiments, the alkyl carboxylic or sulfonic acid is substituted with one or more hydroxyl groups. Non-limiting examples include glycolic acid, 4-hydroxybutyric acid, and lactic acid.

In some embodiments, an organic acid may include more than one carboxylic acid group or more than one sulfonic acid group (e.g., two, three, or more carboxylic acid groups). Non-limiting examples include oxalic acid, fumaric acid, maleic acid, and glutaric acid. In organic acids containing multiple carboxylic acids (e.g., from two to four carboxylic acid groups), one or more of the carboxylic acid groups may be esterified. Non-limiting examples include succinic acid monoethyl ester, monomethyl fumarate, monomethyl or dimethyl citrate, and the like.

In some embodiments, the organic acid may include more than one carboxylic acid group and one or more hydroxyl groups. Non-limiting examples of such acids include tartaric acid, citric acid, and the like. In some preferred embodiments, the organic acid is citric acid, sodium citrate, calcium citrate, or a combination thereof.

In some embodiments, the organic acid is an aryl carboxylic acid or an aryl sulfonic acid. Non-limiting examples of aryl carboxylic and sulfonic acids include benzoic acid, toluic acids, salicylic acid, benzenesulfonic acid, and p-toluenesulfonic acid.

Additional non-limiting examples of suitable organic acids include 2,2-dichloroacetic acid, 2-hydroxyethanesulfonic acid, 2-oxoglutaric acid, 4-acetamidobenzoic acid, 4-aminosalicylic acid, acetic acid, adipic acid, ascorbic acid (L), aspartic acid (L), camphoric acid (+), camphor-10-sulfonic acid (+), capric acid, caproic acid, caprylic acid, cinnamic acid, cyclamic acid, decanoic acid, dodecylsulfuric acid, ethane-1,2-disulfonic acid, ethanesulfonic acid, formic acid, fumaric acid, galactaric acid, gentisic acid, glucoheptonic acid, gluconic acid, glucuronic acid, glutamic acid, glycerophosphoric acid, glycolic acid, hippuric acid, isobutyric acid, lactobionic acid, lauric acid, malonic acid, mandelic acid, methanesulfonic acid, naphthalene-1,5-disulfonic acid, naphthalene-2-sulfonic acid, oleic acid, palmitic acid, pamoic

acid, pyroglutamic acid, sebacic acid, stearic acid, and undecylenic acid.

Preferably, the organic acid is selected from the group consisting of citric acid, malic acid, lactic acid, benzoic acid, tartaric acid, and mixtures thereof. In some preferred embodiments, the organic acid is or comprises citric acid or a salt thereof.

In some embodiments, the product comprises an alkali metal salt of an organic acid. For example, at least a portion of the organic acid may be present in the product in the form of an alkali metal salt. Suitable alkali metal salts include lithium, sodium, and potassium. In some embodiments, the alkali metal is sodium or potassium. In some embodiments, the alkali metal is sodium. In some embodiments, the product comprises an organic acid and a sodium salt of the organic acid. In some embodiments, the organic acid is or comprises sodium citrate, such as trisodium citrate.

The amount of organic acid present in the product may vary. The oral product may comprise from about 0.01% to about 10% by weight of organic acid, present as one or more organic acids, based on the total weight of the oral product. In some embodiments, the oral product comprises at least about 0.01%, at least about 0.1 %, about 0.2%, about 0.3%, about 0.4%, about 0.5%, about 0.6%, about 0.7%, about 0.8%, about 0.9%, about 1%, about 2%, about 3%, about 4%, about 5%, about 6%, about 7%, about 8%, about 9%, or at least about 10% organic acid by weight, based on the total weight of the oral product. In some preferred embodiments, the oral product comprises from about 0.01% to about 5% by weight of organic acid based on the weight of the oral product. For example, the oral product comprises an organic acid in an amount of from about 0.1% to about 2.5% by weight of the oral product. In the case where a salt of an organic acid is added (e.g., citric acid anhydrate), the percent by weight is calculated based on the weight of the free acid, not including any counter-ion which may be present.

In certain embodiments the organic acid inclusion is sufficient to provide a product pH of from about 4.0 to about 9.0, such as from about 4.5 to about 7.0, or from about 5.5 to about 7.0, from about 4.0 to about 5.5, or from about 7.0 to about 9.0. In some embodiments, the organic acid inclusion is sufficient to provide a product pH of from about 4.5 to about 6.5, for example, from about 4.5, about 5.0, or about 5.5, to about 6.0, or about 6.5. In some embodiments,, the organic acid is provided in a quantity sufficient to provide a pH of the product of from about 5.5 to about 6.5, for example, from about 5.5, about 5.6, about 5.7, about 5.8, about 5.9, or about 6.0, to about 6.1, about 6.2, about 6.3, about 6.4, or about 6.5.

In other embodiments, a mineral acid (e.g., hydrochloric acid, sulfuric acid, phosphoric acid, or the like) is added to adjust the pH of the product to the desired value.

Organic acids (e.g., citric acid) may be added neat (i.e., as a solid) or in solution, for example, in water. In some embodiments, the organic acid is added as a 50% aqueous solution.

*Salts*

The oral product may further comprise a salt. This may be included in an amount sufficient to provide desired sensory attributes to the product. Non-limiting examples of suitable salts include sodium chloride, potassium chloride, ammonium chloride, flour salt, sodium acetate, sodium citrate, and the like. The salt may be included in any suitable amount, such as at least about 0.5% by weight, such as at least about 1% by weight, such as at least about 1.5% by weight of the oral product. In some embodiments, the oral product may comprise salt in an amount of from about 0.5% to about 10% by weight, such as from about 1% to about 7.5% by weight, such as from about 1.5% to about 5% by weight, based on the total weight of the oral product.

*Colouring agents*

A colouring agent may be employed in amounts sufficient to provide the desired physical attributes to the product. Examples of colouring agents include various dyes and pigments, such as caramel colouring and titanium dioxide. Natural colouring agents such as curcumin, beet juice extract, spirulina; also a variety of synthetic pigments may also be used. The amount of colorant utilised in the oral product can vary, but when present is typically up to about 3% by weight, such as from about 0.1 %, about 0.5%, or about 1%, to about 3% by weight, based on the total weight of the oral product.

*Filler or Bulking Agents*

Depending on the form of the product, the oral product may comprise a filler or bulking agent. Fillers or bulking agents, for example, may fulfil multiple functions, such as enhancing certain organoleptic properties such as texture and mouthfeel, enhancing cohesiveness or compressibility of the product, and the like.

In some embodiments, the filler is a porous particulate material and is cellulose-based. For example, the filler or bulking agent may be a non-tobacco plant material or derivative thereof, including cellulose materials derived from such sources. Examples of cellulosic non-tobacco plant material include cereal grains (e.g., maize, oat, barley, rye, buckwheat, and the like), sugar beet (e.g., FIBREX® brand filler available from International Fiber Corporation), bran fiber, and mixtures thereof. In some embodiments, the filler is a cellulose material selected from the group consisting of maize fiber,

oat fiber, barley fiber, rye fiber, buckwheat fiber, sugar beet fiber, bran fiber, bamboo fiber, wood pulp fiber, cotton fiber, citrus pulp fiber, grass fiber, willow fiber, poplar fiber, cocoa fiber, derivatives thereof, and combinations thereof. In some embodiments, the filler is a cellulose material selected from the group consisting of sugar beet fiber, wood pulp fiber, bamboo fiber, derivatives thereof, and combinations thereof.

In some embodiments, the filler is derived from wood pulp fiber. One particularly suitable filler for use in the products described herein is microcrystalline cellulose ("MCC"). The MCC may be synthetic or semi-synthetic, or it may be obtained entirely from natural celluloses. The MCC may be selected from the group consisting of AVICEL® grades PH-100, PH-101, PH-102, PH-103, PH-105, PH-112, PH-113, PH-200, PH-300, PH-301, PH-302, VIVACEL® grades 101, 102, 12, 20 and EMOCEL® grades 50M and 90M, and the like, and mixtures thereof.

In some embodiments, the filler is a non-tobacco plant material or a derivative thereof. Non-limiting examples of derivatives of non-tobacco plant material include starches (e.g., from potato, wheat, rice, corn), natural cellulose, and modified cellulosic materials. Additional examples of potential fillers include maltodextrin, dextrose, calcium carbonate, calcium phosphate, lactose, mannitol, xylitol, and sorbitol. Combinations of these fillers can also be used.

"Starch" as used herein may refer to pure starch from any source, modified starch, or starch derivatives. Starch is present, typically in granular form, in almost all green plants and in various types of plant tissues and organs (e.g., seeds, leaves, rhizomes, roots, tubers, shoots, fruits, grains, and stems). Starch can vary in composition, as well as in granular shape and size. Often, starch from different sources has different chemical and physical characteristics. A specific starch can be selected for inclusion in the product based on the ability of the starch material to impart a specific organoleptic property to product. Starches derived from various sources can be used. For example, major sources of starch include cereal grains (e.g., rice, wheat, and maize) and root vegetables (e.g., potatoes and cassava). Other examples of sources of starch include acorns, arrowroot, arracacha, bananas, barley, beans (e.g., favas, lentils, mung beans, peas, chickpeas), breadfruit, buckwheat, canna, chestnuts, colacasia, katakuri, kudzu, malanga, millet, oats, oca, Polynesian arrowroot, sago, sorghum, sweet potato, quinoa, rye, tapioca, taro, tobacco, water chestnuts, and yams. Certain starches are modified starches. A modified starch has undergone one or more structural modifications, often designed to alter its high heat properties. Some starches have been developed by genetic modifications, and are considered to be "modified" starches. Other starches are obtained and subsequently modified. For example, modified starches can be starches that have been subjected to chemical reactions, such as esterification, etherification, oxidation, depolymerization (thinning) by acid catalysis or oxidation in the presence of base, bleaching, transglycosylation and depolymerization (e.g., dextrinization in the presence of a catalyst), cross-linking, enzyme treatment, acetylation, hydroxypropylation, and/or partial hydrolysis. Other starches are modified by heat treatments, such as pregelatinization, dextrinization, and/or cold water swelling processes. Certain modified starches include monostarch phosphate, distarch glycerol, distarch phosphate esterified with sodium trimetaphosphate, phosphate distarch phosphate, acetylated distarch phosphate, starch acetate esterified with acetic anhydride, starch acetate esterified with vinyl acetate, acetylated distarch adipate, acetylated distarch glycerol, hydroxypropyl starch, hydroxypropyl distarch glycerol, and starch sodium octenyl succinate.

Other suitable fillers or bulking agents include sugar alcohols. Sugar alcohols are polyols derived from monosaccharides or disaccharides that have a partially or fully hydrogenated form. Sugar alcohols have, for example, about 4 to about 20 carbon atoms and include erythritol, arabitol, ribitol, isomalt, maltitol, dulcitol, iditol, mannitol, xylitol, lactitol, sorbitol, and combinations thereof. In some embodiments, where present, the filler may be selected from the group consisting of isomalt, maltitol and mixtures thereof.

*Binding agent*

In some embodiments, the oral product may further comprise at least one binder. A binder (or combination of binders) may be employed in the product in certain embodiments, in amounts sufficient to provide the desired physical attributes and physical integrity to the product. Binders can be organic or inorganic, or a combination thereof. Representative binders include cellulose derivatives, povidone, sodium alginate, starch-based binders, pectin, carrageenan, pullulan, zein, and the like, and combinations thereof. The amount of binder utilised in the product can vary, but may be up to about 30% by weight, and certain embodiments are characterised by a binder content of at least about 0.1% by weight, such as from about 1% to about 30% by weight, or about 1% to about 10% by weight, based on the total weight of the oral product.

In some embodiments, the binder comprises pectin. Pectins are natural polymers related to carbohydrates and which are acidic heteropolysaccharides (polysaccharides comprising multiple monosaccharide units). As opposed to carbohydrates, the pectin C-6 position contains a carboxylic acid (or corresponding methyl ester or carboxamide) group instead of a hydroxymethyl group. The principal subunit is known as galacturonic acid, which can be copolymerised with L-rhamnose. Other sugars are featured as side-chain substituents. Pectin acts as a thickening and gelling agent. Pectin isolated from sources such as apple pomace, citrus peels, sugarbeet waste from sugar manufacturing, sunflower heads discarded from seed harvesting, mango waste, and other commercially available pectins may be used. In combination with certain sugars, under acidic conditions (e.g., a pH of from about 2.5 to about 5), or in the presence of a gelation agent

(calcium or other divalent alkaline earth elements), pectins may provide a gel or gum consistency to products as disclosed herein. In some embodiments, the binder comprises low methoxy pectin. Suitable low methoxy pectins include, for example, "GENU® pectin type LM-104 AS", available from CP Kelco, Atlanta, GA, USA In some embodiments, the binder comprises low methoxy pectin in combination with a gelation agent. In some embodiments, the gelation agent comprises calcium ions, such as, but not limited to, calcium diphosphate. In some embodiments, the binder comprises a high methoxy pectin in combination with an organic acid, described herein below. In some embodiments, the binder comprises a high methoxy pectin in combination with citric acid.

When present, a pectin binder is typically present in an amount of up to about 3% by weight, for example, from about 0.1, about 0.2, about 0.3, about 0.4, about 0.5, about 0.6, about 0.7, about 0.8, about 0.9, or about 1, to about 1.1, about 1.2, about 1.3, about 1.4, about 1.5, about 1.6, about 1.7, about 1.8. about 1.9, about 2, about 2.1, about 2.2, about 2.3. about 2.4, about 2.5, about 2.6, about 2.7, about 2.8, about 2.9, or about 3% by weight, based on the total weight of the oral product.

In some embodiments, the binder comprises a cellulose derivative. In certain embodiments, the cellulose derivative is a cellulose ether (including carboxyalkyl ethers), meaning a cellulose polymer with the hydrogen of one or more hydroxyl groups in the cellulose structure replaced with an alkyl, hydroxyalkyl, or aryl group. Non-limiting examples of such cellulose derivatives include methylcellulose, hydroxypropylcellulose ("HPC"), hydroxypropylmethylcellulose ("HPMC"), hydroxyethyl cellulose, and carboxymethylcellulose ("CMC"). In some embodiments, the cellulose derivative is or comprises HPC. In some embodiments, the cellulose derivative is a combination of HPC and HPMC. In some embodiments, the oral product comprises from about 1% to about 10% of the cellulose derivative (such as HPC) by weight of the oral product, with certain embodiments comprising from about 1% to about 5% by weight of cellulose derivative (such as HPC), based on the weight of the product.

In certain embodiments, the binder includes a gum, for example, a natural gum. As used herein, a natural gum refers to polysaccharide materials of natural origin that have binding properties, and which are also useful as a thickening or gelling agents. Representative natural gums derived from plants, which are typically water soluble to some degree, include xanthan gum, guar gum, gum arabic, ghatti gum, gum tragacanth, karaya gum, locust bean gum, gellan gum, and combinations thereof. When present, natural gum binder materials may be present in an amount of up to about 5% by weight, for example, from about 0.1, about 0.2, about 0.3, about 0.4, about 0.5, about 0.6, about 0.7, about 0.8, about 0.9, or about 1%, to about 2, about 3, about 4, or about 5% by weight, based on the total weight of the product.

*Thickeners*

The oral product may comprise a thickening agent in some embodiments. Suitable thickening agents may include a hydrocolloid, such as xanthan gum, guar gum, konjac gum, gum tragacanth and gum Arabic. For example, xanthan gum is understood to be a thickening agent that thickens compositions when added during cold processing (i.e. when heat is not applied). Where present, a thickening agent (e.g. xanthan gum) may be included in an amount of from about 0.001% to about 5% by weight, and preferably from about 0.01% to about 1% by weight of the oral product.

*Other additives*

Other ingredients such as preservatives (e.g., potassium sorbate), disintegration aids (e.g., croscarmellose sodium, crospovidone, sodium starch glycolate, pregelatinized corn starch, and the like), and/or antioxidants can also be used. Typically, such ingredients, where used, are used in amounts of up to about 10% by weight, for example at least about 0.1% by weight, such as about 0.5 to about 10% by weight of the oral product. A disintegration aid may be employed in an amount sufficient to provide control of desired physical attributes of the oral product such as, for example, by providing loss of physical integrity and dispersion of the various component materials upon contact of the formulation with water (e.g., by undergoing swelling upon contact with water).

Examples of further types of additives include zinc or magnesium salts selected to be relatively water soluble for compositions with greater water solubility (e.g., magnesium or zinc gluconate) or selected to be relatively water insoluble for compositions with reduced water solubility (e.g., magnesium or zinc oxide), or combinations thereof. See, for example, those representative components, combination of components, relative amounts of those components, and manners and methods for employing those components, set forth in US Pat. No. 9,237,769 to Mua et al., US Pat. No. 7,861,728 to Holton, Jr. et al., US Pat. App. Pub. No. 2010/0291245 to Gao et al., and US Pat. App. Pub. No 2007/0062549 to Holton, Jr. et al., each of which is incorporated herein by reference. Typical inclusion ranges for such additional additives can vary depending on the nature and function of the additive and the intended effect on the final product, with an example range of up to about 10% by weight, based on total weight of the oral product (e.g., about 0.1 to about 5% by weight).

In some embodiments, the oral product comprises a magnesium salt. A non-limiting example of a suitable magnesium salt is magnesium gluconate. In some embodiments, the oral product comprises magnesium in an amount by weight from about 0.1% to about 2%, or from about 0.2 to about 1%, based on elemental magnesium.

The aforementioned additives can be employed together (e.g., as additive formulations) or separately (e.g., individual additive components can be added at different stages involved in the preparation of the final product).

Oral Product

The products or compositions as described herein are configured for oral use. The term "configured for oral use" as used herein means that the product is provided in a form such that during use, saliva in the mouth of the user causes one or more of the components of the product (e.g., active ingredients) to pass into the mouth of the user. In certain embodiments, the product is adapted to deliver active ingredients and optionally flavouring agent to a user through mucous membranes in the user's mouth, the user's digestive system, or both.

The combination of active ingredients may be present in an amount of from about 0.01% to about 20% by weight. For example, the combination of active ingredients may be present in an amount of from about 0.05% to about 15% by weight, such as from about 0.1% to about 10% by weight. Preferably, the combination of active ingredients is present in an amount of from about 0.1% to about 15% by weight of the oral product, more preferably from about 1% to about 10% by weight of the oral product.

The liquid oral product may be in the form of a shot; i.e. a drink that may be consumed quickly, e.g. in a single gulp or a couple of gulps. Formulating the oral product in the form of a shot may be advantageous as it provides a convenient and easy-to-use mode of administration that does not take up much room during storage (e.g. in a fridge or cupboard) and is easily portable by the user so can be taken on the go. Shots may also provide an efficient delivery vehicle for actives, whereby a level of active sufficient to provide good efficacy can be delivered to the user without the need to consume large volumes of liquid. Additionally, the shots may be formulated to have a pleasant taste. Drinking a shot may be considered to be more palatable and easier to a user than swallowing a tablet, for example.

The liquid oral product may also be in the form of a larger beverage that is consumed more slowly over several gulps. The liquid oral product may have a volume of from about 1 mL to about 250 mL, such as from about 1 mL to about 200 mL. The liquid oral product may have a volume of from about 100 mL to about 250 mL and be provided in a package such as a carton, cup, can or bottle of liquid.

The liquid oral product may have a volume of from about 10 mL to about 100 mL, such as from about 25 mL to about 75 mL. In such embodiments, the liquid oral product may be considered to be a shot. For example, the volume of liquid may be about 60 mL or about 30 mL.

As noted above, the oral product may further comprise water in an amount of from about 50% to about 99.9% by weight of the oral product. In some embodiments, the oral product comprises water in an amount of from about 75% to about 99.5% by weight, such as from about 80% to about 99% by weight, such as from about 90% to about 97.5% by weight of the oral product. In some embodiments, the oral product comprises water in an amount of from about 90% to about 99.5% by weight of the oral product, and may be from about 95% to about 99% by weight of the oral product. Where present, the water may include tap water, rain water, mineralised water, or distilled water.

The liquid oral product may comprise the combination of active ingredients in an amount of from about 0.1% to about 15% by weight of the oral product, and water in an amount of from about 85% to about 99.9% by weight of the oral product. The liquid oral product may comprise the combination of active ingredients in an amount of from about 0.5% to about 12% by weight of the oral product, and water in an amount of from about 88% to about 99.5% by weight of the oral product.

In any of the above embodiments, the liquid oral product may further comprise zinc or zinc gluconate, optionally in an amount of from about 0.001% to about 2% by weight of the oral product. In preferred embodiments, zinc or zinc gluconate may be present in an amount of about 0.005% to about 1% by weight of the oral product.

In some embodiments, there is provided a package in the form of a bottle or can that contains the liquid oral product. The package may be a bottle containing the desired volume of liquid oral product.

*Solid oral dosage forms*

In some embodiments, the oral product is a solid oral dosage form. A solid oral product as described herein may take various forms, including pastilles and chews. As used herein, the term "solid" means that the products can substantially sustain their physical shape when unsupported by external means, e.g. packaging etc. Thus, they are considered to be solid, solid-like, in solid form or in solid-like form at room temperature. For the avoidance of doubt the solid product remains substantially solid at up to 30°C. In some embodiments, the oral product is in solid form, such as in the form of chews or pastilles. In some embodiments, the oral product is a chew or pastille.

The oral products as disclosed herein can be formed into a variety of shapes, including pills, tablets, spheres, strips, films, sheets, coins, cubes, beads, ovoids, obloids, cylinders, bean-shaped, sticks, or rods. Cross-sectional shapes of the product can vary, and example cross-sectional shapes include circles, squares, ovals, rectangles, and the like. Such shapes can be formed in a variety of manners using equipment such as moving belts, nips, extruders, granulation devices,

compaction devices, and the like.

In some embodiments, the solid oral product is in a form selected from the group consisting of a chew, or a pastille. In other embodiments, the oral product is in solid form, such as in the form of loose moist snuff, loose dry snuff, chewing tobacco-type form, pelletized pieces, extruded or formed strips, pieces, rods, or sticks, finely divided ground powders, finely divided or milled agglomerates of powdered pieces and components, flake-like pieces, molded processed pieces, gums, films, readily water-dissolvable or water-dispersible films or strips, capsule-like materials, tablets, lozenges. In some embodiments, the oral product is a tablet or lozenge. In some embodiments, the oral product may be a chewing gum product. In some embodiments, the oral product is in the form of moist snuff or snus, which may or may not contain tobacco.

*Chews*

In some embodiments, the product can be chewable, meaning the product has a mild resilience or "bounce" upon chewing, and possesses a desirable degree of malleability. A product in chewable form may be entirely dissolving, or may be in the form of a non-dissolving gum in which only certain components (e.g., active ingredients, flavour, sweetener) dissolve, leaving behind a non-dissolving matrix.

As described herein a "chew" is a confectionary-type product that may be chewed by the user before dissolving in the mouth (i.e. a dissolvable chew) or may be a chewing gum. Preferably, the "chew" dissolves fully in the mouth of the user. The product may be dissolvable. As used herein, the terms "dissolve," "dissolving," and "dissolvable" refer to products having aqueous-soluble components that interact with moisture in the oral cavity and enter into solution, thereby causing gradual consumption of the product. According to one aspect, the dissolvable product is capable of lasting in the user's mouth for a given period of time until it completely dissolves. Dissolution rates can vary over a wide range, from about 1 minute or less to about 60 minutes. For example, fast release products typically dissolve and/or release the desired component(s) (e.g., active ingredient, flavour, and the like) in about 2 minutes or less, often about 1 minute or less (e.g., about 50 seconds or less, about 40 seconds or less, about 30 seconds or less, or about 20 seconds or less). Dissolution can occur by any means, such as melting, mechanical disruption (e.g., chewing), enzymatic or other chemical degradation, or by disruption of the interaction between the components of the product.

In other embodiments, the products do not dissolve during the product's residence in the user's mouth.

Chewable embodiments generally include a binding agent, such as a natural gum, pectin, agar, carrageenan, starch, or a combination thereof. In some embodiments, the binding agent comprises or is pectin.

In some embodiments, the chewable product (i.e. "chew") comprises the combination of active ingredients, at least one bulking agent selected from the group consisting of a sugar alcohol, or at least one a sugar, or a combination of at least one sugar alcohol and at least one sugar, and at least one binding agent. In some embodiments, the chewable product (i.e. "chew") comprises the combination of active ingredients in an amount of from about 0.1% to about 10% by weight of the oral product, at least one bulking agent selected from the group consisting of a sugar alcohol, or at least one a sugar, or a combination of at least one sugar alcohol and at least one sugar, and at least one binding agent. The chewable product or chew may also optionally include a sweetening agent and/or a flavouring agent.

Representative chew compositions and products may incorporate from about 0.1% to about 20% by weight of the combination of active ingredients, from about 0.1% to about 10% of a binding agent (e.g. pectin, agar, carrageenan, starch, or a combination thereof), and at least about 30% by weight of at least one bulking agent selected from the group consisting of a sugar alcohol, or at least one a sugar, or a combination of at least one sugar alcohol and at least one sugar, based on the total weight of the oral product. Optionally, the oral product may further comprise about 0.01 to about 2% by weight of a sweetening agent, and/or from about 0.1% to about 5% by weight of at least one flavoring agent, based on the total weight of the oral product. The particular percentages and choice of ingredients will vary depending upon the desired flavor, texture, and other characteristics. For example, the combination of active ingredients may be present in a chew in an amount of from about 0.1% to about 10% by weight of the oral product.

In some embodiments, the oral chew product comprises pectin and an organic acid, along with one or more sugar alcohols in an amount of at least 30% by weight of the oral product.

In some embodiments, the oral chew product comprises at least one bulking agent selected from the group consisting of a sugar alcohol, or a sugar, or a combination of at least one sugar alcohol and at least one sugar. In some embodiments, the at least one bulking agent is selected from a sugar alcohol, or a sugar. In some embodiments, the at least one bulking agent is selected from sugar alcohol(s). In some embodiments, the at least one bulking agent is selected from sugar(s). In some embodiments, the at least one bulking agent is selected from a combination of at least one sugar alcohol and at least one sugar.

The total amount of bulking agent(s) (preferably sugar alcohol) may be at least about 30% by weight, such as at least about 40% by weight, such as at least about 45% by weight of the oral product. In some preferred embodiments, the total amount of bulking agent(s) (preferably sugar alcohol) may be at least about 50% by weight of the oral product.

In some embodiments, the total amount of sugar alcohol is present in an amount of from about 30% to about 99% by weight, such as from about 40% to about 99% by weight, such as from about 45% to about 99% by weight, such as from

about 50% to about 99% by weight, such as from about 60% to about 95% by weight, and preferably from about 70% to about 90% by weight of the oral product. The sugar alcohol may preferably be present in an amount of from about 80% to about 95% by weight of the oral product. In some preferred embodiments, the total amount of bulking agent(s) (preferably sugar alcohol) is present in an amount of from about 40% to about 60% by weight of the oral product. Alternatively, in some preferred embodiments, the total amount of sugar alcohol is from about 50% to about 90% by weight of the oral product. In such embodiments, the bulking agent may consist of sugar alcohol(s).

The sugar alcohol may be selected from the group consisting of isomalt, erythritol, sorbitol, arabitol, ribitol, maltitol, dulcitol, iditol, mannitol, xylitol, lactitol, or mixtures thereof. In some embodiments, the sugar alcohol is selected from the group consisting of maltitol, sorbitol, erythritol, mannitol, lactitol, xylitol, isomalt, and mixtures thereof. In some embodiments, the sugar alcohol is selected from the group consisting of maltitol, isomalt, and mixtures thereof. In some embodiments, the sugar alcohol is or comprises isomalt. In some embodiments, the sugar alcohol is or comprises maltitol. In some embodiments, the sugar alcohol comprises a combination of isomalt and maltitol.

In some embodiments, a sugar substitute may be an alternative to sugar alcohols, or used in combination with one or more sugar alcohols. Suitable sugar substitutes include allulose, soluble tapioca fiber, inulin, and combinations thereof. In some embodiments, sugar is included as an alternative to sugar alcohols, or is used in combination with one or more sugar alcohols. Where present, sugar may be included in the form of glucose, fructose, galactose, sucrose, or mixtures thereof. In some embodiments, the oral chew product comprises sugar (e.g. sucrose) in combination with at least one sugar alcohol (e.g. maltitol and/or isomalt). The sugar may be present in any suitable amount, such as from 10% to about 50%, such as from about 20% to about 40% by weight of the oral product.

In some embodiments the bulking agents consists of at least one sugar alcohol selected from maltitol and/or isomalt and sucrose. In some embodiments the at least one bulking agent consists of maltitol and sucrose. In some embodiments the at least one bulking agent consists of isomalt and sucrose. In some embodiments, the bulking agent comprises a combination of maltitol and sugar. The maltitol may be present in an amount of from about 20% to about 50%, and the sugar may be present in an amount of from about 10% to about 50% by weight of the oral product. In some embodiments, the bulking agent comprises a combination of isomalt and sugar. The isomalt may be present in an amount of from about 20% to about 50%, and the sugar may be present in an amount of from about 10% to about 50% by weight of the oral product.

In some embodiments, the binding agent is selected from the group consisting of pectin, agar, carrageenan, starch, and mixtures thereof. In some preferred embodiments, the binding agent is or comprises pectin. In some embodiments, the binding agent (e.g. pectin) is present in an amount of from about 0.1% to about 10% by weight of the oral product. Preferably, the binding agent (e.g. pectin) is present in an amount of from about 1 % to about 5% by weight of the oral product.

The oral product may also comprise an organic acid, a gelation agent or both to crosslink the pectin.

In some embodiments, the oral chew product comprises an acidifying agent. The acidifying agent may be an organic or inorganic acid. The organic acid may be any suitable organic acid. Suitable organic acids are as described hereinabove in the section titled "Additives". For example, the organic acid may be selected from the group consisting of citric acid, malic acid, lactic acid, benzoic acid, tartaric acid, and mixtures or salts thereof. The organic acid may, for example, include a combination of citric acid and a salt of citric acid (e.g. a sodium salt).

Oral products of the present disclosure in the form of a chew may contain various amounts of water. For example, the water content of the chew product may be provided within a specified range so as to dictate the final form of the product. The water content of the chew products described herein, prior to use by a consumer of the product, may vary within such ranges according to the desired properties and characteristics, in addition to dictating the final form of the product. For example, chew-type products may possess a water content in the range of from about 1% to about 20%, such as from about 1% to about 10% by weight of the oral product.

The oral product in the form of a chew may comprise:

(a) the combination of active ingredients as described herein in an amount of from about 0.1% to about 10% by weight of the oral product;

(b) at least one bulking agent selected from the group consisting of a sugar alcohol, or a sugar, or a combination of at least one sugar alcohol and at least one sugar, wherein the total amount of sugar alcohol and/or sugar is from about 40% to about 99% by weight of the oral product;

(c) at least one binding agent in an amount of from about 0.1% to about 10% by weight of the oral product;

optionally (d) a flavouring agent, an organic acid and/or a sweetening agent.

The oral chew product may have a weight of from about 0.1 g to about 10 g, such as from about 0.5 g to about 5 g. Preferably, the oral chew product has a weight of from about 1 g to about 5 g. An exemplary chew product may have a

weight of about 4 g.

In some embodiments, the chew may further comprise a coating, such as a coating oil. The coating may comprise an oil or wax; for example, sunflower oil and/or carnuba wax. Suitable coatings are described in further detail in respect of the pastille products, which discussion equally applies to the chews in full. For example, the chew may be coated with an overcoat material. Devices for providing outer coating layers to compacted pelletized compositions are available as CompuLab 24, CompuLab 36, Accela-Cota 48 and Accela-Cota 60 from Thomas Engineering.

When present, a coating may comprise a film-forming polymer, such as a cellulosic polymer, an optional plasticizer, and optional flavorants, colorants, salts, sweeteners or other additives of the types set forth herein. The coating compositions may be aqueous in nature and can be applied using any pellet or tablet coating technique known in the art, such as pan coating. Example film-forming polymers include cellulosic polymers such as methylcellulose, hydroxypropyl cellulose (HPC), hydroxypropyl methylcellulose (HPMC), hydroxyethyl cellulose, and carboxy methylcellulose. Example plasticizers include aqueous solutions or emulsions of glyceryl monostearate and triethyl citrate. Additional potential coatings include food grade shellac, oils, such as sunflower oils, waxes such as carnuaba wax, and combinations thereof.

*Pastilles*

In some embodiments, the products disclosed herein may be in the form of a dissolvable and lightly chewable pastille product for oral use. As used herein, the term "pastille" refers to a dissolvable oral product made by solidifying a liquid or gel composition, such as a composition that includes a gelling or binding agent, so that the final product is a hardened solid gel. A pastille product may alternatively be referred to as a soft lozenge. In certain embodiments, the pastille products of the disclosure are characterised by sufficient cohesiveness to withstand light chewing action in the oral cavity without rapidly disintegrating. The pastille products of the disclosure typically do not exhibit a highly deformable chewing quality as found in conventional chewing gum. See, for example, the smokeless tobacco pastilles, pastille formulations, pastille configurations, pastille characteristics and techniques for formulating or manufacturing pastilles set forth in US Pat. Nos. 9,204,667 to Cantrell et al.; 9,775,376 to Cantrell et al.; 10,357,054 to Marshall et al.; which are incorporated herein by reference. Certain products can exhibit, for example, one or more of the following characteristics: crispy, granular, chewy, syrupy, pasty, fluffy, smooth, and/or creamy. In certain embodiments, the desired textural property can be selected from the group consisting of adhesiveness, cohesiveness, density, dryness, fracturability, graininess, gumminess, hardness, heaviness, moisture absorption, moisture release, mouthcoating, roughness, slipperiness, smoothness, viscosity, wetness, and combinations thereof.

Pastille products of the present disclosure typically comprise a combination of active ingredients in an amount of from about 0.1% to about 10% by weight of the oral product, a binding agent, and a sugar alcohol and/or sugar (e.g. as a filler component). Any active ingredient as discussed herein is meant to be suitable for use as an active ingredient in the pastille products according to the present disclosure.

In some embodiments, the active ingredients may be provided in the pastille in liquid form or in a dry powder or particulate form. In some embodiments, each of the active ingredients included in the combination of active ingredients is provided in the pastille in a dry powder form.

A binding agent (or combination of two or more binding agents) may be employed in amounts sufficient to provide the desired physical attributes and physical integrity to the pastille products. In some embodiments, the binding agent in a pastille may comprise pectin or a gum. A representative amount of binding agent (e.g. pectin or gum) may make up at least about 5% or at least about 10% of the total weight of the pastille product. In certain embodiments, the binding agent(s) (e.g. pectin or a gum) will be present in an amount of at least about 30% by weight, at least about 35% by weight, at least about 40% by weight, at least about 45% by weight, or at least about 50% by weight, based on the total weight of the oral product. In some embodiments, the binding agent (e.g. pectin or a gum) may be present in an amount of about 35% to about 55% by weight of the oral product. Preferably, the total amount of binding agent (e.g. pectin or a gum) within the pastille product will not exceed about 55% by weight of the oral product. Often, the amount of binding agent (e.g. pectin or a gum) within a desirable product will not exceed about 65%, and frequently will not exceed about 60% by weight of the oral product.

In some embodiments, the binding agent may comprise pectin. The pastille product may include pectin as the only binding agent, or pectin may be included in combination with a gum.

In certain embodiments, the binding agent is or comprises a gum. The gum may include a natural gum. Particularly, natural gums (e.g., such as gum arabic) may be incorporated into the pastille products as a softener. Advantageously, use of a natural gum as a softener provides the desired textural qualities necessary for forming pastille products, particularly those described herein. Particularly, it should be noted that increasing the amount of a natural gum (e.g., gum arabic) while, subsequently, decreasing the amount of sugar alcohol and/or sugar can advantageously increase softness in the resulting pastille product. As used herein, a natural gum refers to polysaccharide materials of natural origin that are useful as softening agents. Representative natural gums derived from plants, which are typically water soluble to some degree, include xanthan gum, guar gum, gum arabic, ghatti gum, gum tragacanth, karaya gum, locust bean gum, gellan gum, and combinations thereof. Preferably, gum arabic may be used as an example natural gum which provides the

above noted softening characteristics when incorporated into the pastille products of the present disclosure.

As noted above, pastille products of the present disclosure may comprise at least one sugar, or at least one sugar alcohol, or a combination of at least one sugar and at least one sugar alcohol (e.g. in the form of a filler component). Sugar alcohols are particularly advantageous as filler components in the pastilles of the disclosure because such materials contribute some sweetness and do not disrupt the desired chewable characteristics of the final product. In some embodiments, the sugar alcohol may be selected from the group consisting of maltitol, sorbitol, erythritol, mannitol, lactitol, xylitol and isomalt. In some embodiments, isomalt may be incorporated as the sole filler component. A sugar alcohol and/or sugar is typically added to products of the disclosure in the form of an aqueous solution or suspension, such as a solution or suspension with a solids content of about 50 to about 90% by weight. Combinations of a sugar alcohol and/or sugar with a further filler component can also be used. A filler component often fulfills multiple functions, such as enhancing certain organoleptic properties such as texture and mouthfeel, enhancing cohesiveness or compressibility of the product, and the like. In some embodiments, the filler comprises a sugar substitute, such as one or more of allulose, soluble tapioca fiber, and inulin. Such sugar substitutes may be an alternative to sugar alcohols, or used in combination with one or more sugar alcohols.

*Melts*

In some embodiments, the product can be meltable as discussed, for example, in US Patent App. Pub. No. 2012/0037175 to Cantrell et al., incorporated by reference herein in its entirety. As used herein, "melt," "melting," and "meltable" refer to the ability of the product to change from a solid state to a liquid state. That is, melting occurs when a substance (e.g., a product as disclosed herein) changes from solid to liquid, usually by the application of heat. The application of heat in regard to a product as disclosed herein is provided by the internal temperature of a user's mouth. Thus, the term "meltable" refers to a product that is capable of liquefying in the mouth of the user as the product changes phase from solid to liquid, and is intended to distinguish products that merely disintegrate in the oral cavity through loss of cohesiveness within the product that merely dissolve in the oral cavity as aqueous-soluble components of the product interact with moisture.

Generally, meltable products comprise a lipid. In some embodiments, the composition comprises a lipid. The lipid is typically a fat, oil, or wax substance derived from animal or plant material (e.g., plant-derived fats), and typically comprises mostly triglycerides along with lesser amounts of free fatty acids and mono- or diglycerides. In certain embodiments, the lipid is a solid or semi-solid at room temperature (i.e., 25°C) and capable of at least partially liquefying when subjected to the temperature of the oral cavity of the user (i.e., "melting"). Example plant-derived fats are comprised primarily of saturated or unsaturated fatty acid chains (most of which are bound within triglyceride structures) having a carbon length of about 10 to about 26 carbon atoms, or about 14 to about 20 carbon atoms, or about 14 to about 18 carbon atoms.

In some embodiments, the lipid comprises an oil and, in particular, a food grade oil, including fractionated oils. Such oils include, but are not limited to, vegetable oils (e.g., acai oil, almond oil, amaranth oil, apricot oil, apple seed oil, argan oil, avocado oil, babassu oil, beech nut oil, ben oil, bitter gourd oil, black seed oil, blackcurrant seed oil, borage seed oil, borneo tallow nut oil, bottle gourd oil, brazil nut oil, buffalo gourd oil, butternut squash seed oil, cape chestnut oil, canola oil, carob cashew oil, cocoa butter, cocklebur oil, coconut oil, corn oil, cothune oil, coriander seed oil, cottonseed oil, date seed oil, dika oil, egus seed oil, evening primrose oil, false flax oil, flaxseed oil, grape seed oil, grapefruit seed oil, hazelnut oil, hemp oil, kapok seed oil, kenaf seed oil, lallemantia oil, lemon oil, linseed oil, macadamia oil, mafura oil, marula oil, meadowfoam seed oil, mongongo nut oil, mustard oil, niger seed oil, nutmeg butter, okra seed oil, olive oil, orange oil, palm oil, papaya seed oil, peanut oil, pecan oil, perilla seed oil, persimmon seed oil, pequi oil, pili nut oil, pine nut oil, pistachio oil, pomegranate seed oil, poppyseed oil, pracaxi oil, prune kernel oil, pumpkin seed oil, quinoa oil, ramtil oil, rapeseed oil, rice bran oil, royle oil, sacha inchi oil, safflower oil, sapote oil, seje oil, sesame oil, shea butter, soybean oil, sunflower oil, taramira oil, tea seed oil, thistle oil, tigernut oil, tobacco seed oil, tomato seed oil, walnut oil, watermelon seed oil, wheat germ oil, and combinations thereof), animal oils (e.g., cattle fat, buffalo fat, sheep fat, goat fat, pig fat, lard, camel fat, tallow, liquid margarine, fish oil, fish liver oil, whale oil, seal oil, and combinations thereof), and mineral oils.

In certain embodiments, the plant-derived fats of the present disclosure include palm oil, (including fractionated palm oil) palm kernel oil, soybean oil, cottonseed oil, and mixtures thereof. In one embodiment, the lipid is a blend of palm oil and palm kernel oil. The lipid can be, for example, hydrogenated, partially hydrogenated, or non-hydrogenated. Example embodiments of lipids can be purchased under the brand names CEBES®, CISAO®, or CONF AO®, available from AarhusKarlshamn USA Inc.

The melting point of the lipid is typically about 29°C or above, such as about 29°C to about 49°C, or about 36°C to about 45°C, or about 38°C to about 41 °C. In some embodiments, use of lipids with a melting point of less than about 36°C is not advantageous due to possible melting during product storage or handling. One test for determining the melting point of lipids is the Mettler dropping point method (ASTM D3954-15, Standard Test Method for Dropping Point of Waxes, ASTM International, West Conshohocken, PA, 2015, www.astm.org.).

When present, the amount of lipid within the composition may vary. In certain embodiments, the amount of lipid is at least about 10%, at least about 20%, or at least about 30%, on a dry weight basis of the composition. In certain embodiments, the amount of lipid is less than about 70%, less than about 60%, or less than about 50%, on a dry weight basis. Example lipid weight ranges include about 10 to about 70% dry weight, such as about 35 to about 50% dry weight. In some embodiments, the amount of lipid is about 35, about 40, about 45, or about 50% by weight of the total oral product.

In some embodiments, the oral product comprises a lipid. In one embodiment, the lipid is an oil selected from the group consisting of palm oil, palm kernel oil, soybean oil, sunflower oil, cottonseed oil, coconut oil, and combinations thereof, wherein the oil may be hydrogenated, partially hydrogenated, or non-hydrogenated. In one embodiment, the lipid is a trans- hydrogenated filling fat of medium hardness such as Confao® 5, available from AarhusKarlshamn USA Inc., 131 Marsh Street, Port Newark, NJ 07114.

In some embodiments, the product in meltable form comprises a lipid in an amount of from about 35 to about 50% by weight of the oral product, and a sugar alcohol in an amount of from about 35 to about 55% by weight of the oral product. In some embodiments, the sugar alcohol is isomalt, erythritol, sorbitol, arabitol, ribitol, maltitol, dulcitol, iditol, mannitol, xylitol, lactitol, or a combination thereof. In some embodiments, the sugar alcohol is or comprises isomalt. In some embodiments, a sugar substitute may be an alternative to the sugar alcohol, or used in combination with one or more sugar alcohols. Suitable sugar substitutes include allulose, soluble tapioca fiber, inulin, and combinations thereof.

*Tablets*

In certain embodiments, the product is in the form of a compressed or molded tablet. An exemplary tablet dosage form weighs from about 250 mg to about 1500 mg, such as about 250 mg to about 700 mg, or from about 700 mg to about 1500 mg. The tablet can have any of a variety of shapes, including traditional pill or tablet shapes. Generally, the product in tablet form comprises a glucose-polysaccharide blend and a sugar alcohol. In some embodiments, the glucose-polysaccharide blend is present in an amount of from about 35 to about 50% by weight, based on the total weight of the product; and the sugar alcohol is present in an amount of from about 30 to about 45% by weight, based on the total weight of the product. In some embodiments, the sugar alcohol is isomalt, erythritol, sorbitol, arabitol, ribitol, maltitol, dulcitol, iditol, mannitol, xylitol, lactitol, or a combination thereof. In some embodiments, the sugar alcohol is or comprises isomalt.

When in the form of a tablet, the product may be dissolvable. As used herein, the terms "dissolve," "dissolving," and "dissolvable" refer to products having aqueous-soluble components that interact with moisture in the oral cavity and enter into solution, thereby causing gradual consumption of the product. According to one aspect, the dissolvable product is capable of lasting in the user's mouth for a given period of time until it completely dissolves. Dissolution rates can vary over a wide range, from about 1 minute or less to about 60 minutes. For example, fast release products typically dissolve and/or release the desired component(s) (e.g., active ingredient, flavour, and the like) in about 2 minutes or less, often about 1 minute or less (e.g., about 50 seconds or less, about 40 seconds or less, about 30 seconds or less, or about 20 seconds or less). Dissolution can occur by any means, such as melting, mechanical disruption (e.g., chewing), enzymatic or other chemical degradation, or by disruption of the interaction between the components of the product. In other embodiments, the products do not dissolve during the product's residence in the user's mouth.

*Lozenge*

In some embodiments, the products disclosed herein may be in the form of a dissolvable lozenge product configured for oral use. Example lozenge-type products of the invention have the form of a lozenge, tablet, microtab, or other tablet-type product. See, for example, the types of nicotine-containing lozenges, lozenge formulations, lozenge formats and configurations, lozenge characteristics and techniques for formulating or manufacturing lozenges set forth in US Pat. Nos. 4,967,773 to Shaw; 5,110,605 to Acharya; 5,733,574 to Dam; 6,280,761 to Santus; 6,676,959 to Andersson et al.; 6,248,760 to Wilhelmsen; and 7,374,779; US Pat. Pub. Nos. 2001/0016593 to Wilhelmsen; 2004/0101543 to Liu et al.; 2006/0120974 to Mcneight; 2008/0020050 to Chau et al.; 2009/0081291 to Gin et al.; and 2010/0004294 to Axelsson et al.; which are incorporated herein by reference.

Lozenge products are generally described as "hard", and are distinguished in this manner from soft lozenges (i.e., pastilles). Hard lozenges are mixtures of sugars and/or carbohydrates in an amorphous state. Although they are made from aqueous syrups, the water, which is initially present, evaporates as the syrup is boiled during processing so that the moisture content in the finished product is very low, such as 0.5% to 1.5% by weight. To obtain lozenges that are hard and not tacky, the temperature of the melt generally must reach the hard crack stage, with an example temperature range of 149° to 154°C.

Lozenge-type products, in some embodiments, may exhibit translucence or transparency. The desired transparency or translucency of the product can be quantified by any known method. For example, optical methods such as turbidimetry (or nephelometry) and colorimetry may be used to quantify the cloudiness (light scattering) and the color (light

absorption), respectively, of the products. Translucency can also be confirmed by visual inspection by simply holding the product up to a light source and determining if light travels through the material or product in a diffuse manner.

The lozenge-type products of the present disclosure may incorporate various different additives in addition to the combination of active ingredients, and may be prepared according to a variety of different methods commonly known in the art for preparing lozenge-type products. Example compositions, products, and methods of preparing such products will be detailed herein below.

Lozenge products of the present disclosure typically include a composition comprising the combination of active ingredients in an amount of less than about 2% by weight, a sugar substitute in an amount of at least about 80% by weight, and a sugar alcohol syrup. Any active ingredient as discussed herein is suitable for use as an active ingredient in the lozenge products provided herein. In some embodiments, the active ingredient may be provided in liquid form or in a dry powder or particulate form. As noted above, the active ingredient typically is present in an amount from about 0.1% to about 10% by weight, such as, e.g., from about 0.1% to about 10% by weight, such as, e.g., from about 0.1 %, about 0.5%, about 1%, about 1.5%, about 2%, about 2.5%, about 3%, about 3.5%, about 4%, or about 4.5% by weight, to about 5.5%, about 6%, about 6.5%, about 7%, about 7.5%, about 8%, about 8.5%, about 9%, about 9.5%, or about 10% by weight, based on the total weight of the product. In some embodiments, the active ingredient may be present in an amount of less than about 10%, less than about 9%, less than about 8%, less than about 7%, less than about 6%, less than about 5%, less than about 4%, less than about 3%, less than about 2%, or less than about 1% by weight, based on the total weight of the product.

In some embodiments, the lozenge product comprises a sugar substitute. The sugar substitute is typically provided in pure, solid form (e.g., granular or powdered form). In certain embodiments, the sugar substitute is dry, comprising a very low water content. For example, the sugar substitute can comprise less than about 5% water by weight, less than about 3% water by weight, less than about 2% water by weight, or less than about 1% water by weight. In certain embodiments, the sugar substitute is capable of forming a glassy matrix. The formation of a glassy matrix is commonly characterized by a translucent/transparent appearance.

Typically, the sugar substitute is substantially non-hygroscopic. Non-hygroscopic materials typically do not absorb, adsorb, and/or retain a significant quantity of moisture from the air. Non-hygroscopic materials can provide the benefit of reducing the tendency of the lozenge product to tackify upon exposure to humidity. The sugar substitute can be any sugarless material (i.e., sucrose-free material) and can be natural or synthetically produced. The sugar substitute used in the products described herein can be nutritive or non-nutritive. For example, the sugar substitute is commonly a sugar alcohol. Sugar alcohols that may be useful according to the present invention include, but are not limited to, erythritol, threitol, arabitol, xylitol, ribotol, mannitol, sorbitol, dulcitol, iditol, isomalt, maltitol, lactitol, polyglycitol, and mixtures thereof. For example, in certain embodiments, the sugar alcohol is selected from the group consisting of erythritol, sorbitol, and isomalt. The amount of sugar substitute in the lozenge products can vary, but is typically at least about 75%, at least about 80%, at least about 85%, or at least about 90%, or at least about 95% by weight of the product.

In certain embodiments, the sugar substitute comprises one or more sugar alcohols. For example, in one embodiment, the sugar substitute is isomalt. In some embodiments, the sugar substitute is one or more of allulose, soluble tapioca fiber, and inulin. Such sugar substitutes may be an alternative to sugar alcohols, or used in combination with one or more sugar alcohols.

In some embodiments, the lozenge products of the present disclosure may comprise a syrup, e.g., a sugar syrup or a sugar alcohol syrup. "Sugar alcohol syrup" as used herein is intended to refer to a thick solution of sugar alcohol in water, e.g., having greater than about 40% solids, preferably having greater than about 50% solids, greater than about 60% solids, greater than about 70% solids, or greater than about 80% solids. Typically, the solid content of the sugar alcohol syrup primarily comprises the named sugar alcohol (i.e., maltitol syrup typically comprises greater than about 80%, greater than about 85%, or greater than about 90% by weight maltitol on a dry basis). Sugar alcohol syrups are generally prepared by heating a solution of the sugar alcohol in water and cooling the mixture to give a viscous composition. The resulting syrup is typically characterized by a relatively high concentration of sugar alcohol and relatively high stability (i.e., the sugar alcohol typically does not crystallise from solution, e.g., at room temperature).

The syrup, e.g., sugar alcohol syrup, desirably is capable of affecting the recrystallisation of a melted sugar substitute. One example sugar alcohol syrup that is particularly useful according to the present disclosure is maltitol syrup. Other sugar alcohol syrups can be used, including, but not limited to, com syrup, golden syrup, molasses, xylitol, mannitol, glycerol, erythritol, threitol, arabitol, ribitol, mannitol, sorbitol, dulcitol, iditol, isomalt, lactitol, and polyglycitol syrups. Such sugar alcohol syrups can be prepared or can be obtained from commercial sources. For example, maltitol syrups are commercially available from such suppliers as Corn Products Specialty Ingredients. Although sugar alcohol syrups may be preferred, sugar syrups can, in certain embodiments, be used in place of or in combination with the sugar alcohol syrup. For example, in some embodiments, corn syrup, golden syrup, and/or molasses can be used.

The amount of sugar alcohol syrup added to the lozenge composition mixture is typically that amount required to slow recrystallisation of the sugar substitute in melted form. It should be noted that it may be possible to vary the amount of sugar alcohol syrup depending on the composition of the remaining ingredients to ensure that the recrystallisation is sufficiently slow to provide a material with the desired characteristics (e.g., a desired level of translucency/transparency).

Accordingly, the amount of sugar alcohol syrup can vary, but typically ranges from about 0.1% to about 2%, often from about 0.5% to about 1.5%, and more often about 1% by weight of the lozenge product mixture. In certain embodiments, the amount of sugar alcohol syrup is higher, for example, up to about 2% by weight of the mixture, up to about 5% by weight of the mixture, up to about 10% by weight of the mixture, or up to about 20% by weight of the mixture.

Representative lozenge compositions and products may incorporate about 10% by weight or less of the combination of active ingredients, about 0.01 to about 2% artificial sweetener, about 1% to about 5% humectant, about 1% to about 5% natural sweetener, at least about 80% of a sugar substitute, about 0.1% to about 10% of a sugar alcohol syrup, one or more flavorants in an amount of up to about 5%, and salt in an amount up to about 3%, based on the total weight of the product. The particular percentages and choice of ingredients will vary depending upon the desired flavor, texture, and other characteristics.

Oral products of the present disclosure in the form of a lozenge may contain various amounts of water. The water content of the lozenge described herein, prior to use by a consumer of the product, may vary within such ranges according to the desired properties and characteristics, in addition to dictating the final form of the product. For example, lozenge-type products typically possess a water content in the range of about 0.1 to about 5% by weight of the product. Preferably, the moisture content of a lozenge product, as present within a single unit of product prior to insertion into the mouth of the user, is less than about 5%, less than about 3%, less than about 2%, or less than about 1% by weight of the product. In some embodiments, the moisture content of a lozenge product as described herein may be within the range of about 0.1% to about 5%, about 0.5 to about 3%, or about 1 to about 2 % by weight of the product.

*Powders or pouched products*

In some embodiments, the oral product may be in the form of a powder. The powder may be a free-flowing powder. The powder may be contained in loose form within a container, and may thus be used in a form similar to tobacco snuff where the user takes a pinch of powder from the container and places the powder in the oral cavity. Alternatively or additionally, the powder may be incorporated into a moisture-permeable (e.g. saliva-permeable) pouch, similar to a snus-type product. The pouched product may be configured for insertion into the oral cavity of a user; i.e. it may be a pouched oral product.

In some embodiments, the product of the present disclosure is in the form of a pouched oral product. Such a pouched product comprises the oral product as described herein, disposed within a moisture-permeable container (e.g., a water-permeable pouch or saliva-permeable pouch). For example, the pouched product may comprise the oral product in a powder form incorporated within the saliva-permeable pouch.

Such compositions in the moisture-permeable pouch format are typically used by placing one pouch containing the composition in the mouth of a human subject/user. Generally, the pouch is placed somewhere in the oral cavity of the user, for example under the lips, in the same way as moist snuff products are generally used. The pouch preferably is not chewed or swallowed. Exposure to saliva then causes some of the components of the composition therein (e.g., the active ingredients and/or any flavours) to pass through e.g., the moisture-permeable pouch and provide the user with flavour and satisfaction, and the user is not required to spit out any portion of the composition. After about 10 minutes to about 60 minutes, typically about 15 minutes to about 45 minutes, of use/enjoyment, substantial amounts of the composition have been ingested by the human subject, and the pouch may be removed from the mouth of the human subject for disposal.

In some embodiments, the pouch is saliva-permeable. This means that the pouch is made of a saliva-permeable pouch material. The pouch material used in oral pouched products is typically a dry-laid bonded nonwoven comprising viscose rayon fibres (i.e. regenerated cellulose) and an acrylic polymer that acts as binder in the nonwoven material and provides for heat-sealing of the pouches during manufacturing thereof. The pouch material may also comprise synthetic fibres (e.g. polyester) in addition to viscose fibres. The viscose nonwoven material normally used for smokeless tobacco pouches is similar to the fabric used in tea bags. Nonwovens are fabrics that are neither woven nor knitted. Methods for the manufacturing of nonwoven materials are commonly known in the art. Further information on nonwovens is found in "Handbook of Nonwovens" by S. Russel, published by Woodhead Pub I. Ltd., 2007. In some embodiments, the pouch material is a fleece material. In some embodiments, the pouch material is a non-woven material. In some embodiments, the pouch material is a non-woven fleece material. In some embodiments, the pouch material comprises viscose, such as viscose rayon fibres. In some embodiments, the pouch material comprises regenerated cellulose fibres. In some embodiments, the pouch material comprises polyester fibres; the polyester fibres may constitute the pouch material or may be included in combination with viscose (such as regenerated cellulose fibres).

In some embodiments, the pouch material comprises a binder that provides for heat sealing of the pouches during manufacture. In some embodiments, the pouch material comprises an acrylic binder. In some embodiments, the pouch material comprises an acrylic binder in combination with viscose and/or polyester fibres.

Suitable packets, pouches or containers of the type used for the manufacture of smokeless tobacco products are available under the tradenames CatchDry, Ettan, General, Granit, Goteborgs Rape, Grovsnus White, Metropol Kaktus, Mocca Anis, Mocca Mint, Mocca Wintergreen, Kicks, Probe, Prince, Skruf and TreAnkrare. The composition may be

contained in pouches and packaged, in a manner and using the types of components used for the manufacture of conventional snus types of products. The pouch provides a moisture-permeable container of a type that may be considered to be similar in character to the mesh-like type of material that is used for the construction of a tea bag. Components of the composition readily diffuse through the pouch and into the mouth of the user. Non-limiting examples of suitable types of pouches are set forth in, for example, US Pat. Nos. 5,167,244 to Kjerstad and 8,931 ,493 to Sebastian etal.; as well as US Patent App. Pub. Nos. 2016/0000140 to Sebastian et al.; 2016/0073689 to Sebastian et al.; 2016/0157515 to Chapman et al.; and 2016/0192703 to Sebastian et al., each of which is incorporated herein by reference. Pouches can be provided as individual pouches, or a plurality of pouches (e.g., 2, 4, 5, 10, 12, 15, 20, 25 or 30 pouches) can be connected or linked together (e.g., in an end-to-end manner) such that a single pouch or individual portion can be readily removed for use from a one-piece strand or matrix of pouches.

An example pouch may be manufactured from materials, and in such a manner, such that during use by the user, the pouch undergoes a controlled dispersion or dissolution. Such pouch materials may have the form of a mesh, screen, perforated paper, permeable fabric, or the like. For example, pouch material manufactured from a mesh-like form of rice paper, or perforated rice paper, may dissolve in the mouth of the user. As a result, the pouch and composition each may undergo complete dispersion within the mouth of the user during normal conditions of use, and hence the pouch and composition both may be ingested by the user. Other examples of pouch materials may be manufactured using water dispersible film forming materials (e.g., binding agents such as alginates, carboxymethylcellulose, xanthan gum, pullulan, and the like), as well as those materials in combination with materials such as ground cellulosics (e.g., fine particle size wood pulp). Preferred pouch materials, though water dispersible or dissolvable, may be designed and manufactured such that under conditions of normal use, a significant amount of the composition contents permeate through the pouch material prior to the time that the pouch undergoes loss of its physical integrity. If desired, flavoring ingredients, disintegration aids, and other desired components, may be incorporated within, or applied to, the pouch material.

The amount of the oral product contained within each pouched product unit, for example, a pouch, may vary. In some embodiments, the weight of the composition within each pouch is at least about 50 mg, for example, from about 50 mg to about 1 gram (1,000 mg), such as from about 100 mg to about 900 mg, such as from about 200 mg to about 800 mg, such as from about 500 mg to about 700 mg. In some smaller embodiments, the weight of the composition within each pouch may be from about 100 mg to about 300 mg. For a larger embodiment, the weight of the composition within each pouch may be from about 300 mg to about 700 mg. If desired, other components can be contained within each pouch.

The moisture content of the oral product may vary depending on the format in which the composition is provided. In some embodiments as described hereinabove, the oral product may be in the form of moist snuff or snus and/or may be provided in pouched formats. In some embodiments (e.g. for snus-type products), the moisture content of the composition (before insertion of the product into the user's mouth) may be at least about 20% by weight, such as at least 30% by weight, such as at least 40% by weight, such as at least 50% by weight of the oral product. In some embodiments (for example, for snus-type products; e.g. non-pouched or pouched snus products), the moisture content of the composition (before insertion of the product into the user's mouth) may be from about 20% to about 70% by weight, such as from about 30% to about 60% by weight, such as from about 40% to about 55% by weight of the oral product.

In some embodiments, the oral product may be a snus-type or snuff-type product that is in 'dry' form. In such embodiments, the moisture content of the oral product may be no greater than about 10% by weight, such as no greater than about 5% by weight of the oral product. For example, the moisture content may be from about 0.1% to about 10% by weight, such as from about 1% to about 5% by weight of the oral product.

When in the form of a pouched oral product, the oral product typically comprises a filler. The filler may preferably be a cellulose material selected from the suitable materials described hereinabove. In some preferred embodiments, the filler is or comprises at least MCC. The amount of filler can vary, but is typically at least about 5% to about 95% by weight of the oral product, based on the total weight of the oral product. In some embodiments, the filler (such as a cellulose material, such as MCC) may be present in the oral product in an amount of from about 5% to about 95% by weight, such as from about 10% to about 90% by weight, such as from about 15% to about 85% by weight, such as from about 20% to about 80% by weight, such as from about 25% to about 75% by weight, such as from about 30% to about 70% by weight, such as from about 35% to about 65% by weight, such as from about 40% to about 60% by weight of the oral product. In some embodiments, the filler (such as a cellulose material, such as MCC) may be present in an amount of from about 45% to about 55% by weight of the oral product.

Package

According to some embodiments described herein, there is provided a package containing an oral product as described herein. For example, the package may contain the oral product in powdered form. In such embodiments, the package may be in the form of a tin or plastic container. Alternatively or additionally, the package may contain the oral product in the form of a chew, lozenge, pastille, tablet, or the like. The package may be in the form of a blister pack, tin or plastic container containing such oral dosage forms.

According to some embodiments described herein, there is provided a package containing at least one pouched oral product as described herein. A pouched product as described herein can be packaged within any suitable inner packaging material and/or outer container. See also, for example, the various types of containers for smokeless types of products that are set forth in US Pat. Nos. 7,014,039 to Henson et al.; 7,537,110 to Kutsch et al.; 7,584,843 to Kutsch et al.; 8,397,945 to Gelardi et al., D592,956 to Thiellier; D594,154 to Patel et al.; and D625,178 to Bailey et al.; US Pat. Pub. Nos. 2008/0173317 to Robinson et al.; 2009/0014343 to Clark et al.; 2009/0014450 to Bjorkholm; 2009/0250360 to Bellamah et al.; 2009/0266837 to Gelardi et al.; 2009/0223989 to Gelardi; 2009/0230003 to Thiellier; 2010/0084424 to Gelardi; and 2010/0133140 to Bailey et al; 2010/0264157 to Bailey et al.; and 2011/0168712 to Bailey et al. which are incorporated herein by reference. For example, the package may be a tin or plastic container which contains a plurality of the pouched oral products.

**Processes**

The manner by which the various components of the composition (e.g., active ingredients and any additives) are combined may vary. The components noted above, which may be in liquid or dry solid form, can be admixed in a pre-treatment step prior to mixture with any remaining components of the product, or simply mixed together with all other liquid or dry ingredients.

The various components of the product may be contacted, combined, or mixed together using any mixing technique or equipment known in the art. Any mixing method that brings the product ingredients into intimate contact can be used, such as a mixing apparatus featuring an impeller or other structure capable of agitation. Examples of mixing equipment include casing drums, conditioning cylinders or drums, liquid spray apparatus, conical-type blenders, ribbon blenders, mixers available as FKM130, FKM600, FKM1200, FKM2000 and FKM3000 from Littleford Day, Inc., Plough Share types of mixer cylinders, Hobart mixers, and the like. See also, for example, the types of methodologies set forth in US Pat. Nos. 4,148,325 to Solomon et al.; 6,510,855 to Korte et al.; and 6,834,654 to Williams, each of which is incorporated herein by reference. Manners and methods for formulating products will be apparent to those skilled in the art. See, for example, the types of methodologies set forth in US Pat. No. 4,148,325 to Solomon et al.; US Pat. No. 6,510,855 to Korte et al.; and US Pat. No. 6,834,654 to Williams, US Pat. Nos. 4,725,440 to Ridgway et al., and 6,077,524 to Bolder et al., each of which is incorporated herein by reference.

*Method of preparing liquid products*

In accordance with some embodiments described herein, there is provided a process for preparing an oral product as described herein, the oral product preferably being in the form of a liquid, the process comprising the steps of:

(a) combining active ingredients,

(b) contacting the active ingredients with water, and

(c) mixing the active ingredients with water to prepare an oral product.

The combination of active ingredients may be as described hereinabove.

The active ingredients may be provided in the form of a liquid extract, a liquid oil or a powder. When in the form of a powder, step (c) may comprise mixing the active ingredients with water until said active ingredients have dissolved in the water.

Step (b) and/or step (c) may preferably be carried out at ambient or room temperature (20-25°C). Alternatively, the temperature may be elevated in order to assist with dispersion or dissolution of the active ingredients in the water. For example, step (b) may comprise contacting the active ingredients with water at a temperature of from about 20-100°C, such as from about 30-90°C, or from about 40-80°C. Step (b) may comprise mixing the active ingredients with water at a temperature of from about 20-1 00°C, such as from about 30-90°C, or from about 40-80°C.

The process may comprise adding any additional additives at any stage. For example, any additives may be added to the active ingredients prior to combination with water and/or after the active ingredients have been combined with water. The additives may also be added to the water prior to contacting the water with the combination of active ingredients. Step (a) may comprise an optional step of combining any additives (e.g. acidifying agent) with the combination of active ingredients. Step (b) may comprise an optional step of contacting the active ingredients with water and an additive. Step (c) may comprise an optional step of adding additives to the mixture and mixing the additives with the combination of active ingredients and water.

In some embodiments, the oral product further comprises one or more additives. In such embodiments, the one or more additives may be combined with water prior to addition of the active ingredients. Optionally, the step of combining

the one or more additives with water may comprise heating the combination, e.g. to a temperature of from about 60°C to about 80°C, to achieve dissolution of the additive(s). Further additives, such as a sweetener, humectant, colouring agent or the like, may be added at this stage or may be added during and/or after the step of combining the active ingredients with the water.

The resulting liquid product may be in the form of a solution or dispersion of active ingredients in water. Preferably the liquid product is in the form of a solution of active ingredients in water.

*Method of preparing chew products*

As described herein, the product may be in chewable form. In such embodiments, the process may comprise the steps of:

(a) contacting at least one binding agent and at least one bulking agent selected from the group consisting of a sugar alcohol, or a sugar, or a combination of at least one sugar alcohol and at least one sugar, and optionally adding water;

(b) heating the mixture of binding agent, sugar alcohol and/or sugar, and optionally water;

(c) adding a combination of active ingredients;

(d) allowing the resulting mixture to set to provide an oral chew product.

The combination of active ingredients may be as described hereinabove.

Step (b) of heating the mixture may comprise heating the mixture to a temperature of about 70°C to about 150°C, such as from about 80°C to about 125°C, such as from about 90°C to about 100°C. Step (b) may comprise heating the mixture to boiling, optionally while stirring the mixture. Step (d) preferably comprises a cooling step. In some embodiments, the resulting mixture from step (c) is allowed to cool in order to set to provide an oral chew product. For example, after the active ingredients are added in step (c), the resulting mixture may be deposited into a mold and the heat removed. The mixture may be allowed to cool to room temperature passively. Alternatively, the mixture may be placed into a cool water bath, fridge or freezer in order to reduce the temperature. Preferably, cooling happens at room temperature to allow the product to set.

The combination of active ingredients may be added during the heating step (b). In some embodiments, the combination of active ingredients is added while the mixture of binding agent, sugar alcohol and/or sugar and optionally water is boiling. Alternatively, the combination of active ingredients may be added after cooling the mixture of binding agent, sugar alcohol and/or sugar and optionally water.

Any additives may be added at any stage during the above process. In some embodiments, an acidifying agent is added after removing the mixture from the heat; i.e. during step (c) or (d). In some embodiments, a flavouring agent and/or colouring agent is added after removing the mixture from the heat; i.e. during step (c) or (d). In some embodiments, a sweetener is added during step (a). The sweetener may therefore be included in the mixture that is heated in step (b).

For the preparation of the product in chewable form, generally, a binder (e.g. pectin, agar, carrageenan, starch, or a combination thereof) is pre-blended with all or a portion of the sugar alcohol/sugar, sweetener, or combination thereof). Water is added, and the mixture heated to boiling with stirring. Any remaining sugar alcohol or sweetener is added to the boiling mixture, along with the active ingredients, followed by any buffering agent. The mixture is cooked to a degrees brix from about 50 to about 80. Heat is removed, and any acidifying agent and/or flavorant added, along with any colorant, and the mixture thoroughly combined. The composition is deposited into molds for storage at ambient temperature.

In some embodiments, the composition is deposited in a starch mold. Starch trays with molded shapes are prepared and pre-heated at 60°C for at least 1- 2 hours. The starch can be any starch as disclosed herein above. In some embodiments, the starch is corn starch. In some starch molded embodiments, pectin binder is pre-blended with a portion of the sugar alcohol (e.g. isomalt or maltitol). Water is added, and the mixture heated to boiling with stirring. Further sugar alcohol (e.g. maltitol syrup and/or isomalt) are added to the boiling mixture, along with the active ingredients. The mixture is cooked to around 78 brix. Heat is removed, and any sweetener (e.g., sucralose or acesulfame K) and flavorant added, along with any colorant and acidifying agent (e.g. citric acid solution), and the mixture thoroughly combined. The hot mixture is deposited into starch molds for storage at ambient temperature. The resulting chews are removed from the starch mold, and any excess starch removed.

In other starch molded embodiments, a gum powder (e.g. pectin, agar, carrageenan, starch, or a combination thereof) is mixed with water until lump-free. Sugar alcohol and/or sugar (e.g. isomalt and/or maltitol syrup) and any sweetener (e.g. sucralose) are mixed together and the mixture heated to 82-104 °C. The gum powder solution is added into the sugar alcohol/sugar and mixed thoroughly. The active ingredient(s), and any colour and flavour are added to above solution and mixed thoroughly. The mixture is cooked at 93-104°C until a degrees brix of 50-80 is achieved. A solution of

acidifying agent (e.g. citric acid and/or trisodium citrate dihydrate) in water is prepared and added to the hot mixture. Any gelling agents (e.g. dicalcium phosphate solution) is then added into the mixture if necessary. The hot mixture is deposited into the prepared starch molds and kept in an oven at 60°C overnight, or until proper setting is achieved. The resulting chews are removed from the starch mold, and any excess starch removed. In some embodiments, the chews are coated, e.g. with coating oil or CAPOL 410 (available from Centerchem, Inc.). The coating process may be carried out in the same manner as described in detail below with respect to pastille products.

In other embodiments, the composition is deposited in a starchless mold. In such embodiments, a gum powder (e.g. pectin, agar, carrageenan, starch, or a combination thereof) is mixed with water until lump free. Maltitol syrup, sucralose, and optionally isomalt, are mixed together and the mixture heated to 82-104 °C. The gum powder solution is added into the maltitol solution and mixed thoroughly. The active ingredient(s), and any colour and flavour are added to and the mixture mixed thoroughly. The mixture is cooked at 93-104°C until a degrees brix of 50-80 is achieved. A solution of citric acid and optionally trisodium citrate dihydrate in water is prepared and added to the hot mixture to achieve a pH between 2.5 and 4. Any gelling agents (e.g. dicalcium phosphate solution) is then added into the mixture if necessary. The hot mixture is deposited into the starchless molds and left at room temperature, until proper setting is achieved.

The chew product may be held in the mold (starch or starchless) for a predetermined duration of time such as, for example, about 10 minutes to about 24 or even 48 hours, so as to allow the chew product to cure and solidify.

According to other aspects of the present disclosure, rather than using molds to prepare the chew product, an extrusion process may be employed in which the final chew product is extruded as described hereinbelow with respect to pastille extrusion methods.

*Method of preparing pastille products*

The manners and methods used to formulate and manufacture a pastille product as described herein can vary. For example, the compositions forming the pastille products may be prepared such that the mixture thereof may be used in a starch molding process for forming the pastille product. Example pastille production processes are set forth in US Pat. Nos. 4, 725,440 15 to Ridgway et al and 6,077,524 to Bolder et al., which are incorporated by reference herein. In some embodiments, the compositions for forming the pastille products may be prepared such that the mixture thereof may be used in a starchless molding process (e.g., not including a starch-based component in the molding process) for forming the pastille product.

In some embodiments, the process comprises heating a gum, and optionally hydrating that gum component with water, and then stirring the combination of active ingredients into the heated gum component. Generally, the gum may be heated to a temperature in the range of about 60°C to about 80°C for a period of a few seconds to a few minutes. In some embodiments, the gum may be heated to a temperature of about 71 °C before stirring in the combination of active ingredients, to allow the active ingredients to dissolve therein. In some instances, an aqueous mixture is formed in a separate container by mixing one or more additives (e.g., such as salts, sweeteners, humectants, emulsifiers, flavouring agents, and others) with water to form the aqueous mixture. Then, the aqueous mixture may be admixed with the heated gum (including the at least one active ingredient that has been added therein) to form a mixture in the form of a slurry. In some embodiments, the at least one sugar alcohol/sugar component may be added separately to this mixture, or, in other embodiments, the at least one sugar alcohol/sugar may be combined with the gum and the active ingredient prior to addition to the mixture. In some instances, the at least one sugar alcohol/sugar may be heated in yet another separate container and added to the mixture separately. For example, in some embodiments, the at least one sugar alcohol/sugar (which may optionally include isomalt/maltitol/erythritol) may be heated to a temperature in the range of about 160°C to about 190°C before addition to the mixture. In some embodiments, the at least one sugar alcohol/sugar may be heated to a temperature of at least about 160°C, at least about 170°C, at least about 180°C, or at least about 190°C. In some instances, the heated sugar alcohol/sugar may be allowed to cool to a temperature in the range of about 120°C to about 160°C prior to addition to the mixture. In some embodiments, for example, the heated sugar alcohol/sugar may be cooled to a temperature of about 160°C or less, about 150°C or less, about 140°C or less, or about 130°C or less prior to addition to the mixture.

In some instances, the heated (and optionally cooled) sugar alcohol/sugar may be combined with the mixture (e.g., including the heated gum, the at least one active ingredient, and the aqueous mixture) and stirred using a high shear mixer or a Hobart mixing bowl with a whipping attachment to provide a pastille composition, which may also be in the form of a slurry. The pastille composition may then be heated to an elevated temperature for a period of time, for example, heated to between about 40°C to about 80°C, and typically heated to about 71 °C, for a period of about 1 to about 3 minutes, for example, to dissolve any dry ingredient within the pastille composition. The heating step can be characterized as heating at a temperature of at least about 50°C, at least about 60°C, or at least about 70°C. The pastille composition may typically have a moisture content of at least about 40 percent by weight water, based on the total weight of the composition.

According to some aspects, the pastille composition, in the form of a slurry, may optionally be put through a deaerating step or process prior to being received in a mold or being subjected to other processing steps, so as to reduce or

eliminate air bubbles present in the slurry mixture. Air bubbles entrapped within the slurry may affect the final weight of the pastille product, which could lead to a lack of weight uniformity between units of the final product. As such, any deaerating methods and systems may be employed for removing such air bubbles from the slurry material. For example, the slurry may be placed under reduced pressure (i.e., below atmospheric pressure) to pull the air bubbles out of the slurry mixture. In some instances, a vacuum deaerating process may be employed in which the slurry mixture is placed in a vacuum deaerator for deaerating the slurry mixture using pressure reduction. In some instances, the slurry mixture may be under vacuum for about 1 to about 10 minutes, and typically for about 3 to about 5 minutes. The deaerating step may be observed and adjusted accordingly in order to controllably remove the gaseous components from the slurry mixture.

The viscosity of the heated and deaerated slurry mixture may be measured using, for example, a Brookfield viscometer HA Series, SC4 water jacket, 27/13R sample chamber and a No. 27 spindle. The pastille composition may have a viscosity of about 5.7 Pascal-seconds (Pa·s) to about 6.2 Pa·s when heated to a temperature of about 38°C, about 4.9 Pa s to about 5.4 Pa·s when heated to a temperature of about 43°C, and about 4.2 Pa·s to about 4.7 Pa·s when heated to a temperature of about 50°C. In some instances, extra water may be added to the pastille composition so as to provide a desired viscosity thereof. Once the desired viscosity is achieved, the heated pastille composition may then be deposited into a mold, such as, for example, a starch mold. While the process as further described herein is directed to forming a pastille product using a starch mold, it is noted that other types of molds may be used in the process, such as, for example, starchless molds, pectin molds, plastic tray molds, silicone tray molds, metallic tray molds, neoprene tray molds, and the like.

In instances involving the use of starch molds, the starch molds may be pre-dried to remove moisture content from the starch mold itself. That is, prior to receiving the slurry or viscous pastille composition, the starch mold may be subjected to an elevated temperature to drive out moisture in the starch mold. For example, in some instances, the starch mold may initially have a moisture content of about 10-15% by weight. Such levels of moisture could potentially have an effect on the uniformity of the resultant product. In this regard, certain moisture levels in the starch mold could potentially have a wrinkling or pruning effect on the product such that the final product has a shrivelled or otherwise wrinkled appearance. As such, the starch mold may be dried at an elevated temperature to reduce the moisture content of the starch mold to between about 4 and about 10% by weight, and preferably between about 6 and about 8% by weight, based on the total weight of the starch mold. By taking such steps, the product may, in some instances, be more uniformly consistent in appearance. Furthermore, the starch mold may be heated to an elevated temperature prior to receiving the pastille composition such that the starch mold itself is at an elevated temperature when receiving the pastille composition.

The pastille composition may remain in the starch mold at an elevated temperature such as, for example, at between about 40°C to about 80°C (e.g., at least about 40°C or at least about 50°C), and typically at about 60°C. The pastille composition may be held at the elevated temperature for a predetermined duration of time such as, for example, about 12- 48 hours, and typically about 24 hours, so as to allow the pastille composition to cure and solidify into pastille form, while driving the moisture content of the pastille composition to a desired final moisture level. As noted above, in some embodiments, the desired final moisture level of the pastille product may be within the range of about 5 to about 25% by weight, or about 8 to about 20% by weight, or about 10 to about 15% by weight, based on the total weight of the product unit. In this regard, curing generally refers to the solidification process in which moisture loss occurs, the viscosity of the composition is raised, and chemical and physical changes begin to occur (e.g., crystallisation, crosslinking, gelling, film forming, etc.). The pastille composition is allowed to cool and thereafter removed from the starch mold. In some instances, the pastille composition may be allowed to cool at refrigerated or below ambient temperatures. An air blower / shaker device can be used to remove starch remnants from the pastille composition after being removed from the starch mold.

The pastille product is then allowed to post-cure for a time and at a temperature suitable to allow the product to become equilibrated to a desired moisture, shape and form. The time and temperature can vary without departing from the invention and depend in part on the desired final characteristics of the product. In one embodiment, the post-cure is conducted at ambient temperature for at least about 20 hours after being removed from the mold.

A pastille product may be provided in individual pieces weighing between about 0.5 grams to about 5 grams, although aspects of the present disclosure are not limited to such weights.

The curing times and temperatures of the pastille product can be varied as desired. In this regard, such variables may affect the final visual appearance of the pastille product. For example, extended curing times and/or low curing temperatures may affect the final outer configuration or contours of the pastille product. That is, the rate of drying and/or curing of the product can affect the final properties of the product. In some instances, for example, lowering the curing temperature and extending the curing time may cause the pastille product to have a relatively smooth outer surface. In contrast, curing at higher temperatures for shorter period of times can lead to a roughened or wrinkled appearance in the product.

According to other aspects of the present disclosure, rather than using molds to prepare the pastille product, an extrusion process may be employed in which the final pastille product is extruded. In some instances, the pastille composition in slurry form may be formed into a sheet and allowed to dry to a moisture content, for example, of about 15 percent to about 25 percent by weight water to form a tacky or otherwise pasty material, which is in a form capable of

physical handling. The material may then be chopped or otherwise cut into smaller pieces using, for example, a mixer. The chopped material may then be extruded through an extrusion device to any shape/size desired, including shapes that may be difficult or impossible to achieve with a mold. In some instances, the extruded product may then be dried to achieve a desired moisture content. A similar type process is described, for example, in U.S. Pat. No. 3,806,617 to Smylie et al., which is incorporated herein by reference in its entirety. Further, the pastille composition may be subjected to a co-extrusion process with another composition.

Shapes such as, for example, rods and cubes can be formed by first extruding the material through a die having the desired cross-section (e.g., round or square) and then optionally cutting the extruded material into desired lengths. Techniques and equipment for extruding tobacco materials are set forth in US Pat. Nos. 3,098,492 to Wursburg; 4,874,000 to Tamol et al.; 25 4,880,018 to Graves et al.; 4,989,620 to Keritsis et al.; 5,072,744 to Luke et al.; 5,829,453 to White et al.; and 6,182,670 to White et al.; each of which is incorporated herein by reference. Example extrusion equipment suitable for use include food or gum extruders, or industrial pasta extruders such as Model TP 200/300 available from Emiliomiti, LLC of Italy. In some instances, a single machine may be capable of achieving multiple steps of the processes described herein, such as, for example, kneader systems available from Buss AG.

The pastille product can be provided in any suitable predetermined shape or form, and most preferably is provided in the form having a general shape of a pill, pellet, tablet, coin, bead, ovoid, obloid, cube, or the like. The mouthfeel of the pastille product preferably has a slightly chewable and dissolvable quality with a mild resilience or "bounce" upon chewing that gradually leads to greater malleability during use. According to one aspect, the pastille product is preferably capable of lasting in the user's mouth for about 10-15 minutes until it completely dissolves. Preferably, the products do not, to any substantial degree, leave any residue in the mouth of the user thereof, and do not impart a slick, waxy, or slimy sensation to the mouth of the user.

According to some embodiments, the pastille composition may be coated with a coating substance after being removed from the starch mold and prior to drying. For example, a glazing or anti-sticking coating substance, such as, for example, CAPOL 410 (available from Centerchem, Inc.), may be applied to the pastille composition to provide free-flowing properties. The coating composition may comprise an oil or wax; for example, sunflower oil and/or carnuba wax.

Outer coatings can also help to improve storage stability of the pastille products of the present disclosure as well as improve the packaging process by reducing friability and dusting. Devices for providing outer coating layers to the products of the present disclosure include pan coaters and spray coaters, and particularly include the coating devices available as CompuLab 24, CompuLab 36, Accela-Cota 48 and Accela-Cota 60 from Thomas Engineering. An example outer coating comprises a film-forming polymer, such as a cellulosic polymer, an optional plasticizer, and optional flavorants, colorants, salts, sweeteners or other additives of the types set forth herein. The coating compositions are usually aqueous in nature and can be applied using any pellet or tablet coating technique known in the art, such as pan coating. Example film-forming polymers include cellulosic polymers such as methylcellulose, hydroxypropyl cellulose (HPC), hydroxypropyl methylcellulose (HPMC), hydroxyethyl cellulose, and carboxy methylcellulose. Example plasticizers include aqueous solutions or emulsions of glyceryl monostearate and triethyl citrate.

In one embodiment, the coating composition comprises up to about 75% by weight of a film-forming polymer solution (e.g., about 40 to about 70% by weight based on total weight of the coating formulation), up to about 5% by weight of a plasticizer (e.g., about 0.5 to about 2% by weight), up to about 5% by weight of a sweetener (e.g., about 0.5 to about 2% by weight), up to about 10% by weight of one or more colorants (e.g., about 1 to about 5% by weight), up to about 5% by weight of one or more flavorants (e.g., about 0.5 to about 3 % by weight), up to about 2% by weight of a salt such as NaCl (e.g., about 0.1 to about 1 5 % by weight), and the balance water. Example coating compositions and methods of application are described in U.S. Application No. 12/876,785 to Hunt et al.; filed September 7, 2010, and which is incorporated by reference herein.

Although the foregoing description focuses on compositions that are uniform throughout each product unit, products can also be formed with multiple different formulations having different properties in the same product unit. For example, two different compositions can be deposited in a single mold to produce a layered product. Still further, two different compositions could be co-extruded to form a product with different characteristics across its cross-section. Such a process could be used to provide a product with two different compositions featuring different dissolution rates such that a first portion of the product dissolves at a first rate (e.g., a faster rate) and a second portion dissolves at a second, slower rate.

*Method of preparing tablet products*

In some embodiments, the product is in the form of a compressed pellet or tablet. In one embodiment, the process for making the pellet or tablet involves first mixing the bulk filler (e.g., EMDEX®) and the active ingredients. The remaining composition ingredients (e.g., sugar alcohol and any other desired components, such as binders, colorants, sweeteners, flavors, and the like) are then added. Optionally, a colorant can may be added to one of the composition components in a separate step prior to mixing with the remaining components of the composition. The mixing of the composition can be

accomplished using any mixing device. The final composition is then compressed into pellet or tablet form using conventional tableting techniques and optionally coated. Compressed composition pellets can be produced by compacting the composition, including any associated formulation components, in the form of a pellet, and optionally coating each pellet with an overcoat material. Example compaction devices, such as compaction presses, are available as Colton 2216 and Colton 2247 from Vector Corporation and as 1200i, 2200i, 3200, 2090, 3090 and 4090 from Fette Compacting. Devices for providing outer coating layers to compacted pelletized compositions are available as CompuLab 24, CompuLab 36, Accela-Cota 48 and Accela-Cota 60 from Thomas Engineering.

When present, a coating typically comprises a film-forming polymer, such as a cellulosic polymer, an optional plasticizer, and optional flavorants, colorants, salts, sweeteners or other additives of the types set forth herein. The coating compositions are usually aqueous in nature and can be applied using any pellet or tablet coating technique known in the art, such as pan coating. Example film-forming polymers include cellulosic polymers such as methylcellulose, hydroxypropyl cellulose (HPC), hydroxypropyl methylcellulose (HPMC), hydroxyethyl cellulose, and carboxy methylcellulose. Example plasticizers include aqueous solutions or emulsions of glyceryl monostearate and triethyl citrate. Additional potential coatings include food grade shellac, waxes such as carnuaba wax, and combinations thereof.

*Methods of preparing lozenge products*

The manners and methods used to formulate and manufacture a lozenge product as described herein above can vary. For example, the compositions can be prepared via any method commonly used for the preparation of hard boiled confections. Example methods for the preparation of hard confections can be found, for example, in LFRA Ingredients Handbook, Sweeteners, Janet M. Dalzell, Ed., Leatherhead Food RA (Dec. 1996), pp. 21-44, which is incorporated herein by reference.

Typically, a first mixture of ingredients is prepared. The composition of the first mixture of ingredients can vary; however, it typically comprises a sugar substitute and may contain various additional substances (e.g., the sugar alcohol syrup, NaCl, preservatives, further sweeteners, water, and/or flavourings). In certain embodiments, it comprises the sugar substitute, salt, and vanillin. In other embodiments, the first mixture comprises the sugar substitute and the sugar alcohol syrup. Typically, the first mixture of ingredients does not contain the active ingredients; although, it some embodiments, the active ingredients may be incorporated into the first mixture of ingredients.

The first mixture of ingredients is heated until it melts; subsequently, the mixture is heated to or past the hard crack stage. In confectionary making, the hard crack stage is defined as the temperature at which threads of the heated mixture (obtained by pulling a sample of cooled syrup between the thumb and forefinger) are brittle or as the temperature at which trying to mold the syrup results in cracking. According to the present method, the temperature at which the hard crack stage is achieved can vary, depending on the specific makeup of the product mixture but generally is between about 145°C and about 170°C. Typically, the mixture is not heated above about 171 °C, which is the temperature at which caramelisation begins to occur. In the processes of the present disclosure, the mixture is typically heated to the hard crack stage temperature or above and then allowed to cool. The heating can be conducted at atmospheric pressure or under vacuum. Typically, the method of the present invention is conducted at atmospheric pressure.

In one example embodiment, the first mixture of ingredients comprises a high percentage of isomalt and the mixture is heated to about 143°C. Once all components are dissolved, the temperature is raised past the hard crack stage (e.g., to about 166°C). The mixture is heated to this temperature and then removed from the heat to allow the mixture to cool.

In certain embodiments, the active ingredients and, optionally, additional components (e.g., additional sweeteners, fillers, flavouring agents, and water) as described above are separately combined in a second mixture. The second mixture is added to the first mixture of ingredients, typically after the first mixture of ingredients has been removed from the heat. The addition of the second mixture may, in some embodiments, occur only after the heated first mixture of ingredients has cooled to a predetermined temperature (e.g., in certain embodiments, to about 132 °C). In certain embodiments, one or more flavouring agents are added to the second mixture immediately prior to adding the mixture to the first, heated mixture of ingredients. Certain flavouring agents are volatile and are thus preferably added after the mixture has cooled somewhat. The combined mixture is then formed into the desired shape. In certain embodiments, the mixture is poured directly into molds, formed (e.g., rolled or pressed) into the desired shape, or extruded. If desired, the mixture can be extruded or injection molded. In certain embodiments, the mixture is formed or extruded into a mold of desired shape in an enclosed system, which may require decreased temperature and which may limit evaporation of certain mixture components. For example, such a system may limit the evaporation of volatile components including, but not limited to, flavorants. Other methods of producing lozenges are also intended to be encompassed herein.

Typical conditions associated with manufacture of food-grade lozenge products such as described herein include control of heat and temperature (i.e., the degree of heat to which the various ingredients are exposed during manufacture and the temperature of the manufacturing environment), moisture content (e.g., the degree of moisture present within individual ingredients and within the final composition), humidity within the manufacturing environment,

atmospheric control (e.g., nitrogen atmosphere), airflow experienced by the various ingredients during the manufacturing process, and other similar types of factors. Additionally, various process steps involved in product manufacture can involve selection of certain solvents and processing aids, use of heat and radiation, refrigeration and cryogenic conditions, ingredient mixing rates, and the like. The manufacturing conditions also can be controlled due to selection of the form of various ingredients (e.g., solid, liquid, or gas), particle size or crystalline nature of ingredients of solid form, concentration of ingredients in liquid form, or the like. Ingredients can be processed into the desired composition by techniques such as extrusion, compression, spraying, and the like.

In certain embodiments, the lozenge product may be transparent or translucent. As used herein, "translucent" or "translucency" refers to materials allowing some level of light to travel therethrough diffusely. In certain embodiments, lozenge products of the present disclosure can have such a high degree of clarity that the material can be classified as "transparent" or exhibiting "transparency," which is defined as a material allowing light to pass freely through without significant diffusion. The clarity of the lozenge product is such that there is some level of translucency as opposed to opacity (which refers to materials that are impenetrable by light).

Transparency/translucency can be determined by any means commonly used in the art; however, it is commonly measured by spectrophotometric light transmission over a range of wavelengths (e.g., from about 400-700 nm). Alternatively, optical methods such as turbidimetry (or nephelometry) and colorimetry may be used to quantify the cloudiness (light scattering) and the color (light absorption), respectively, of the lozenge products provided herein. Translucency can also be confirmed by visual inspection by simply holding the material (e.g., extract) or product up to a light source and determining if light travels through the product in a diffuse manner.

*Method of preparing melt products*

In some embodiments, the product is in meltable form. For preparation of meltable products, the lipid is typically heated to slightly above the melting temperature such that the lipid is liquefied. Optionally, active ingredients, flavoring agents, and/or lecithin can be added to the liquefied lipid at this stage. Thereafter, all or a portion of the liquefied lipid can be blended with the dry blend and mixed until the product reaches the desired level of homogeneity or until the desired textural properties are achieved. The mixture is milled (e.g., in a dry roll mill) until the particle size is less than about 20 microns. The milled isomalt-palm oil is combined with any remaining lipid, and the dry ingredients and flavor mixed in. The base is generally warmed to a fluid consistency.

In some embodiments, a sugar alcohol (e.g., isomalt) is added to a mixer bowl, and a portion of the total lipid (e.g., melted palm oil) is added, along with salt and emulsifier.

Additional lipid is added with mixing until adhesive clumps form. The clumped mixture is transferred portion-wise to a 3 roll mill and processed to a particle size of less than 50 microns, or about 20 microns. The refined mixture is transferred to a mixer bowl, and the remaining lipid added with mixing. The mixture is warmed as necessary to maintain a fluid consistency.

Sweetener, flavor, and active ingredient(s) are added with mixing. Mixing is continued until a homogenous composition is obtained. The mixture is allowed to rest for a period of time, such as about 10 to 15 minutes. The composition can be divided into discrete portions, such as by pouring the composition into a sheet-like structure, cooling, and then cutting the structure into individual portions, or by depositing the composition into molds and allowing to cool. The molds may be starch molds or starchless molds. In particular embodiments, the molds are starchless.

The melt composition may be held in the mold (starch or starchless) for a predetermined duration of time such as, for example, from about 1 to about 15 minutes, to allow the melt composition to cool and solidify. Optionally, the molds containing the melt product may be cooled by refrigeration to accelerate solidification.

According to other aspects of the present disclosure, rather than using molds to prepare the melt product, an extrusion process may be employed in which the final melt product is extruded as described herein above with respect to pastille extrusion methods.

*Methods of preparing pouched oral products*

Where the product is in the form of a pouched oral product, the process may comprise the steps of:

(a) combining active ingredients;

(b) contacting the combination of active ingredients and at least one filler to provide the oral product.

The combination of active ingredients may be as described hereinabove.

In some embodiments, the step (b) comprises mixing the at least one filler and the combination of active ingredients. In some embodiments, the combination of active ingredients is in solid form (e.g. in the form of a powder). The

combination of active ingredients may be mixed directly with the filler to provide the oral product. In some embodiments, the combination of active ingredients may be dissolved in a hydrophilic solvent (e.g. water and/or alcohol) prior to contacting the filler. For example, the combination of active ingredients may be dissolved in water or alcohol (e.g. ethanol or propylene glycol) before being mixed with the filler. The process may, in such embodiments, comprise the step of drying the product so as to remove the solvent. For example, the product may be dried via heating, freeze-drying, spray-drying, or simply leaving the product at room temperature for a certain period of time. Preferably, the drying step comprises leaving the product at room temperature for a period of 1 hour to 48 hours to remove the solvent.

The process may then further comprise the step of pouching the oral product using a pouch material as described hereinabove.

**Use**

Also provided herein is piperine or a geometric isomer thereof for use in increasing the blood-brain barrier permeation of at least one p-glycoprotein substrate compound, wherein the at least one p-glycoprotein substrate compound has a log D value at pH 7.4 from about -2.5 to about 4.0; the compound being as defined herein. In preferred embodiments the at least one p-glycoprotein substrate compound is selected from crocetin crocin, withanolide A, withanone, or a combination thereof.

The use of the present disclosure may also be expressed as a method of treatment. For example, a method of increasing the blood-brain barrier permeation of at least one p-glycoprotein substrate compound which has a log D value at pH 7.4 from about -2.5 to about 4.0; the compound being as defined herein, the method comprising orally administering the compound to a subject in combination with piperine or a geometric isomer thereof.

It will be understood that all disclosure herein concerning the oral product applies to the disclosed processes, methods and uses. The disclosure is not repeated for conciseness.

**EXAMPLES**

Aspects of the present invention are more fully illustrated by the following examples, which are set forth to illustrate certain aspects of the present invention and are not to be construed as limiting thereof.

Example 1: p-glycoprotein substrate compound identification

Botanical active ingredients were identified which can be used to provide an effect in a human or animal when consumed in an oral product, and which include one or more compounds predicted using modelling software GastroPlus® (ADMET Predictor Module, available from SimulationsPlus) to have a medium or low BBB permeability. High permeability was defined as greater than 100 nm/s, medium BBB permeability was defined as 10 to 100 nm/s, and low BBB permeability was defined as less than 10 nm/s.

The botanicals selected were Ashwagandha, Holy Basil and Saffron. The compounds predicted to have low or medium BBB permeability are listed in Table 1. For the experiments of Example 1, these compounds were obtained from a commercial source but they may also be extracted from the respective botanical and then evaluated for p-glycoprotein substrate activity. Suitable extraction methods are known in the art. The log D value at pH 7.4, and the log P value were obtained from the modelling software GastroPlus® (ADMET Predictor Module, available from SimulationsPlus), and the molar mass of each compound was obtained from the literature (e.g. a chemical structure database such as www.chemspider.com).

**Table 1**

| **Botanical** | **Compounds** | ***In silico* BBB permeability prediction** | **Log D at pH 7.4[1]** | **Log P[2]** | **Molar mass (g/mol)** |
|---|---|---|---|---|---|
| Saffron | Safranal | Medium | 2.48 | 2.48 | 150.2 |
| | Crocetin | Low | 0.16 | 3.88 | 328.4 |
| | Crocin | Low | -1.70 | -1.70 | 977.0 |
| Ashwagandha | Withanolide A | Low | 3.18 | 3.18 | 470.6 |
| | Withanolide B | Low | 4.13 | 4.13 | 454.6 |
| | Withanone | Medium | 3.06 | 3.06 | 470.6 |

(continued)

| Botanical | Compounds | *In silico* BBB permeability prediction | Log D at pH 7.4[1] | Log P[2] | Molar mass (g/mol) |
|---|---|---|---|---|---|
| Holy Basil | Apigenin | Medium | 2.53 | 2.86 | 270.2 |
| | Oleanolic acid | Low | 5.10 | 7.26 | 456.7 |
| | Ursolic acid | Low | 4.89 | 7.02 | 456.7 |
| | Luteolin 7-glucuronide | Low | -1.77 | 0.19 | 462.4 |
| 1 = with root mean square error (RMSE) of log 0.56 and mean absolute error of log 0.41<br>2 = with root mean square error (RMSE) of log 0.30 and mean absolute error of log 0.23 | | | | | |

To evaluate whether the compounds were p-glycoprotein substrates, a MDCK-MDR1 permeability assay was employed. Such assays are known in the art and provide a well-established *in vitro* cell-based model for BBB permeation.

MDCK-MDR1 cells originate from transfection of Madin-Darby canine kidney (MDCK) cells with the MDR1 gene (ABCB1), the gene encoding for the efflux protein P-glycoprotein. Assessing transport in both directions across the cell monolayer - apical to basolateral (A2B) and basolateral to apical (B2A) - with/without a known, potent and commercially available P-gp inhibitor (GF 120918), enables the identification of a compound as a P-gp substrate.

The MDCK-MDR1 permeability assay protocol was as follows:

| | |
|---|---|
| Test Article Concentration | 1 or 10 μm |
| Cell line | MDCK-MDR1 cell line heterologously expressing the human P-gp transporter (MDR1) |
| Subculture | At 80-90% confluence |
| Number of Replicates | 3 |
| Incubation Time | 60 minutes |
| Growth conditions | 37°C, 5% $CO_2$ |
| pH | 7.4 |
| Membrane Integrity Marker | Lucifer Yellow |
| Control Compounds | Digoxin, metoprolol, atenolol, GF 120918 |
| Analysis Method | LC-MS/MS quantification |

The results obtained for the tested compounds are shown in Table 2 below.

**Table 2**

| Compound | Compound Alone | | + P-gp inhibitor (GF 120918) | |
|---|---|---|---|---|
| | A2B Apparent permeability (Papp), nm/s | B2A Apparent permeability (Papp), nm/s | A2B Apparent permeability (Papp), nm/s | B2A Apparent permeability (Papp), nm/s |
| Safranal | 900 | 433 | 784 | 361 |
| Crocetin | 16.7 | 58.4 | 43.2 | 28.9 |
| Crocin | 2.2 | 31.2 | 20.1 | 22.8 |
| Withanone | 12.3 | 316 | 158 | 197 |
| Withanolide A | 36.5 | 222 | 173 | 200 |
| Withanolide B | 120 | 133 | 135 | 116 |
| Oleanoic acid | 1.74 | 3.5 | 2 | 2.85 |
| Ursolic acid | 0.68 | 2.1 | 4.8 | 1.4 |

(continued)

| Compound | Compound Alone | | + P-gp inhibitor (GF 120918) | |
|---|---|---|---|---|
| | A2B Apparent permeability (Papp), nm/s | B2A Apparent permeability (Papp), nm/s | A2B Apparent permeability (Papp), nm/s | B2A Apparent permeability (Papp), nm/s |
| Apigenin | 97.1 | 191 | 105 | 120 |
| Luteolin-7-glucoronide | 2.05 | 0.76 | 1.75 | 1.34 |

Increased A2B and decreased B2A in presence of P-gp inhibitor identifies the compounds as a P-gp substrate. Hence, it can be seen that 5 out of the 10 tested compounds identified as p-glycoprotein substrates: saffron (2/3), ashwagandha (2/3), holy basil (1/4). The p-glycoprotein substrate compounds were crocetin, crocin, withanone, withanolide A, and ursolic acid.

The results for safranal and withanone are shown in Figure 1 where "A2B" is the left-hand bar of each entry (e.g. of Safranal alone, Safranal + GF (inhibitor), Withanone alone, and Withanone + GF (inhibitor)), and "B2A" is the right-hand bar of each entry.

Example 2: Evaluation of efflux inhibitors piperine and quercetin to enhance BBB permeation

Piperine and quercetin were identified as possible compounds to use in combination with the compounds tested in Example 1 because each is known in the art as a p-glycoprotein inhibitor. The log D value at pH 7.4, the log P value, and the apparent MDCK transwell permeability value were obtained from the modelling software GastroPlus® (ADMET Predictor Module, available from SimulationsPlus) and are shown in Table 3. It can be seen that both compounds also have similar log D at pH 7.4 and log P values.

**Table 3**

| Compound | Log D at pH 7.4 | Log P |
|---|---|---|
| Piperine | 2.65 | 2.65 |
| Quercetin | 1.53 | 1.96 |

Piperine and quercetin were each used in combination with the compounds tested in Example 1. The individual compounds and the combinations were tested for BBB permeability using the same MDCK-MDR1 protocol as in Example 1, except piperine/quercetin were added at a concentration of 30 μM and each compound was added at a concentration of 1 or 10 μM. These concentrations were selected based on the sensitivity of the detection method as is standard in the art; the EC50 value for each of piperine and quercetin is, for instance, 30 μM meaning that there was adequate inhibition without oversaturation of the cells. The transport from apical to basolateral (A2B) across the cell monolayer was evaluated.

The results and concentrations used are shown in Table 4 below. The results are also shown in Figures 2 to 11.

**Table 4**

| Compound | Concentratio n (μM) | Compound Alone | + Piperine (30 μM) | + Quercetin (30 μM) |
|---|---|---|---|---|
| | | A2B Apparent permeability (Papp), nm/s | A2B Apparent permeability (Papp), nm/s | A2B Apparent permeability (Papp), nm/s |
| Safranal | 10 | 900 | 750 | 820 |
| Crocetin | 10 | 16.7 | 44.5 | 49.5 |
| Crocin | 10 | 2.2 | 22.4 | 3.5 |
| Withanone | 1 | 12.3 | 155 | 30 |
| Withanolide A | 1 | 36.5 | 163 | 67.1 |
| Withanolide B | 1 | 120 | 144 | 157 |
| Oleanoic acid | 10 | 1.74 | 1.52 | 2.4 |

(continued)

| Compound | Concentratio n (μM) | Compound Alone | + Piperine (30 μM) | + Quercetin (30 μM) |
|---|---|---|---|---|
| Ursolic acid | 10 | 0.675 | 0.581 | 1.14 |
| Apigenin | 10 | 97.1 | 78.1 | 97 |
| Luteolin-7-glucoronide | 1 | 2.05 | 2.25 | 3.42 |

High BBB permeability was defined as greater than 100 nm/s. Medium BBB permeability was defined as 10 to 100 nm/s and low BBB permeability was defined as less than 10 nm/s. Thus the following conclusions can be drawn:

- Safranal has a high permeability alone (900 nm/s) and when combined with piperine (750 nm/s) or quercetin (820 nm/s);
- Crocetin has a medium permeability when used alone and in combination with piperine or quercetin but an increase from 16.7 nm/s to 44.5 or 49.5 nm/s is observed with piperine and quercetin respectively;
- Crocin has a low permeability (2.2 nm/s) when used alone or in combination with quercetin (3.5 nm/s) but medium permeability (22.4 nm/s) when used in combination with piperine;
- Withanone has a medium permeability (12.3 nm/s) when used alone, or in combination with quercetin (30 nm/s), but a high permeability (155 nm/s) when used in combination with piperine;
- Withanolide A has a medium permeability (36.5 nm/s) when used alone or in combination with quercetin (67.1 nm/s), but a high permeability (163 nm/s) when used in combination with piperine;
- Withanolide B has a high permeability alone (120 nm/s) and when combined with piperine (144 nm/s) or quercetin (157 nm/s);
- Oleanoic acid has a low permeability alone (1.74 nm/s) and when combined with piperine (1.52 nm/s) or quercetin (2.4 nm/s);
- Ursolic acid has a low permeability alone (0.675 nm/s) and when combined with piperine (0.581 nm/s) or quercetin (1.14 nm/s);
- Apigenin has a medium permeability alone (97.1 nm/s) and when combined with piperine (78.1 nm/s) or quercetin (97 nm/s);
- Luteolin-7-glucoronide has a low permeability alone (2.05 nm/s) and when combined with piperine (2.25 nm/s) or quercetin (3.42 nm/s).

When combined with selected actives, piperine can therefore enhance permeation across the BBB *in vitro.* Not only is this enhancement more effective than quercetin, but the enhanced actives are all p-glycoprotein substrate compounds - as demonstrated by Example 1 - having a log D value at pH 7.4 between -2.5 and 4.5 (see Table 1). Ursolic acid, for instance, is a p-glycoprotein substrate but has a log D value at pH 7.4 of 4.89 and the results show that there is no effect when this compound is combined with piperine.

As both piperine and quercetin are P-gp inhibitors, it was believed that they would have the same effect when combined with the compounds of Example 1, particularly those compounds identified as P-gp substrates. It was, for instance, believed that the P-gp inhibitors (piperine or quercetin) would compete for the binding sites on the P-gp protein, thereby diminishing the interaction between the P-gp substrates (withanone, withanolide A, crocin or crocetin) and P-gp, which would decrease efflux and subsequently increase permeation from blood to brain, i.e. A2B. This was not, however, the trend observed. Instead, there was a significant difference between the piperine and quercetin combinations. Unlike piperine, quercetin did not, for instance, significantly enhance the BBB permeability of crocin, withanone, or withanolide A.

The various embodiments described herein are presented only to assist in understanding and teaching the claimed features. These embodiments are provided as a representative sample of embodiments only, and are not exhaustive and/or exclusive. It is to be understood that advantages, embodiments, examples, functions, features,

structures, and/or other aspects described herein are not to be considered limitations on the scope of the invention as defined by the claims or limitations on equivalents to the claims, and that other embodiments may be utilised and modifications may be made without departing from the scope of the claimed invention. Various embodiments of the invention may suitably comprise, consist of, or consist essentially of, appropriate combinations of the disclosed elements, components, features, parts, steps, means, etc., other than those specifically described herein. In addition, this disclosure may include other inventions not presently claimed, but which may be claimed in future

## Claims

**1.** An oral product comprising a combination of active ingredients, wherein the combination of active ingredients comprises:

i. piperine or a geometric isomer thereof, and
ii. at least one p-glycoprotein substrate compound having a log D value at pH 7.4 from about -2.5 to about 4.5.

**2.** The oral product according to claim 1, wherein the log D value at pH 7.4 of the at least one p-glycoprotein substrate compound is from about -2.5 to about 4.0.

**3.** The oral product according to claim 1 or claim 2, wherein the log D value at pH 7.4 of the at least one p-glycoprotein substrate compound is from about -2.0 to about 0.5, or from about 3.0 to about 3.5.

**4.** The oral product according to any preceding claim, wherein the at least one p-glycoprotein substrate compound has a log P value of from about -2.5 to about 5.0, preferably from about -1.0 to about 4.0.

**5.** The oral product according to any preceding claim, wherein the at least one p-glycoprotein substrate compound has a molar mass of greater than about 300 g/mol, preferably greater than about 375 g/mol.

**6.** The oral product according to any preceding claim, wherein the at least one p-glycoprotein substrate compound has a molar mass of no more than about 1500 g/mol, preferably no more than about 1200 g/mol.

**7.** The oral product according to any preceding claim, wherein the at least one glycoprotein substrate compound is derived from a botanical.

**8.** The oral product of claim 7, wherein the botanical is selected from ginseng, maca root, passionflower, lemon balm, chamomile, saffron, cocoa, Echinacea, and *Camellia sinensis,* preferably wherein the botanical is selected from ginseng and saffron.

**9.** The oral product of any preceding claim, wherein the at least one glycoprotein substrate compound is selected from crocetin, crocin, withanolide A, withanone, or a combination thereof, preferably wherein the at least p-glycoprotein substrate compound is selected from withanolide A, withanone, or a combination thereof.

**10.** The oral product of any preceding claim, wherein piperine or the geometric isomer thereof is present in an amount from about 0.0001 wt% to about 0.5 wt%, based on the total weight of the oral product.

**11.** The oral product of any preceding claim, wherein the at least one p-glycoprotein substrate compound is present in an amount from about 0.0001 wt% to about 0.1 wt%, based on the total weight of the oral product.

**12.** The oral product of any preceding claim, wherein piperine or the geometric isomer thereof is present in an amount from about 5 mg to about 20 mg in the oral product, preferably wherein piperine is present in an amount from about 8 mg to about 18 mg in the oral product.

**13.** The oral product of any preceding claim, wherein the oral product is a liquid dosage form, optionally wherein the liquid oral dosage form has a volume of from about 20 mL to about 100 mL of liquid; or wherein the oral product is a solid oral product in chewable form, preferably wherein the oral product is in the form of a dissolvable chew.

**14.** The oral product of any one of claims 1 to 12, wherein the oral product is a liquid dosage form and further comprises water in an amount of from about 50 wt% to about 99.9 wt%, based on the total weight of the oral product, or wherein

the oral product is a solid oral product in chewable form and further comprises at least one binding agent, and at least one bulking agent selected from the group consisting of a sugar alcohol, a sugar, or a combination thereof.

**15.** Piperine or a geometric isomer thereof for use in increasing the blood-brain barrier permeation of at least one p-glycoprotein substrate compound, wherein the at least one p-glycoprotein substrate compound has a log D value at pH 7.4 from about -2.5 to about 4.5, preferably wherein the at least one p-glycoprotein substrate compound is selected from crocetin, crocin, withanolide A, withanone, or a combination thereof.

FIGURE 1

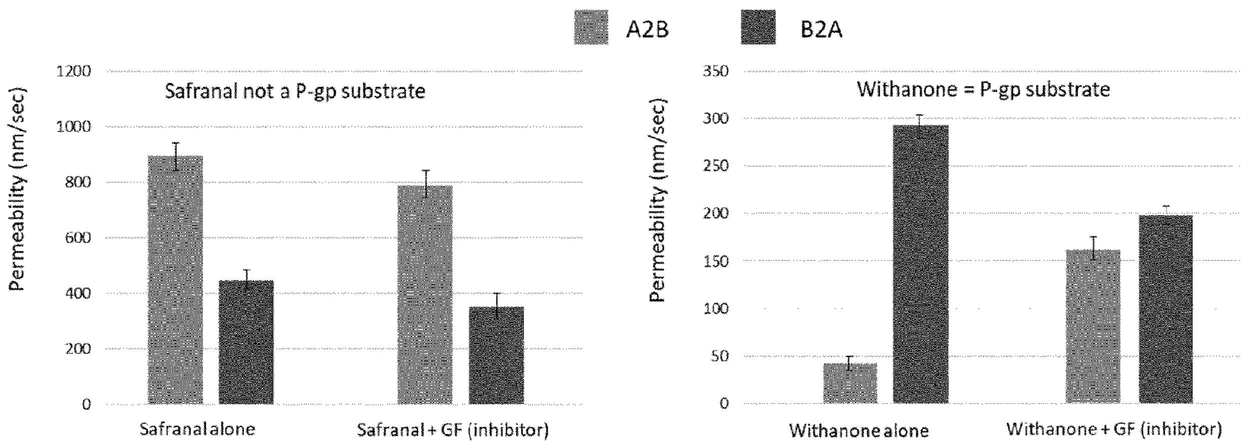
A2B
B2A
Safranal not a P-gp substrate
Permeability (nm/sec)
1200
1000
800
600
400
200
0
Safranal alone
Safranal + GF (inhibitor)
Withanone = P-gp substrate
Permeability (nm/sec)
350
300
250
200
150
100
50
0
Withanone alone
Withanone + GF (inhibitor)

FIGURE 2

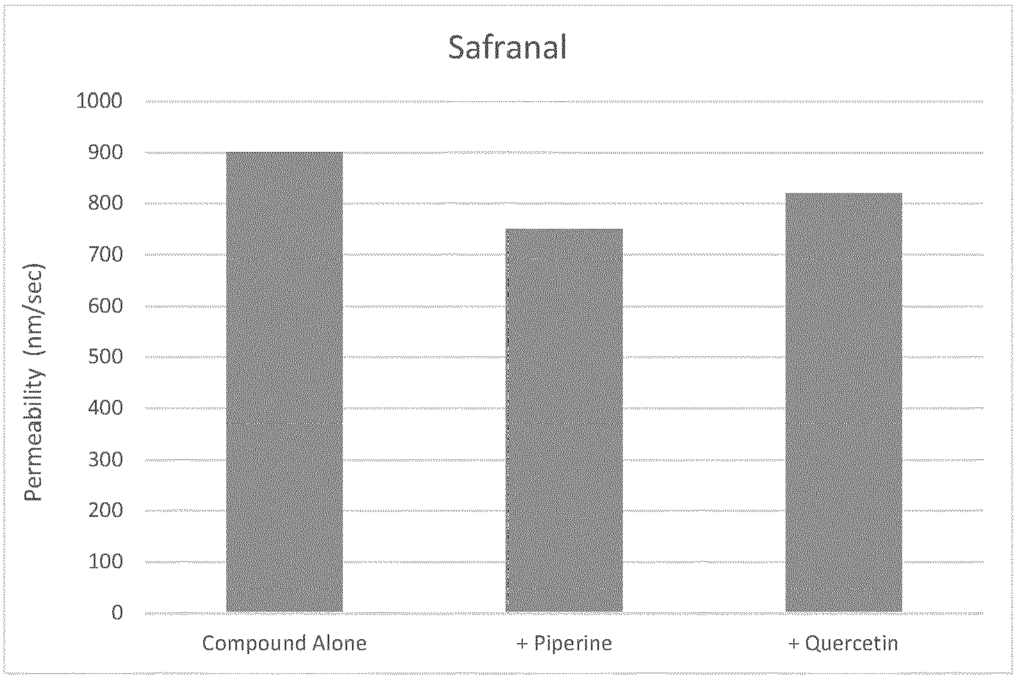


FIGURE 3

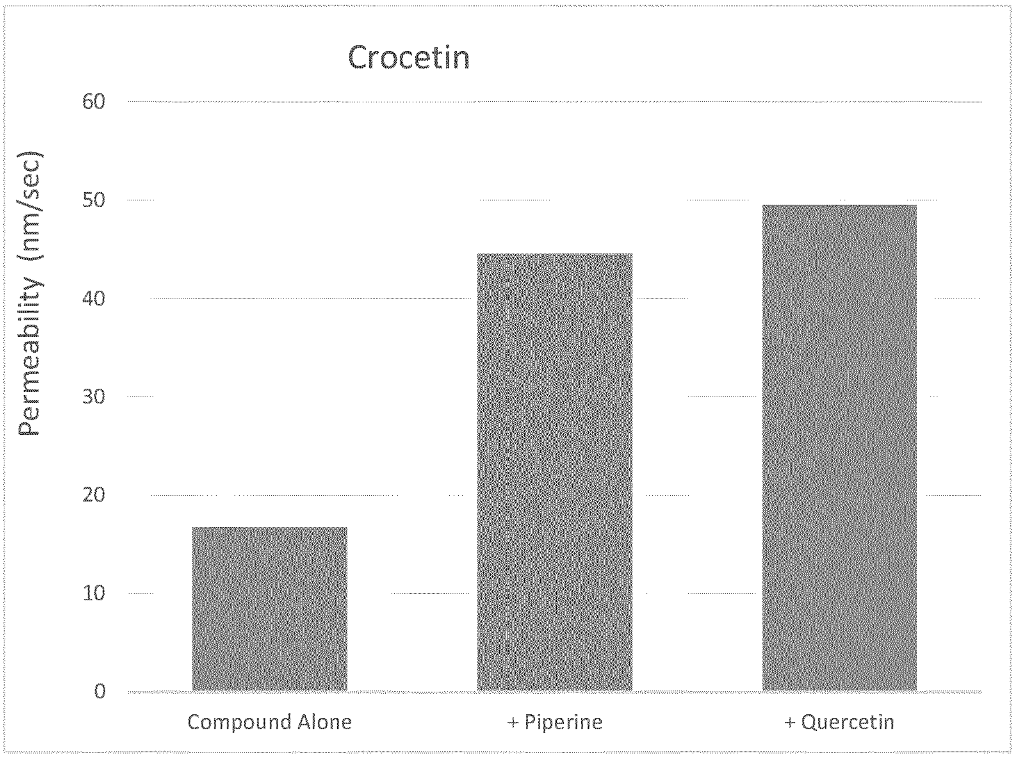

FIGURE 4

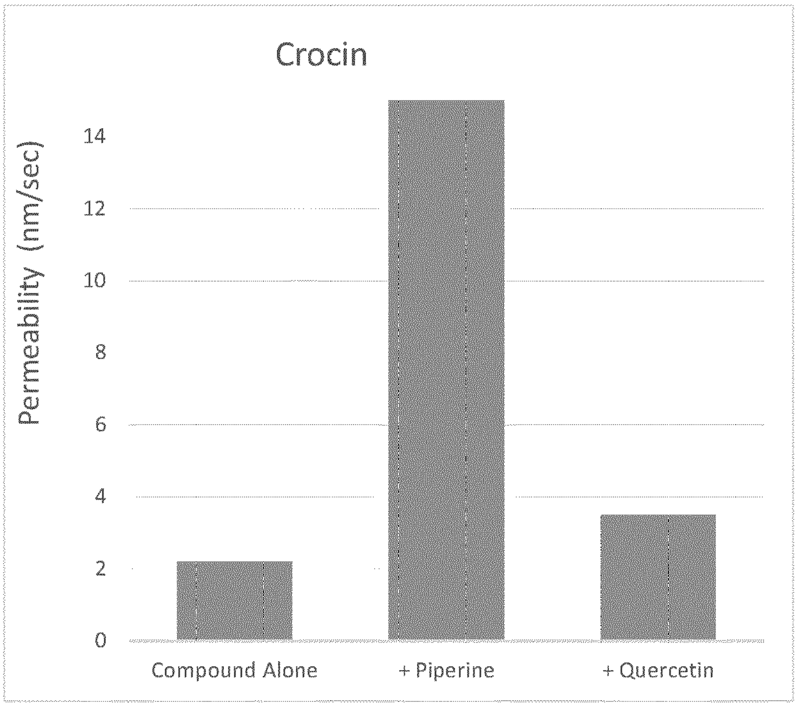


FIGURE 5

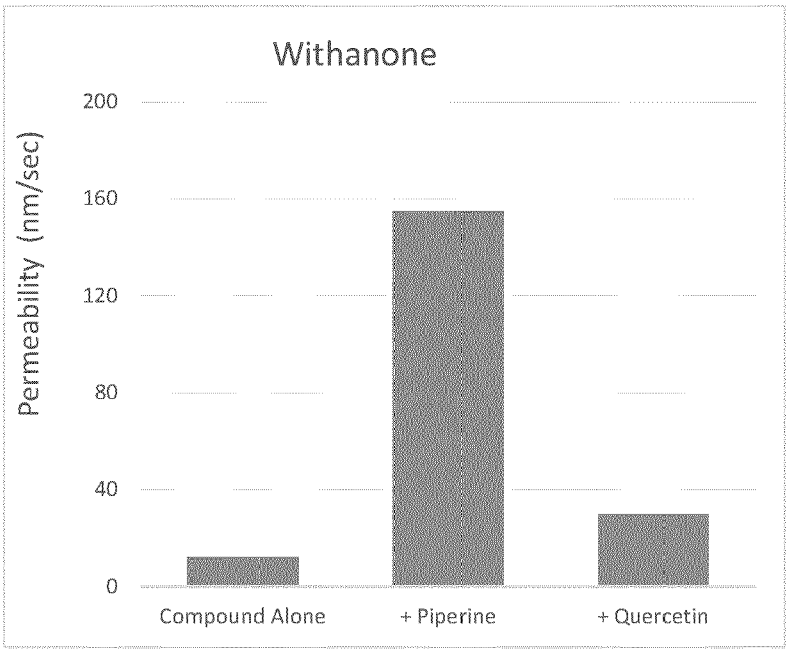

FIGURE 6

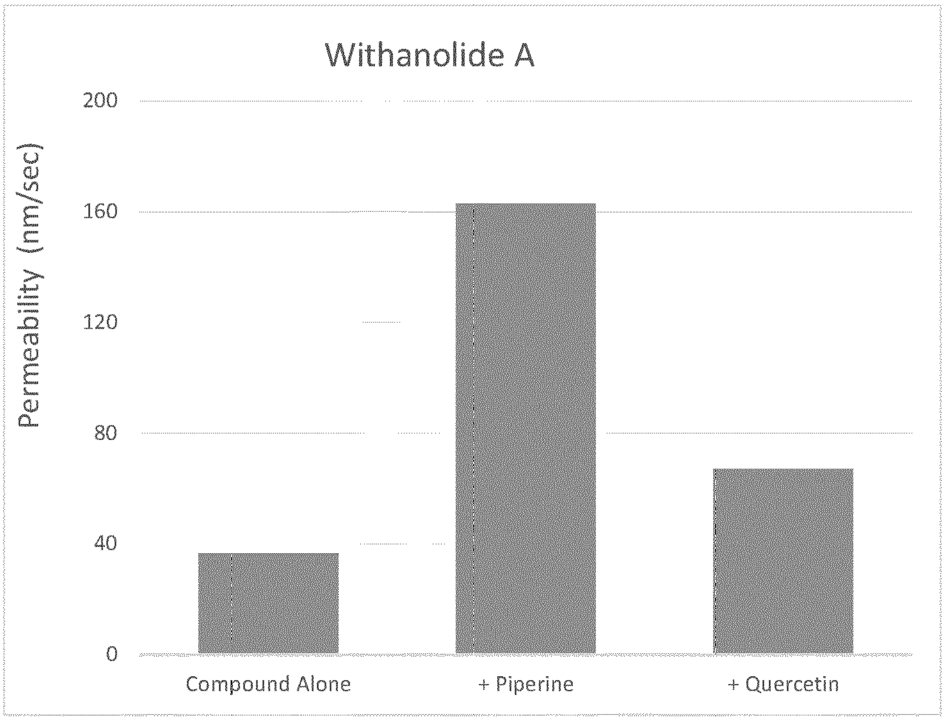


FIGURE 7

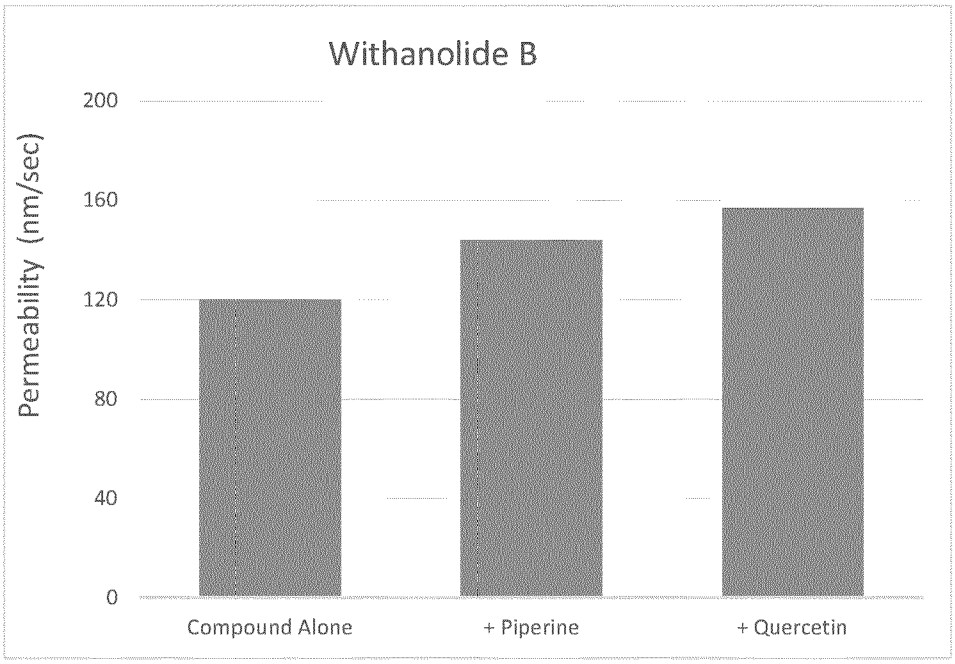

FIGURE 8

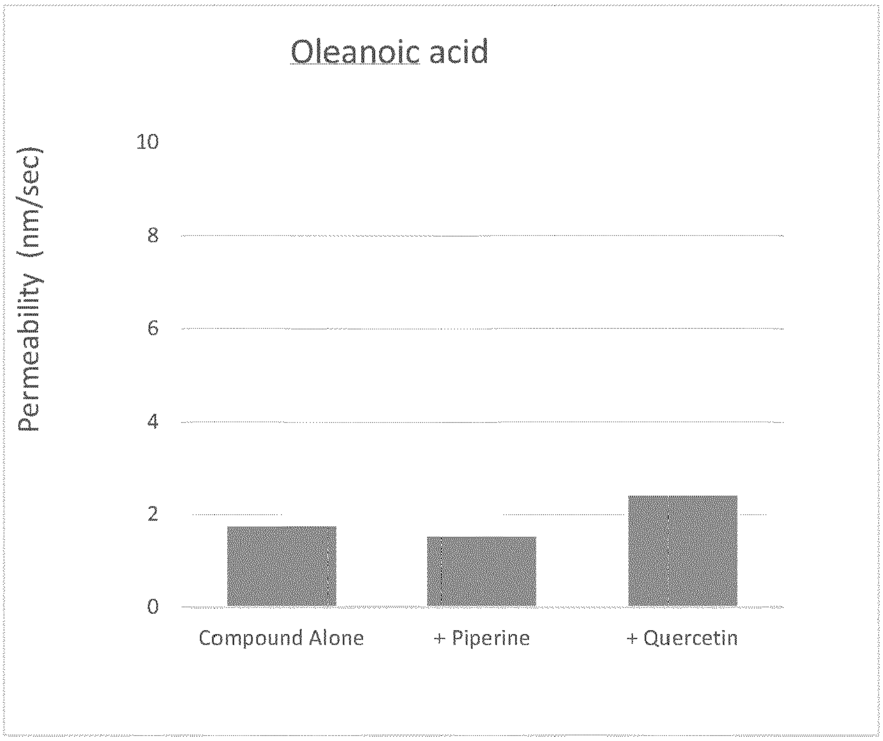


FIGURE 9

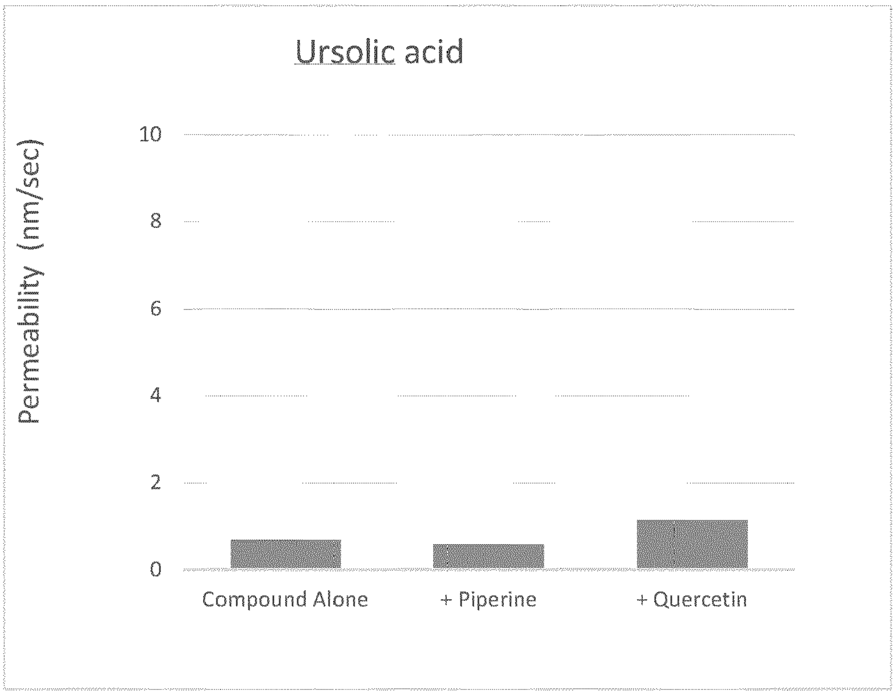

FIGURE 10

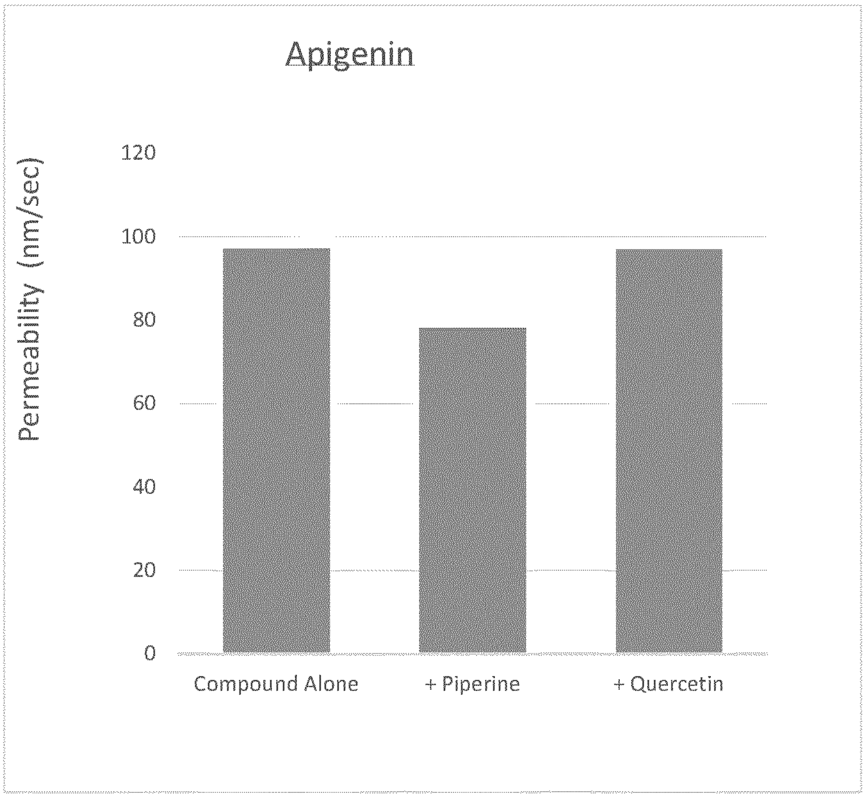


FIGURE 11

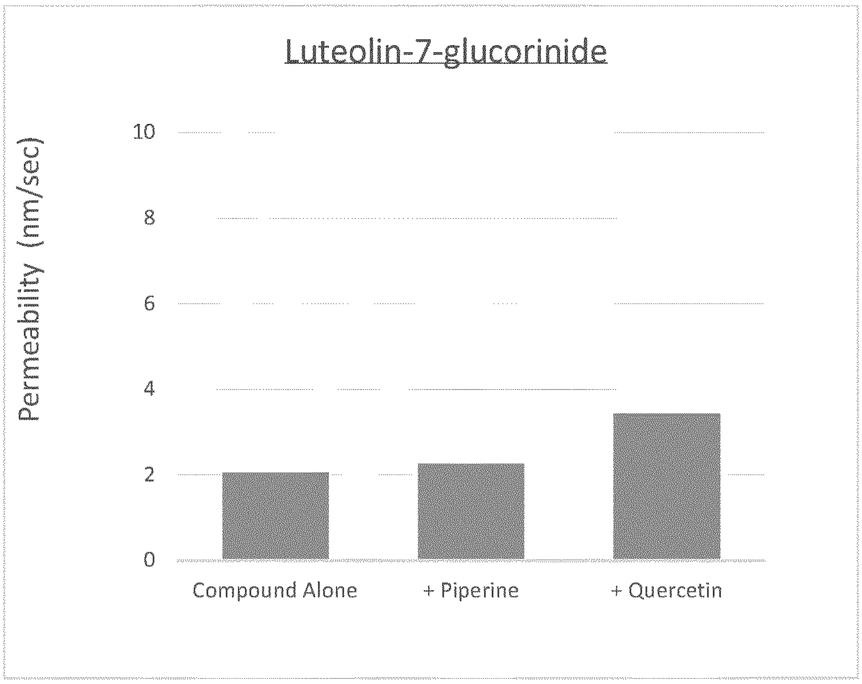

Europäisches Patentamt
European Patent Office
Office européen des brevets

# EUROPEAN SEARCH REPORT

Application Number
EP 24 16 6746

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2023/404969 A1 (MAJEED MUHAMMED [IN] ET AL) 21 December 2023 (2023-12-21) * paragraphs [0023], [0053]; table 7 * | 1-15 | INV. A23L33/105 A61K31/00 |
| X | US 2019/373920 A1 (GRAYELI YASS VOSSOUGH [US]) 12 December 2019 (2019-12-12) * paragraph [0029]; claims 16,17,; figures 2-4 * | 1-15 | |
| X | DATABASE GNPD [Online] MINTEL; 24 October 2016 (2016-10-24), anonymous: "Power To Sleep PM", XP055956336, Database accession no. 4368387 * abstract * | 1,15 | |
| X | Xyngular Corporation: "PRODUCT INGREDIENT LIST", , 1 January 2018 (2018-01-01), XP093045459, Retrieved from the Internet: URL:https://xyngular-xbo-media.s3.amazonaws.com/Products%20and%20Kits/US%20Product%20Ingredient%20List%20with%20Pics%20and%20Niacin%20update.pdf [retrieved on 2023-05-09] * the whole document * | 1,15 | TECHNICAL FIELDS SEARCHED (IPC): A23L A61K |
| X | US 2020/337985 A1 (MORLEY MYRIAM [US]) 29 October 2020 (2020-10-29) * paragraphs [0042], [0059] * | 1,15 | |

The present search report has been drawn up for all claims

1

| Place of search | Date of completion of the search | Examiner |
|---|---|---|
| The Hague | 20 August 2024 | Galleiske, Anke |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.........................................................
& : member of the same patent family, corresponding document

EPO FORM 1503 03.82 (P04C01)

## ANNEX TO THE EUROPEAN SEARCH REPORT ON EUROPEAN PATENT APPLICATION NO. EP 24 16 6746

This annex lists the patent family members relating to the patent documents cited in the above-mentioned European search report. The members are as contained in the European Patent Office EDP file on
The European Patent Office is in no way liable for these particulars which are merely given for the purpose of information.

20-08-2024

| Patent document cited in search report | Publication date | Patent family member(s) | Publication date |
|---|---|---|---|
| US 2023404969 A1 | 21-12-2023 | US 2023404969 A1 | 21-12-2023 |
| | | WO 2023249651 A1 | 28-12-2023 |
| US 2019373920 A1 | 12-12-2019 | US 2019373920 A1 | 12-12-2019 |
| | | WO 2019236996 A1 | 12-12-2019 |
| US 2020337985 A1 | 29-10-2020 | US 10709659 B1 | 14-07-2020 |
| | | US 2020337985 A1 | 29-10-2020 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 9237769 B, Mua
- US 7861728 B, Holton, Jr.
- US 20100291245, Gao
- US 20070062549, Holton, Jr.
- US 9204667 B, Cantrell
- US 9775376 B, Cantrell
- US 10357054 B, Marshall
- US 20120037175, Cantrell
- US 4967773 A, Shaw
- US 5110605 A, Acharya
- US 5733574 A, Dam
- US 6280761 B, Santus
- US 6676959 B, Andersson
- US 6248760 B, Wilhelmsen
- US 7374779 B
- US 20010016593 A, Wilhelmsen
- US 20040101543 A, Liu
- US 20060120974 A, Mcneight
- US 20080020050 A, Chau
- US 20090081291 A, Gin
- US 20100004294 A, Axelsson
- US 5167244 A, Kjerstad
- US 8931493 B, Sebastian
- US 20160000140, Sebastian
- US 20160073689 A, Sebastian
- US 20160157515 A, Chapman
- US 20160192703 A, Sebastian
- US 7014039 B, Henson
- US 7537110 B, Kutsch
- US 7584843 B, Kutsch
- US 8397945 B, Gelardi
- US D592956 S, Thiellier
- US D594154 S, Patel
- US D625178 S, Bailey
- US 20080173317 A, Robinson
- US 20090014343 A, Clark
- US 20090014450 A, Bjorkholm
- US 20090250360 A, Bellamah
- US 20090266837 A, Gelardi
- US 20090223989 A, Gelardi
- US 20090230003 A, Thiellier
- US 20100084424 A, Gelardi
- US 20100133140 A, Bailey
- US 20100264157 A, Bailey
- US 20110168712 A, Bailey
- US 4148325 A, Solomon
- US 6510855 B, Korte
- US 6834654 US, Williams
- US 6834654 B, Williams
- US 4725440 A, Ridgway
- US 6077524 A, Bolder
- US 472544015 B, Ridgway
- US 3806617 A, Smylie
- US 3098492 A, Wursburg
- US 4874000 A, Tamol
- US 254880018 B, Graves
- US 4989620 A, Keritsis
- US 5072744 A, Luke
- US 5829453 A, White
- US 6182670 B, White
- US 87678510, Hunt


### Non-patent literature cited in the description


- **CHEN** ; **LIU**. *Adv Drug Deliv Rev.*, 15 May 2012, vol. 64 (7), 640-65
- LFRA Ingredients Handbook, Sweeteners. Leatherhead Food RA, 1996, 21-44